(19) Europäisches Patentamt
European Patent Office
Office européen des brevets

(11) **EP 3 322 484 B1**

(12) # EUROPEAN PATENT SPECIFICATION

(45) Date of publication and mention of the grant of the patent:
**24.03.2021 Bulletin 2021/12**

(21) Application number: **16825114.8**

(22) Date of filing: **13.07.2016**

(51) Int Cl.:
***G01N 33/574*** *(2006.01)*

(86) International application number:
**PCT/US2016/042100**

(87) International publication number:
**WO 2017/011559 (19.01.2017 Gazette 2017/03)**

(54) **METHOD FOR DETECTING CANCER STEM CELLS**

VERFAHREN ZUM NACHWEIS VON TUMORSTAMMZELLEN

MÉTHODE DE DÉTECTION DE CELLULES SOUCHES CANCÉREUSES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **14.07.2015 US 201562192204 P**

(43) Date of publication of application:
**23.05.2018 Bulletin 2018/21**

(73) Proprietor: **Medimmune, LLC**
**Gaithersburg, Maryland 20878 (US)**

(72) Inventors:
- **VAN VLERKEN-YSLA, Lilian Emilia**
  **Gaithersburg, MD 20878 (US)**
- **HURT, Elaine Marie**
  **Gaithersburg, MD 20878 (US)**

(74) Representative: **AstraZeneca**
**Milstein Building**
**Granta Park**
**Cambridge CB21 6GH (GB)**

(56) References cited:

| | |
|---|---|
| **WO-A1-2009/156994** | **US-A1- 2007 099 209** |
| **US-A1- 2012 148 592** | **US-A1- 2013 109 629** |
| **US-A1- 2014 154 799** | **US-A1- 2014 155 332** |

- **KANG-JIE CHEN ET AL: "Selective Recruitment of Regulatory T Cell through CCR6-CCL20 in Hepatocellular Carcinoma Fosters Tumor Progression and Predicts Poor Prognosis", PLOS ONE, vol. 6, no. 9, 14 September 2011 (2011-09-14), page e24671, XP055320611, DOI: 10.1371/journal.pone.0024671**
- **RUBIE CLAUDIA ET AL: "CCL20/CCR6 expression profile in pancreatic cancer", JOURNAL OF TRANSLATIONAL MEDICINE, BIOMED CENTRAL, vol. 8, no. 1, 10 May 2010 (2010-05-10), page 45, XP021078872, ISSN: 1479-5876, DOI: 10.1186/1479-5876-8-45**
- **KLEEFF J ET AL: "DETECTION AND LOCALIZATION OF MIP-3ALPHA/LARC/EXODUS, A MACROPHAGE PROINFLAMMATORY CHEMOKINE, AND ITS CCR6 RECEPTOR IN HUMAN PANCREATIC CANCER", INTERNATIONAL JOURNAL OF CA, JOHN WILEY & SONS, INC, US, vol. 81, no. 4, 17 May 1999 (1999-05-17), pages 650-657, XP008054224, ISSN: 0020-7136, DOI: 10.1002/(SICI)1097-0215(19990517)81:4<650: :AI D-IJC23>3.0.CO;2-#**
- **DARASH-YAHANA MERAV ET AL: "Pro-tumorigenic roles for CCL20, CCR6, IL-23 and Th17 cells in human prostate cancer", AMERICAN ASSOCIATION FOR CANCER RESEARCH. PROCEEDINGS OF THE ANNUAL MEETING, AMERICAN ASSOCIATION FOR CANCER RESEARCH, US, vol. 49, 1 April 2008 (2008-04-01), page 1281, XP009123895, ISSN: 0197-016X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 322 484 B1

Printed by Jouve, 75001 PARIS (FR)

**(Cont. next page)**

- KRYCZEK ET AL. IL -22+ CD 4+ T CELLS PROMOTE COLORECTAL CANCER STEMNESS VIA STAT3 TRANSCRIPTION FACTOR ACTIVATION AND INDUCTION OF THE METHYLTRANSFERASE DOT1 L vol. 40, 15 May 2014, pages 772 - 784, XP055344548

## Description

### BACKGROUND

Cancer stem cells (CSCs), as their name implies, are a subset of cancer cells with stem cell like characteristics of pluripotency and unlimited self-renewal. As such, it is believed that this subpopulation of cells is responsible for tumor formation and adaptation to its environment. Several lines of evidence now indicate that CSCs are likely responsible for drug resistance, metastasis, and relapse of cancer, particularly in instances with minimal residual disease. In fact, recent clinical evidence showed that the fraction of breast cancer cells that survived following standard-of-care therapy was enriched in cells bearing a CSC signature (Creighton et al., 2009). In a similar study, patients with 5q deletion MDS were also found to have residual populations of malignant stem cells in their bone marrow, despite having entered clinical and cytogenetic remission (Tehranchi et al., 2010). In many clinical indications, it is now believed that despite initial therapeutic response, the retention of a distinct subset of resistant cancer cells can lead to relapse and potentially metastasis.

Since their initial identification, cancer stem cells have been discovered and validated in many tumor types. Following the original identification of a CSC in a model of AML (Lapidot et al., 1994), CSCs were discovered and validated in a number of hematological and solid tumor malignancies. The first evidence of CSCs in solid tumors came from breast cancer, where CSCs were found to bear a $CD44^+/CD24^-$ surface marker phenotype (Al-Hajj et al., 2003). CSCs in pancreatic cancer have also been well characterized. While various reports identify pancreatic CSCs as either $Epcam^+/CD44^+/CD24^+$ (Li et al., 2007) or $CD133^+$ (Hermann et al., 2007), in the inventors' hands, the triple positive $Epcam^+/CD44^+/CD24^+$ signature tracks best with the tumorigenic phenotype of a CSC (van Vlerken et al., 2013). A number of reports identify CSCs in colorectal cancer, and research has identified a stem cell origin in the mouse for intestinal and colorectal tumors (Barker et al., 2009). Nevertheless, the state of the art in this field has not reached a consensus regarding the surface marker phenotype for human colorectal CSCs. While many surface markers have been reported, such as CD66 (Gemei et al., 2013), LGR5 (Hirsch et al., 2014), and CD44 and CD133 (Wang et al., 2012), the field lacks any robust confirmation that any of these surface marker phenotypes may represent a consensus for human colorectal CSCs, allowing for other techniques such as non-adherent sphere growth (e.g., colospheres for colorectal CSCs) to find common use in identifying the presence of CSCs.

Drugs that target CSCs are emerging as a critical component of any successful therapy against cancer. Current research is showing that CSCs are responsible for chemoresistance, metastasis, and ultimately relapse of the cancer, even after an initial successful therapeutic intervention. While CSCs are driven by the same major self-renewal pathways that drive pluripotency of embryonic stem cells, knowledge of other cellular pathways that drive CSC activity is still limited.

US 2007/0099209 describes gene expression profiles associated with solid tumor stem cells.

The availability of additional compositions and methods for treatment of cancers, including methods for reducing tumorigenicity of cancer and inhibiting or killing cancer cells, such as cancer stem cells (e.g., inducing apoptosis or differentiation), would allow for more therapeutic options and hold the potential for better clinical outcomes such as disease remission and/or improvement of patient quality of life.

### SUMMARY OF THE DISCLOSURE

The disclosure provides methods for diagnosis, prognosis, quantification, identification, and/or detection of the presence of a cancer stem cell (CSC) in a sample comprising cancer cells, wherein the method comprises:

contacting the sample with an agent that binds to a CCL20 nucleic acid sequence or a CCL20 amino acid sequence;
detecting the presence or absence of binding between the agent and the CCL20 nucleic acid sequence or the CCL20 amino acid sequence; and
identifying the presence of the CSC in the sample upon detection of binding between the agent and the CCL20 nucleic acid sequence or the CCL20 amino acid sequence.

In embodiments of this aspect, the method can further comprise one or more of:

quantifying the amount of the CCL20 nucleic acid sequence or the CCL20 amino acid sequence in the sample;
comparing the amount of the CCL20 nucleic acid sequence or the CCL20 amino acid sequence to a reference level of CCL20 nucleic acid or CCL20 amino acid; and/or
identifying the presence of the CSC in the sample when the detected amount is greater than the reference level.

In yet further embodiments, the method may comprise an agent comprising a detectable moiety. In some embodiments, the agent may comprise a nucleic acid sequence that hybridizes to at least a portion of the CCL20 nucleic acid sequence under stringent hybridization conditions. In some embodiments, the agent may comprise an antibody that specifically binds to at least a portion of the CCL20 amino acid sequence.

## BRIEF DESCRIPTION OF THE DRAWINGS

For the purpose of illustrating the disclosure, there are depicted in the drawings certain aspects of the disclosure.

**Figure 1A-1E** shows that CSCs are found to have increased expression of CCL20 over non-CSCs. CSC-enriching sphere culture promotes the secretion of CCL20 compared to standard monolayer culture in 4 out of 8 breast cancer lines (A), 2 out of 4 pancreatic cancer lines (B), and 6 out of 7 colorectal cancer lines (C) tested. CCL20 levels were measured in picogram/milliliter (pg/mL) from conditioned medium of cells after 4 days in culture by ELISA and pg/mL CCL20 levels were subsequently converted to fold-change compared to monolayer cultured cells of the same line. FACS isolated $CD44^{high/+}$ $CD24^{low/-}$ CSCs from 2 breast cell lines (MDAMB468 and HCC1937) have elevated CCL20 mRNA levels compared with $CD44^{low/-}$ $CD24^{low/-}$ non-CSCs isolated from the same lines (D). Similarly, isolated CSCs from 1 breast (BR_PDX_1) and 2 pancreatic (PA_PDX_1 and PA_PDX_2) patient-derived xenograft (PDX) models also reveals elevated CCL20 mRNA levels compared with non-CSCs isolated from the same line (E). CSCs for breast PDX models were identified as $Epcam^+$ $CD44^+$ $CD24^-$, and for pancreatic PDX models as $Epcam^+$ $CD44^+$ $CD24^+$, while PDX breast non-CSCs were $Epcam^+$ $CD44^-$ $CD24^-$, and pancreatic non-CSCs were $Epcam^-$ $CD44^-$ $CD24^-$. For panels A-C, n = 3 biological replicates/condition/cell line; ***, **, and * denote statistical significance of p<0.001, p<0.01, and p<0.05 respectively between spheres and monolayer culture as determined by t-test. For panels D and E, n= 2 technical replicates/condition/cell line. RE = relative expression of Ct values normalized to 18S ribosomal RNA and expressed compared to non-CSC levels.

**Figure 2** shows CCL20 secretion in sphere culture. Average ±SEM for CCL20 levels measured in conditioned media of sphere cultured cells harvested at day 4. Absolute CCL20 levels were measured by ELISA relative to a standard curve. N = 3 biological replicates/cell line.

**Figure 3A-3F** shows that CSCs directly co-localize with a CCL20+ phenotype. CSCs from monolayer culture of the Bxpc3 pancreatic model are identified as $Epcam^+$ $CD44^{high}$ $CD24^{high}$ by flow cytometry (A), while breast CSCs from the monolayer HCC1937 model are identified as $CD44^{high}$ $CD24^-$ (D). In both Bxpc3 (B) and HCC1937 (E), CSCs are found to have 1.5-fold greater CCL20 expression than the average of the total tumor cell population (total tumor) does as determined by median fluorescence intensity (Median: Comp-APC A) of APC-tagged anti-CCL20. Furthermore, in both Bxpc3 (C) and HCC1937 (F), CSCs, illustrated as black dots, appear to predominantly overlap with a $CCL20^+$ $CCR6^+$ population of cells as determined by multicolor flow cytometry. Scatter of the total tumor cells is illustrated as grey dots for comparison.

**Figure 4A-4F** illustrates CSC-enriched sphere culture causes a dramatic expansion of the $CCL20^+CCR6^+$ population. The frequency of $CCL20^+CCR6^+$ dramatically increases in the Bxpc3 (A, B) and Panc1 (C, D) pancreatic cancer models when cells are cultured under sphere forming conditions (B, D) compared with monolayer culture (A, C) as determined by multicolor flow cytometry. Furthermore, pancreatic CSCs identified from sphere culture as $Epcam^+CD44^+CD24^+$, illustrated as black dots, overlap predominantly with the $CCL20^+CCR6^+$ population in Bxpc3 (E) and Panc1 (F) models. Scatter of the total tumor cells is illustrated as grey dots for comparison.

**Figure 5A-5D** shows that addition of purified CCL20 to culture boosts the growth of CSC-enriched spheres, but not standard monolayer cultured cells. Bxpc3 pancreatic cancer (A), Aspc1 pancreatic cancer (B), and SUM159 breast cancer (C) models were stimulated with recombinant human CCL20 (rhCCL20) at 0, 25, 50, 100, and 200 pg/mL for 4 days in either sphere or monolayer culture, whereby CCL20 was dosed at the time of plating. BT549 cells (D) illustrate that the potentiation of CCL20 on sphere growth is seen with CCL20 from 2 separate commercial sources, R&D systems (CCL20 A) and Life Technologies (CCL20 B), both dosed at 50 pg/mL. The specificity of CCL20 in mediating this growth stimulation was confirmed when addition of an anti-CCL20 monoclonal antibody at 15 μg/mL nullified this effect for both CCL20 A and CCL20 B (D). n = 5 biological replicates/treatment/cell line. ***, **, and * denote statistical significance of p<0.001, p<0.01, and p<0.05 respectively between spheres and monolayer culture at matched concentrations of CCL20 as determined by t-test. ## and # denotes statistical significance of p<0.01 and p<0.05 respectively between basal and CCL20 stimulated spheres, and ^ denotes statistical significance of p<0.05 between CCL20 stimulated cells with and without addition of anti-CCL20, as determined by 1-way ANOVA with Tukey's multiple comparisons.

**Figure 6A-6D** shows that stimulation of cancer cells with CCL20 directly boosts the frequency of CSCs in culture. Addition of 200 pg/mL recombinant human CCL20 to cells in sphere culture directly increases the percentage of CSCs after 4 days in breast and pancreatic cancer models, seen in (A) MDAMB468 breast cancer cells characterized as $CD44^+$ $CD24^-$, (B) in Bxpc3 pancreatic cancer and (C) in Aspc1 pancreatic cancer cells, both characterized as $Epcam^{high}$ $CD44^{high}$ $CD24^{high}$. Bxpc3 spheres stimulated for 4 days with 200 pg/mL show a corresponding increase in the sternness genes, NANOG, SOX2, OCT3/4, BMI1, and EZH2 (D). Panels A-C, n = 3 biological replicates/treatment/cell line. * denotes statistical significance of p<0.05 between cells treated with or without CCL20 as determined by one-sided t-test. Panel D, n = 2 technical replicates/treatment; RE = relative expression of Ct values normalized to 18S ribosomal RNA and expressed compared to -CCL20 levels.

**Figure 7A-7C** shows stimulation of spheres with CCL20 induces the expression of sternness genes. Relative expression (RE) of sternness genes NANOG, SOX2, OCT3/4, EZH2, AND BMI1 in Hs578T (A), Panc1 (B), and SUM159 (C) spheres treated for 4 days with 100 pg/mL recombinant human CCL20 (+ CCL20) compared with untreated spheres (-CCL20). RE = relative expression of Ct values normalized to 18S ribosomal RNA and expressed compared to -CCL20 levels. N = 2 technical replicates/treatment/cell line.

**Figure 8A-8D** shows CCL20 regulates CSCs by signaling through its receptor CCR6. (A) Recombinant human CCL20 (rhCCL20) loses the ability to stimulate sphere growth by 4 days after treatment initiation in Lovo colorectal cancer cells following knockdown of CCR6 with 20nM siRNA, while cells transfected with non-target control siRNA (20nM) retained their growth response to CCL20. (B) $CCR6^+$ cells show an increase in sternness genes 48 hours after stimulation with CCL20 (100 pg/mL) compared with $CCR6^-$ cells isolated from the NCIH508 colorectal cancer model. (C-D) Inhibition of the CCL20-CCR6 axis, either by knockdown of CCL20 or CCR6 (20nM siRNA) resulted in a similar level of sphere growth inhibition of NCIH508 colorectal cancer cells (C) and Hs578T breast cancer cells (D) compared with control siRNA (20nM) treatment. Panel A, n = 5 biological replicates/condition. ***, **, and * denote statistical significance of p<0.001, p<0.01, and p<0.05 respectively between control siRNA and CCR6 siRNA transfected cells at matched concentrations of CCL20 as determined by t-test. Panel B, RE = relative expression of Ct values normalized to 18S ribosomal RNA and expressed compared to $CCR6^-$ levels. Panel C, n = 5 biological replicates/treatment. *** denotes statistical significance of p<0.001 from control siRNA, as determined by 1-way ANOVA with Tukey's multiple comparisons.

**Figure 9A-9E** shows treatment with a neutralizing monoclonal CCL20 antibody significantly reduces CSCs measured by sphere growth and flow cytometry. Sphere growth is reduced following a 4 day treatment with 10 $\mu$g/mL CCL20 IgG, compared with 10 $\mu$g/mL isotype control IgG treatment in (A) Hs578T breast cancer cells, (B) NCIH508 colorectal cancer cells, and (C) Panc 1 pancreatic cancer cells. A single dose 10 $\mu$g/mL CCL20 IgG treatment reduces the percentage of $Epcam^+CD44^+CD24^+$ pancreatic CSCs in Bxpc3 (D) and Panc1 (E) spheres compared with isotype control IgG treatment at the same 10 $\mu$g/mL dose. Panels A-C, n = 5 biological replicates/treatment/cell line. Panels D-E, n = 3 biological replicates/treatment/cell line. ***, **, and * denotes statistical significance of p<0.001, p<0.01, and p<0.05 between control IgG and CCL20 IgG treated cells, respectively, as determined by t-test.

**Figure 10** shows treatment with a neutralizing monoclonal CCL20 antibody significantly inhibits chemotactic migration of CD4+ Tcells towards CCL20. CD4+ Tcells were isolated from human healthy donor blood and allowed to migrate towards 10nM recombinant human CCL20 with 30 nM isotype IgG or anti-CCL20 IgG in transwell migration plates with a polycarbonate filter and 5 $\mu$M pores for 3 hours. n=3 biological replicates/treatment/donor. ** and *** denotes statistical significance of p<0.01 and p<0.001 between isotype IgG and CCL20 IgG, respectively, as determined by t-test.

**Figure 11A-11I** shows a multiparameter flow cytometric analysis of TR1 phenotype. 57% of live cells in the population stained positive for CCR6 (A). Of these CCR6+ cells, 93% were found to be CD3+/CD4+ (B). Of the CD3+/CD4+ cells, 94% were found to be CD25+ (C), and of these CD25+ cells, 85% were confirmed to be FOXP3+ (D). Positive vs. negative gating was determined by full-minus-one (FMO - ) staining whereby samples contained all the stains in the panel minus CD3 (E), CD4 (F), CD25 (G), FoxP3 (H), or CCR6 (I).

**Figure 12A-12C** shows that TR1 cells migrate towards CCL20 secreted by CSCs. Chemotactic attraction of TR1 cells towards 100 nM CCL20 was determined by a transwell migration assay compared with non-specific migration (no CCL20) and compared to inhibition of migration through the addition of neutralizing anti-CCL20 IgG (667 nM) (A). CCL20 concentration in CSC conditioned medium (CSC CM), generated from sphere culture of NCIH508 cells, was determined by ELISA. CCL20 levels in unused SCM media is plotted by comparison to ensure that CCL20 was produced from NCIH508 CSCs (B). Chemotactic attraction of TR1 cells towards CSC CM was also determined by a transwell migration assay compared with non-specific migration (no CCL20) and compared to inhibition of migration through the addition of neutralizing anti-CCL20 IgG (667nM) or isotype control IgG (667 nM). n= 4 replicates/treatment.

**Figure 13A-13E** shows gating controls for Bxpc3 and HCC1937 flow cytometry data from FIG. 3. Full-minus-one (FMO) stains were used to define positive vs. negative staining gates for each protein in multicolor flow cytometry experiments. Bxpc3 cells (top row, A-C) were stained with a 5-color panel for Epcam, CD24, CD44, CCL20, and CCR6, while HCC1937 cells (bottom row, D-E) were stained with a 4 color panel for all the above except Epcam. (A) Full-minus-Epcam (FMO - Epcam) (dashed line, grey shading) compared with the full-stained sample (solid line, white shading). (B, D) Full-minus-CD44 (FMO - CD44) is depicted in grey and full-minus-CD24 (FMO -CD24) is depicted in green. (C, E) Full-minus-CCL20 (FMO - CCL20) is depicted in black and full-minus-CCR6 (FMO -CCR6) is depicted in grey.

**Figure 14A-14B** shows gating controls for Bxpc3 and Panc1 flow cytometry data from FIG. 4. Monolayer and sphere cultured Bxpc3 and Panc1 cells were stained with a 5-color panel for Epcam, CD24, CD44, CCL20, and CCR6. For each experiment, gating controls were run on monolayer cultured cells. Full-minus-CCL20 (FMO -CCL20) is depicted in grey while full-minus-CCR6 (FMO -CCR6) is depicted in black, for both Bxpc3 (A) and Panc1 (B) monolayer cultured cells.

**Figure 15A-15D** shows gating controls for Bxpc3 and Panclflow cytometry data from FIGs. 9 D, E. Sphere cultured Bxpc3 (A, B) and Panc1 (C, D) cells were stained with a 5-color panel for Epcam, CD24, CD44, CCL20, and CCR6. For each experiment, gating controls were run on monolayer cultured cells. (A, C) Full-minus-Epcam (FMO - Epcam) (dashed line, grey shading) compared with the full-stained sample (solid line, white shading). (B, D) Full-minus-CD24 (FMO -CD24) is depicted in black while full-minus-CD44 (FMO - CD44) is depicted in grey.

**Figure 16A-16B** depicts how CCL20 IgG neutralizes CCL20-stimulated CCR6 pathway activity measured through (A) cAMP and (B) beta-arrestin activity. cAMP assay was performed using Ad293 cells overexpressing human CCR6, stimulated with 1 nM CCL20 and 4 mM forskolin, while the beta-arrestin assay was performed using CHOKI cells overexpressing human CCR6, stimulated with 6nM CCL20. n=2 technical repli cates/dose/assay.

**Figure 17A-17B** shows (A) Take rate of Panel tumors (% tumor-bearing animals) is reduced when Panc1 spheres are pre-treated with 10 ug/mL CCL20 IgG compared to Panc1 spheres that were pre-treated with 10 ug/mL isotype IgG for 4 days *in-vitro* prior to tumor inoculation, whereby tumor take is measured from the first instance of palpable tumor formation. (B) Pre-treating Panc1 spheres with 10 ug/mL CCL20 IgG prior to inoculation leads to significantly reduced tumor growth compared with isotype IgG pre-treated Panc1 spheres (10 ug/mL). For panel A, median time to tumor take was determined by Kaplan-Meier survival analysis. For panel B, **, and * denotes statistical significance of $p<0.01$ and $p<0.05$ between isotype IgG and CCL20 IgG treated tumors, respectively, as determined by 2-way ANOVA.

**Figure 18** shows anti-mouse CCL20 IgG (clone 114908) neutralizes CCL20-stimulated CCR6 pathway activity measured through beta-arrestin activity. The assay was performed using CHOKI cells overexpressing mouse CCR6, stimulated with 1.2 nM mouse CCL20.

**Figure 19** shows a representative flow cytometry diagram depicting gating and immune cell analysis in the spleen. Cells were first gated on cd45 as a pan-leukocyte marker. Next Tcells were identified by cd3 staining, dendritic cells by cdllc for total cells or cd11c/cd83 to separate mature and immature dendritic cells, and neutrophils/granulocyte myeloid derived suppressive cells (gMDSC) by cd11b/ly6g. Cd3+ Tcells were further subsetted for cd4 and cd8 staining, and cd4+ cells were then separated into foxp3+ $T_{regs}$ or rorgt+ $Th_{17}$ cells. All gates were determined by FMO controls and kept constant across all samples.

**Figure 20** shows a representative flow cytometry diagram depicting gating and immune cell analysis in the tumor. Cells were first gated on cd45 as a pan-leukocyte marker. Next Tcells were identified by cd3 staining, dendritic cells by cdllc for total cells or cd11c/cd83 to separate mature and immature dendritic cells, and neutrophils/granulocyte myeloid derived suppressive cells (gMDSC) by cd11b/ly6g. Cd3+ Tcells were further subsetted for cd4 and cd8 staining, and cd4+ cells were then separated into foxp3+ $T_{regs}$ or rorgt+ $Th_{17}$ cells. All gates were determined by FMO controls and kept constant across all samples.

**Figure 21A-21E** shows the frequency of immune cell populations in the spleens of 4T1 tumor-bearing mice dosed with 10 mg per kg anti-mouse CCL20 IgG or isotype control IgG, twice weekly for 4 doses. (A) cd3+ cd4+ foxp3+ $T_{reg}$ cells, (B) cd3+ cd4+ ror$\gamma$t+ $Th_{17}$ cells, (C) cd11b+ total dendritic cells, (D) cd11b+ cd83- immature dendritic cells, and (E) cd11b+ cd83+ mature dendritic cells. All cell frequencies are depicted as % of total leukocytes (cd45+ cells). n= 5 animals/group. Statistical significance was determined by two-sided student's t-test.

**Figure 22A-22E** shows the frequency of immune cell populations in the tumors of 4T1 tumor-bearing mice dosed with 10 mg per kg anti-mouse CCL20 IgG or isotype control IgG, twice weekly for 4 doses. (A) cd3+ cd4+ foxp3+ $T_{reg}$ cells, (B) cd3+ cd4+ ror$\gamma$t+ $Th_{17}$ cells, (C) cd11b+ total dendritic cells, (D) cd11b+ cd83- immature dendritic cells, and (E) cd11b+ cd83+ mature dendritic cells. All cell frequencies are depicted as % of total leukocytes (cd45+ cells). n= 5 animals/group. Statistical significance was determined by two-sided student's t-test.

## DETAILED DESCRIPTION

As described herein, the inventors provide description that demonstrates, for the first time, that CCL20 is overexpressed by and is functionally relevant for CSCs. While reports have referred to potential CCL20 expression in some cancers, no reports have identified and related CCL20 expression and function to any CSC phenotype.

The disclosure includes a description and overview of whole genome mRNA analysis on breast CSCs which surprisingly identified that changes in the expression of the gene CCL20 appeared to correlate directly with enrichment or inhibition of CSCs (van Vlerken et al., 2013). Chemokine (C-C motif) ligand 20 (CCL20) is also commonly referred to as macrophage inflammatory protein 3-alpha (MIP-3$\alpha$) or liver activation regulated chemokine (LARC) (Schutyser et al., 2003). CCL20 functions normally as a chemotactic factor for the recruitment of T-, B-, and immature dendritic-cells, and is produced predominantly by cells of the liver, lung, and gastrointestinal tract (Schutyser et al., 2003). Chemokine receptor 6 (CCR6) has been identified as the receptor for CCL20 (Baba et al., 1997) and, to date, is still the lone functional receptor identified for the CCL20 ligand. Despite its role in immune cell recruitment, several lines of evidence have hinted at an association for CCL20 in cancer. One report identified CCL20 as a factor contributing to pathogenesis of pancreatic cancer, whereby the authors concluded a direct effect for CCL20 in stimulating pancreatic cancer growth (Kleeff et al., 1999). While the early identification was associated with pancreatic cancer, the largest body of evidence to date links CCL20 expression with colorectal cancer (Ghadjar et al., 2009). In fact, recent work proposed high serum levels of CCL20 as a biomarker for poor prognosis in colorectal cancer (Iwata et al., 2013). The inventors have identified CCL20 in breast cancer models which has been corroborated by external evidence that shows CCL20 can directly affect migration and proliferation of breast cancer cells (Marsigliante et al., 2013). Although some reports suggest a direct autocrine effect of the CCL20-CCR6 axis on tumor cells, the majority of reports propose that CCL20 overexpression by tumor cells serves to promote immune cell recruitment. While the current disclosure implicates an autocrine function for CCL20 on CSCs, the disclosure also provides that CCL20 secretion by CSCs can aide in recruitment of tumor immune infiltrate. For example, evidence suggests that Tregs (Chen et al. 2011, Liu et al. 2011, Zhang et al. 2014) and $Th_{17}$ cells (Yu et al. 2015) in particular would be prone to tumor infiltration in response to CCL20 production, in addition to B-cells and dendritic cells that are also likely mobilized towards CCL20 produced by CSCs.

As disclosed herein, the inventors uncover a role for chemokine (C-C motif) ligand 20 (CCL20) in CSC growth and activity, a finding that is entirely novel to CSC biology. The disclosure provides illustrative embodiments demonstrating that CSCs of representative breast, pancreatic, and colorectal cancer models overexpress CCL20, and that in turn CCL20 can drive CSC self-renewal and proliferation by signaling through its receptor CCR6, also expressed on CSCs. Neutralizing CCL20 with a monoclonal antibody has a significant effect on CSC inhibition, supporting the great potential for therapies targeting this chemokine to reduce CSCs in the tumor.

It must be noted that, as used in this specification and the appended claims, the singular form "a", "an" and "the" include plural referents unless the context clearly dictates otherwise.

Unless defined otherwise, all technical and scientific terms used herein have the same meaning as commonly understood by one of ordinary skill in the art to which this invention is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, provide one of skill with a general dictionary of many of the terms used in this invention.

The terms "about" and "approximately" in the context of the present invention denote an interval of accuracy that a person skilled in the art will understand to still ensure the technical effect of the feature in question. The term typically encompasses a deviation from the indicated numerical value of about +/- 10%, or about +/-5%.

The determination of percent identity between two sequences is preferably accomplished using the mathematical algorithm of Karlin and Altschul (1993) Proc. Natl. Acad. Sci USA 90: 5873-5877. Such an algorithm is e.g. incorporated into the BLASTn and BLASTp programs of Altschul et al. (1990) J. Mol. Biol. 215: 403-410 available at the NCBI website. The determination of percent identity is preferably performed with the standard parameters of the BLASTn and BLASTp programs.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

As used herein, the terms "antibody" and "antibodies", also known as immunoglobulins, encompass monoclonal antibodies (including full-length monoclonal antibodies), polyclonal antibodies, multispecific antibodies formed from at least two different epitope binding fragments (e.g., multispecifc antibodies, e.g., PCT publication WO2009018386, PCT Application No. PCT/US2012/045229 (PCT publication WO2013006544)), biMabs, human antibodies, humanized antibodies, camelised antibodies, single-chain Fvs (scFv), single-chain antibodies, single domain antibodies, domain antibodies, Fab fragments, F(ab')2 fragments, antibody fragments that exhibit the desired biological activity (e.g. the antigen binding portion), disulfide-linked Fvs (dsFv), and anti-idiotypic (anti-Id) antibodies (including, e.g., anti-Id antibodies to antibodies of the disclosure), intrabodies, and epitope-binding fragments of any of the above. In particular, antibodies include immunoglobulin molecules and immunologically active fragments of immunoglobulin molecules, i.e., molecules that contain at least one antigen-binding site. Antibodies also include peptide fusions with antibodies or portions thereof such as a protein fused to an Fc domain. Immunoglobulin molecules can be of any isotype (e.g., IgG, IgE, IgM, IgD, IgA and IgY), subisotype (e.g., IgG1, IgG2, IgG3, IgG4, IgA1 and IgA2) or allotype (e.g., Gm, e.g., G1m(f, z, a or x), G2m(n), G3m(g, b, or c), Am, Em, and Km(1, 2 or 3)). Antibodies may be derived from any mammal, including, but not limited to, humans, monkeys, pigs, horses, rabbits, dogs, cats, mice, etc., or other animals such as birds (e.g. chickens).

The terms "chemokine receptor CCR6" or "CCR6 receptor", or "CCR6" and "Chemokine ligand 20" or "CCL20" as used herein refer to biological molecules (nucleic acid molecules and amino acid molecules) that are known in the art. One of ordinary skill in the art will be able to identify the polynucleotide and amino acid sequences of CCR6 receptor and CCL20, as well as any orthologous and splice variant isoforms of CCR6 and CCL20 from any sequence database (e.g. the NCBI database).

In some embodiments the terms "CCR6 receptor" or "CCL20" refer to a mammalian CCR6 receptor and CCL20, and in certain embodiments, refer to human CCR6 receptor and human CCL20. As noted above, the sequences of human CCR6 receptor are available from public sequence databases such as, for example, the NCBI database (accession number NM_004367.5 (transcript variant 1; SEQ ID NO:1, encoding SEQ ID NO:2) or NM_031409.3 (transcript variant 2; SEQ ID NO: 3, encoding SEQ ID NO:4). Similarly, the sequences of human CCL20 are publicly available (NCBI accession number NM_004591.2 (transcript variant 1; SEQ ID NO:5, encoding SEQ ID NO:6) or NM_001130046.1 (transcript variant 2; SEQ ID NO:7, encoding SEQ ID NO:8). Other orthologs and variants will be known and/or identifiable to one of skill in the art.

CCR6 is sometimes also referred to as CD196 or CD196 antigen. A number of other terms for CCR6 have been used in the art with varying frequency including, for example, CC-CKR-6, C-C- CKR-6, Chemokine (C-C Motif) Receptor 6, Chemokine (C-C) Receptor 6, C-C Chemokine Receptor Type 6, CKRL3, CKR-L3, Chemokine Receptor-Like 3, STRL22, CMKBR6, G Protein-Coupled Receptor 29, GPR29, Seven-Transmembrane Receptor, Lymphocyte 22, GPRCY4, GPR-CY4, DRY6, LARC Receptor, and BN-1.

Similarly, CCL20, or C-C Motif Chemokine 20, is also known as macrophage inflammatory protein-3$\alpha$ (MIP3 $\alpha$, MIP3-alpha, MIP3A, MIP3a, MIP-3a), liver and activation-regulated chemokine (LARC), CC Chemokine LARC, SCYA20, Small-inducible cytokine A20, Small Inducible Cytokine Subfamily A (Cys-Cys), Member 20, ST38, CKb4, Beta Chemokine Exodus-1, Beta-Chemokine Exodus-1, or exodus-1. As noted above, CCL20 is a 9 kDa CC-type chemokine, which is expressed constitutively at low levels by keratinocytes in various tissues (e.g., skin, intestinal mucosa, liver). While there is redundancy in human chemokine network, CCL20 is the unique chemokine ligand of its receptor, CCR6. CCL20 expression has been described in a variety of human neoplasms, including colorectal, lung, pancreatic and breast human adenocarcinomas, malignant glioma, leukemia, lymphoma and melanoma. To date and prior to the instant disclosure, however, the *in vivo* role of CCL20, in particular in the context of cancer and cancer therapy, has not been established.

### *A. Methods*

As used herein, the term "subject" is intended to include human and non-human animals, particularly mammals. Examples of subjects include human subjects for example a human patient having a disorder, e.g., a disorder described herein, such as cancer, or a normal subject. A "non-human animal" includes all vertebrates, e.g., non-mammals (such as chickens, amphibians, reptiles) and mammals, such as non-human primates, domesticated and/or agriculturally useful animals (such as sheep, dogs, cats, cows, pigs, etc.), and rodents (such as mice, rats, hamsters, guinea pigs, etc.). In particular embodiments, the subject is a human patient.

"Treatment" or "treat" refers to both therapeutic treatment and prophylactic or preventative measures. Those subjects in need of treatment include those already with the disorder as well as those prone to have the disorder or those in which the disorder is to be prevented. When used with reference to a disease or a subject in need of treatment the terms accordingly include, but are not limited to, halting or slowing of disease progression, remission of disease, prophylaxis of symptoms, reduction in disease and/or symptom severity, or reduction in disease length as compared to an untreated subject. In embodiments, the methods of treatment can abate one or more clinical indications of the particular disease being treated.

"Administration" or "administering," as used herein, refers to providing, contacting, and/or delivering a compound or compounds by any appropriate route to achieve the desired effect. Administration may include, but is not limited to, oral, sublingual, parenteral (e.g., intravenous, subcutaneous, intracutaneous, intramuscular, intraarticular, intraarterial, intrasynovial, intrasternal, intrathecal, intralesional or intracranial injection), transdermal, topical, buccal, rectal, vaginal, nasal, ophthalmic, via inhalation, and implants.

"Co-administered," as used herein, refers to simultaneous or sequential administration of multiple compounds or agents. A first compound or agent may be administered before, concurrently with, or after administration of a second compound or agent. The first compound or agent and the second compound or agent may be simultaneously or sequentially administered on the same day, or may be sequentially administered within 1 day, 2 days, 3 days, 4 days, 5 days, 6 days, 1 week, 2 weeks, 3 weeks or one month of each other. Suitably, compounds or agents are co-administered during the period in which each of the compounds or agents are exerting at least some physiological effect and/or has remaining efficacy.

"Contacting," as used herein as in "contacting a cell," refers to contacting a cell directly or indirectly in vitro, ex vivo, or in vivo (i.e. within a subject, such as a mammal, including humans, mice, rats, rabbits, cats, and dogs). Contacting a cell, which may also include "reacting" a cell with or "exposing" a cell to an inhibitor compound, can occur as a result of general administration of a compound or agent to a subject or addition or application of the inhibitor to a vessel containing a cell or tissue or a fluid containing a cell or tissue. Thus, contacting the cell with an inhibitor may refer to administration of an inhibitor to a subject, tissue, or region of a subject or tissue that comprises a cell (e.g., a CSC cell in a localized region or a system in a subject (e.g., lymphatic, circulatory, etc.)) and may not physically contact a target cell. Contacting encompasses the administration to a cell, tissue, mammal, subject, patient, or human. Further, contacting a cell includes adding an agent to a cell culture. Other suitable methods may include introducing or administering an agent to a cell, tissue, mammal, subject, or patient using appropriate procedures and routes of administration as discussed herein or otherwise known in the art.

*Detection Methods*

In some aspects and embodiments the methods disclosed herein provide for and/or comprise one or more methods useful in the detection, identification, and/or quantification of a cancer stem cell (CSC). In some embodiments the method comprises detecting, identifying, and/or quantifying the amount of CCL20 in a biological sample comprising CSCs. In some embodiments, the methods provide for diagnosis, prognosis, quantification, identification, and/or detection of the presence of a cancer stem cell (CSC) in a sample comprising cancer cells, the method can comprise one or more of:

contacting the sample with an agent that binds to a CCL20 nucleic acid sequence or a CCL20 amino acid sequence;
detecting the presence or absence of binding between the agent and the CCL20 nucleic acid sequence or the CCL20 amino acid sequence; and/or
identifying the presence of the CSC in the sample upon detection of binding between the agent and the CCL20 nucleic acid sequence or the CCL20 amino acid sequence.

In yet further embodiments, the method may comprise an agent comprising a detectable moiety. In some embodiments, the agent may comprise a nucleic acid sequence that hybridizes to at least a portion of the CCL20 nucleic acid sequence under stringent hybridization conditions. In some embodiments, the agent may comprise an antibody that specifically binds to at least a portion of the CCL20 amino acid sequence.

In addition to the methods described herein that are useful for detecting, identifying, and/or quantifying a CSC in a sample, these aspects and embodiments can also include any method and technique that is available to one of ordinary skill in the art. As non-limiting examples, CSCs may be identified, detected, and isolated using techniques including flow cytometry based on cell surface markers that are expressed in, or specific for particular CSCs; detection of side-population (SP) phenotypes by dye exclusion (e.g., Hoechst 33342, as disclosed in Moserle, L., et al., Cancer Res. (2008) 68; 5658-5668); ability to grow/proliferate as floating spheres in serum-free medium (e.g., disclosed herein as well as in Ryback, A.P., et al., Biochim. Biophys. Acta (2011) 1813; 683-694); and determination of particular enzymatic activity such as, for example, aldehyde dehydrogenase activity, and levels of polycomb markers including polycomb heterochromatin marker, $H3K27me^3$ levels, and polycomb group protein enhancer of zeste 2 (EZH2).

In some embodiments, for example, elevated aldehyde dehydrogenase (ALDH) expression and activity has been reported in cancer precursor cells of various lineages, (e.g., hematopoietic, mammary, endothelial, mesenchymal, neural) and techniques and commercially available kits (e.g., ALDEFLUOR™, Stemcell Technologies) may be used in connection with the various aspects and embodiments herein. Similarly, while there are currently no universally expressed cell surface markers for identifying CSCs, a number of surface markers have been associated with CSCs and associated tumor types, including, for example, ALDH, CD13, CD15, CD24, CD44, CD90, CD117, CD133, CD166, CD326, (see, e.g., Xia, P., Curr Stem Cell Res Ther. (2014) Mar; 9(2): 102-11; Shimamura, M., et al., Endocr. J., (2014) 61(5):481-90; Tirino, V., et al., FASEB J., (2013) Jan; 27(1):13-24).

In some embodiments, because of the lack of one or more universal surface markers for the detection or identification of a CSC in a sample, methods of detecting, identifying, and/or quantifying comprise cell culture techniques comprising sphere cultures and growth assays such as disclosed herein or otherwise known in the art.

***B. Inhibitors***

In embodiments, the inhibitor may be an "aptamer" which refers to a DNA, RNA or peptide aptamer having specificity for CCL20. A polynucleotide aptamer comprises anywhere from about 10 to about 300 nucleotides in length. Typically, an aptamer ranges from about 30 to about 100 nucleotides in length, and in some embodiments may range from about 10 to 60 nucleotides in length. Aptamers may be prepared by any known method, including synthetic, recombinant, and purification methods, and may be used alone or in combination with other aptamers specific for CCL20.

In some embodiments, the inhibitor may be an antibody that specifically binds CCL20. In some embodiments the antibody may be a monoclonal or polyclonal antibody, or a polyspecific antisera. In such embodiments, the antibody may also include antibody variants or fragments such as, for example, single chain antibodies, diabodies, minibodies, single chain Fv fragments (sc(Fv)), $sc(Fv)_2$ antibodies, Fab fragments, or $F(ab')_2$ fragments, as long as the variant or fragment retains specific binding properties to the target (e.g., CCL20).

In other embodiments, the inhibitor may be an antisense molecule comprising a polynucleotide which is complementary to at least a portion of CCL20 mRNA. In some embodiments, the antisense molecule is suitable for use in methods that inhibits translation of mRNA in a cell. The antisense molecule may comprise DNA, RNA, or both DNA and RNA, as well as chemically modified nucleic acids. Further, in some embodiments the antisense molecule may be single stranded or double stranded. An antisense molecule may comprise about 10 to about 500 nucleotides and, in some embodiments may have any number of lengths, for example, ranging from about 11 to about 200 nucleotides, about 12 to about 100 nucleotides, about 13 to about 75 nucleotides, about 14 to about 50 nucleotides, about 15 to about 40 nucleotides, about 16 to about 30 nucleotides, or about 17 to about 25 nucleotides. One of ordinary skill in the art will appreciate that these ranges represent illustrative embodiments.

In some embodiments, the inhibitor may comprise a siRNA molecule that reduces or inhibits the expression of CCL20. The siRNA molecule can, in some embodiments, be a single stranded or double stranded siRNA molecule that comprises a sequence capable of hybridizing to CCL20 mRNA, and induce RNA interference or another antisense mechanism that reduces or inhibits expression of protein. siRNA molecules may be of any sequence that allows the siRNA molecule to induce RNA interference resulting in reduction or inhibition of the expression of CCL20 protein. The siRNA molecule may, in some embodiments have a length of between 10 and 100, between 12 and 80, between 14 and 60, between 16 and 50, between 17 and 40. Suitably the siRNA has a length ranging from 18 to 30 nucleotides and, in certain embodiments between about 18 and about 26 nucleotides.

In embodiments comprising an inhibitory nucleic acid molecule, such inhibitors can bind to a target CCL20 nucleic acid sequence under stringent binding conditions. The terms "stringent conditions," "stringent binding conditions," or "stringent hybridization conditions" refers to conditions under which a polynucleotide will hybridize to a target sequence, to a detectably greater degree than other sequences (e.g., at least 2-fold over background). One non-limiting example of stringent conditions include those in which hybridization in 50% formamide, 1 M NaCl, 1% SDS at 37°C, and a wash in 0.1x SSC at 60°C to 65°C is performed.

Given the polynucleotide sequence of CCL20, an inhibitory nucleic acid molecule can be designed using motifs and targeted to a region that may be predicted to be effective for inhibitory activity, using standard techniques in the art.

### *C. Kits*

Another aspect of the disclosure relates to a kit. In one aspect, a kit comprises any of the inhibitors, reagents, compositions, or pharmaceutical compositions as described above, and instructions or a label directing appropriate use or administration. Optionally, a kit may also include one or more containers and/or a syringe or other device to facilitate delivery or use. The disclosure contemplates that all or any subset of the components for conducting research assays, diagnostic assays and/or for administering therapeutically effective amounts may be enclosed in the kit. Similarly, the kit may include instructions for making a conjugate by, for example forming a covalent bond between an inhibitor of the disclosure and a therapeutic or diagnostic moiety under suitable conditions to form a conjugate. By way of additional example, a kit for use in a therapeutic method of the disclosure may comprise a solution containing a pharmaceutical formulation of the inhibitor(s) of CCL20 or/and CCR6, or a lyophilized preparation of one or more inhibitors, and instructions for administering the composition to a patient in need thereof and/or for reconstituting the lyophilized product.

The present disclosure also encompasses a finished packaged and labeled pharmaceutical product. This article of manufacture includes the appropriate unit dosage form in an appropriate vessel or container such as a glass vial or other container that is hermetically sealed. In the case of dosage forms suitable for parenteral administration the active ingredient is sterile and suitable for administration as a particulate free solution. In certain aspects, the formulation is suitable for intravenous administration, such as for intravenous infusion to a human or animal.

In a specific aspect, the formulations of the disclosure are formulated in single dose vials as a sterile liquid. Exemplary containers include, but are not limited to, vials, bottles, pre-filled syringes, IV bags, blister packs (comprising one or more pills). Optionally associated with such container(s) can be a notice in the form prescribed by a governmental agency regulating the manufacture, use or sale of pharmaceuticals or biological products, which notice reflects approval by the agency of manufacture, use or sale for human diagnosis and/or administration.

As with any pharmaceutical product, the packaging material and container are designed to protect the stability of the product during storage and shipment. Further, the products of the disclosure include instructions for use or other informational material that advise the physician, technician or patient on how to appropriately prevent or treat the disease or disorder in question. In other words, the article of manufacture includes instruction means indicating or suggesting a dosing regimen including, but not limited to, actual doses, monitoring procedures, etc., and other monitoring information.

A kit for diagnostic assays may comprise a solution containing an inhibitor of CCL20 or alternatively or in addition, reagents for detecting the presence of CCL20. The various reagents may be labeled according to methods known in the art and described herein, including but not limited to labels such as small molecule fluorescent tags, proteins such as biotin, GFP or other fluorescent proteins, or epitope sequences such as his or myc. Similarly, primary antibodies used for detecting CCL20 may be included in the kit. Primary antibodies may be directed to sequences on CCL20 or to labels, tags, or epitopes with which the CCL20 may be labeled. Primary antibodies may, in turn, be labeled for detection, or, if further amplification of the signal is desired, the primary antibodies may be detected by secondary antibodies, which may also be included in the kit.

Kits for research use are also contemplated. Such kits may, for example, resemble kits intended for diagnostic or therapeutic uses but further include a label specifying that the kit and its use is restricted to research purposes only.

***Labels, conjugates and moieties***

The disclosure relates to methods, compositions, and kits that comprise reagents that may be conjugated to labels for the purposes of diagnostics and other assays wherein one or more target molecules may be bound and detected by such labeled reagent(s). Labels include, without limitation, a chromophore, a fluorophore, a fluorescent protein, a phosphorescent dye, a tandem dye, a particle, a hapten, an enzyme and a radioisotope.

In certain aspects, the reagents are conjugated to a fluorophore. The choice of the fluorophore attached to the reagent(s) will determine the absorption and fluorescence emission properties of the conjugated molecule. Physical properties of a fluorophore label that can be used include, but are not limited to, spectral characteristics (absorption, emission and stokes shift), fluorescence intensity, lifetime, polarization and photo-bleaching rate, or combination thereof. All of these physical properties can be used to distinguish one fluorophore from another, and thereby allow for multiplexed analysis. Other suitable properties of the fluorescent label may include cell permeability and low toxicity, for example if labeling of the target(s) is to be performed in a cell or an organism (e.g., a living animal).

In certain aspects, an enzyme is a label and is conjugated to a reagent. Enzymes may be suitable labels because amplification of the detectable signal can be obtained resulting in increased assay sensitivity. The enzyme itself does not produce a detectable response but functions to break down a substrate when it is contacted by an appropriate substrate such that the converted substrate produces a fluorescent, colorimetric or luminescent signal. Enzymes amplify the detectable signal because one enzyme on a labeling reagent can result in multiple substrates being converted to a detectable signal. The enzyme substrate is selected to yield the preferred measurable product, e.g. colorimetric, fluorescent or chemiluminescence. Such substrates are extensively used in the art and are well known by one skilled in the art and include for example, oxidoreductases such as horseradish peroxidase and a substrate such as 3,3'-diaminobenzidine (DAB); phosphatase enzymes such as an acid phosphatase, alkaline and a substrate such as 5-bromo-6-chloro-3-indolyl phosphate (BCIP); glycosidases, such as beta-galactosidase, beta-glucuronidase or beta-glucosidase and a substrate such as 5-bromo-4-chloro-3-indolyl beta-D-galactopyranoside (X-gal); additional enzymes include hydrolases such as cholinesterases and peptidases, oxidases such as glucose oxidase and cytochrome oxidases, and reductases for which suitable substrates are known.

Enzymes and their appropriate substrates that produce chemiluminescence are suitable for some assays. These include, but are not limited to, natural and recombinant forms of luciferases and aequorins. Chemiluminescence-producing substrates for phosphatases, glycosidases and oxidases such as those containing stable dioxetanes, luminol, isoluminol and acridinium esters are additionally useful.

In another aspect, haptens such as biotin, are also utilized as labels. Biotin is useful because it can function in an enzyme system to further amplify the detectable signal, and it can function as a tag to be used in affinity chromatography for isolation purposes. For detection purposes, an enzyme conjugate that has affinity for biotin is used, such as avidin-HRP. Subsequently a peroxidase substrate is added to produce a detectable signal.

Haptens also include hormones, naturally occurring and synthetic drugs, pollutants, allergens, affector molecules, growth factors, chemokines, cytokines, lymphokines, amino acids, peptides, chemical intermediates, nucleotides and the like.

In certain aspects, fluorescent proteins may be conjugated to the reagent(s) as a label. Examples of fluorescent proteins include green fluorescent protein (GFP) and the phycobiliproteins and the derivatives thereof. The fluorescent proteins, especially phycobiliprotein, are particularly useful for creating tandem dye labeled labeling reagents. These tandem dyes comprise a fluorescent protein and a fluorophore for the purposes of obtaining a larger stokes shift wherein the emission spectra is farther shifted from the wavelength of the fluorescent protein's absorption spectra.

In certain aspects, the label is a radioactive isotope. Examples of suitable radioactive materials include, but are not limited to, iodine ($^{121}$I, $^{123}$I, $^{125}$I, $^{131}$I), carbon ($^{14}$C), sulfur ($^{35}$S), tritium ($^{3}$H), indium ($^{111}$In $^{112}$In, $^{113}$mIn, $^{115}$mIn), technetium ($^{99}$Tc, $^{99}$mTc) thallium ($^{201}$Ti), gallium ($^{68}$Ga, $^{67}$Ga), palladium ($^{103}$Pd), molybdenum ($^{99}$Mo), xenon ($^{135}$Xe), fluorine ($^{18}$F), $^{153}$SM, $^{177}$Lu, $^{159}$Gd, $^{149}$Pm, $^{140}$La, $^{175}$Yb, $^{166}$Ho, $^{90}$y, $^{47}$Sc, $^{186}$Re, $^{188}$Re,$^{142}$Pr, $^{105}$ Rh and $^{97}$Ru.

**EXAMPLES**

MATERIALS & METHODS

*Monolayer and sphere culture.* For cell culture experiments, a panel of eight breast cancer cell lines spanning various breast cancer subtypes, MDAMB468, HCC1937, SUM159PT, MCF7, BT549, BT474, T47D, and Hs578T, a panel of four pancreatic cancer cell lines, Aspc1, Bxpc3, HPAC, and Panc1, and a panel of seven colorectal cancer cell lines, LS411N, SW1417, NCI-H508, Colo205, HT29, HCT116, Lovo, were maintained in humidified incubators at 37°C, 5% $CO_2$, in suppliers recommended media. All cell lines were originally obtained from ATCC (Manassas, VA), with the exception of SUM159PT, which was obtained from Asterand (Detroit, MI).

For standard monolayer tissue culture, cells were harvested with 0.25% Trypsin-EDTA (Life Technologies, Grand Island, NY), washed three times in Hank's Balanced Salt Solution (HBSS, Life Technologies), and plated in full serum media to tissue culture treated plates. For sphere culture, cells were harvested with 0.25% Trypsin-EDTA, washed three times in Hank's Balanced Salt Solution, and plated in serum-free defined medium, supplemented with epidermal growth factor (EGF), basic fibroblast growth factor (bFGF), insulin, bovine serum albumin (BSA), and Knockout serum (Life Technologies), to ultra-low attachment tissue culture plates (Corning).

Cell growth in monolayer assays were read after a 4 day incubation period by CellTiter glo® (CTG) luminescent cell viability assay (Promega, Madison, WI) according to suppliers recommendation. Sphere growth assays were read after a 4 day incubation period by direct counting of sphere colonies using High Content Imaging (Arrayscan VTI, Thermo Scientific, Waltham, MA), as well as by CTG assay.

*CCR6-based cell isolation.* CCR6-positive ($CCR6^+$) were separated from CCR6-negative ($CCR6^-$) cells in cell lines by magnetic bead separation. A tetrameric antibody complex (TAC) positive selection system was developed by complexing a mouse-anti-human CCR6 monoclonal antibody (BD Biosciences, San Jose, CA) with the "Do-It-Yourself' EasySep Selection kit (Stem Cell Technologies, Vancouver, BC, Canada). Use of this positive selection kit according to suppliers recommended protocol captured $CCR6^+$ cells by magnetic bead isolation, while $CCR6^-$ cells were retained in the unbound supernatant. Isolated $CCR6^+$ and $CCR6^-$ cells were then plated to monolayer culture and stimulated with recombinant human CCL20 (rhCCL20) as described in other sections.

*Enzyme-Linked ImmunoSorbent Assay (ELISA).* CCL20 levels secreted into conditioned medium of cultured cells were measured at day 4 of culture using a commercially obtained sandwich ELISA (R&D systems, Minneapolis, MN) according to supplier's protocol. CCL20 levels were directly measured in picogram/milliliter (pg/mL), interpolated from a standard curve. Secreted CCL20 levels were normalized to and reported as X fold-change compared to monolayer cultured cells from the same line.

*CCL20 stimulation and inhibition.* Commercially obtained recombinant human CCL20 (rhCCL20 or CCL20) sourced from R&D systems was used for all studies, with exception of data depicted in **FIG. 5D** where recombinant human CCL20 was also obtained from Life Technologies for comparison. Isotype control and anti-CCL20 monoclonal antibodies were also commercially sourced (R&D systems). All materials were reconstituted and stored according to manufacturer's recommendation.

For stimulation and inhibition studies, cells were plated for monolayer or sphere assay as described above. At the time of plating, cells were treated with rhCCL20 or antibody treatments at doses described in the experiments and results. For **FIG. 8A,** cells were transfected with 20nM CCR6 siRNA or non-target control siRNA (pooled constructs, obtained from Santa Cruz Biotechnology, Santa Cruz, CA) for 48 hours prior to addition of rhCCL20, while for **FIG. 8C, 8D,** cells were transfected with 20nM CCL20, CCR6, or non-target control siRNA (pooled constructs, Santa Cruz Biotechnology) at the time of plating, concomitant with antibody treatment of the cells. Transfection of siRNA was performed using lipid (Lipofectamine RNAimax (Life Technologies)) as the carrier, and complexed to siRNA at a ratio of 1:2.5 siRNA:lipid. All treatments ran for 4 days undisturbed, after which cells were transferred and prepared for monolayer/sphere assays, flow cytometry analysis, or RNA extraction as described in previous or later sections.

*Care and use of patient-derived Xenograft animal models.* All animals were housed under 12:12 hour light:dark cycle, with *ad libitum* access to water and chow, and allowed to acclimatize for at least one week prior to study initiation. All animal studies were approved by MedImmune's Institutional Animal Care and Use Committee.

The BR_PDX_1 and PA_PDX_1 models were obtained from Jackson Laboratories as female NSG mice bearing subcutaneous primary human pancreatic tumor xenografts at passage P1 (PA_PDX_1) or passage P2 (BR_PDX_1). The PA_PDX_2 model was obtained from Asterand as primary human pancreatic adenocarcinoma resections. All samples were obtained under informed patient consent, in accordance with their respective IRBs, which we reviewed and approved according to MedImmune's Human Biological Sample Acquisition policy.

All tumor material was propagated by subcutaneous implantation of tumor chunks into the hind flank of six to eight week old female NSG mice (*NOD.*Cg-*Prkdc^scid^ Il2rg^tm1Wjl^*/SzJ*,* Jackson Laboratories) or female Beige Nude XID mice ($Lyst^{bg}Foxn1^{nu}Btk^{xid}$, Envigo) under sterile conditions. Tumors were passaged by this methodology for up to passage 6 (P6), whereby passage 0 (P0) is considered the first xenograft established from the patient biopsy.

When tumors reached approximately $750mm^3$, they were harvested, washed in Hank's Balanced Salt Solution (HBSS), minced with a sterile razor-blade, and dissociated to single cell suspension in 20U/mL collagenase III (Worthington Biochemical). Dissociated tumor cells were used in fluorescence activated cell sorting as described below.

For the *in vivo* tumorigenicity study, Panc1 cells were cultured to sphere forming conditions, under treatment with 10 μg/mL CCL20 IgG or isotype IgG as control started at the time of plating. Following a 4 day culture, spheres were dissociated in StemPro Accutase to single cell suspension. Dissociated sphere cells were mixed 1:1 with Matrigel GFR (Corning) and injected subcutaneously into the hind flank of 6-8 week old female nu/nu mice (*Hsd:Athymic Nude-*

*$Foxn1^{nu}$,* Envigo) at 30,000 cells per mouse. Tumor volume was monitored and measured over time, whereby time-to-tumor-take was recorded as the first observation of a palpable tumor.

*Flow cytometry and fluorescence activated cell sorting (FACS).* Samples were prepared for flow cytometry experiments by incubating live cells with Golgistop (BD Biosciences) for 1 hour at 37°C to arrest CCL20 secretion. Following this, cells were harvested with an accutase cell dissociation solution (Life Technologies), washed and resuspended at approximately $2.5x10^6$ cells per mL into Hank's Balanced Salt Solution (HBSS). Cells were stained with a multicolor panel consisting of all or several of the following antibodies: anti-human CCL20-APC (R&D systems), anti-human CCR6-BV605, anti-human CD24-FITC, anti-human CD44-PE-Cy7, and anti-human Epcam-PerCP-Cy5.5 or Epcam-BV421 (all from BD Biosciences). Primary tumor xenografts were additionally stained with anti-mouse H2Kd-PE antibody (BD Biosciences) to allow all tumor-associated mouse endothelial and stromal cells to be excluded from subsequent analyses. In experiments containing CCL20 staining, cells were stained for all surface receptors prior to fixing and permeabilizing them for anti-CCL20 staining. Single staining controls were used to define compensation matrices, and full-minus-one staining controls were used to define gates. For cytometry experiments on live cells, DAPI (Invitrogen) was added to each sample at 30μM to gate out dead cells. Standard flow cytometry experiments were run on an LSRII with 4-laser configuration, while cell sorting experiments were run on a FACSAriaII (BD Biosciences). FACS gating was determined by fluorescence-minus-one (FMO) staining as shown in **FIG. 13, FIG. 14,** and **FIG. 15.**

*Quantitative RT-PCR.* All samples were processed for RNA extraction using the Ambion RNAaqueous kit (Applied Biosystems). RNA yield and quality was determined by UV spectrophotometry at 260/280nm. From 10-100 nanograms of mRNA of each sample was subjected to reverse transcription PCR (RT-PCR) at 42°C in the presence of random primer oligodeoxyribonucleotides (Life Techologies) and Superscript III reverse transcriptase (Life Technologies). Following RT-PCR, the 20ng cDNA product was pre-amplified for 14 cycles using pooled Taqman gene expression assays (Life Technologies), each at 0.2x concentration, for the following genes:

Table **1.**

| **Gene symbol** | **Taqman assay ID** |
|---|---|
| 18S rRNA | 4310893E |
| SOX2 | Hs01053049_s1 |
| NANOG | Hs02387400_g1 |
| OCT3/4 (POUF1) | Hs04195369_s1 |
| BMI1 | Hs00180411_m1 |
| EZH2 | Hs01016789_m1 |
| CCL20 | Hs01011368_ml |
| CCR6 | Hs01890706_s1 |

Preamplified material for each sample was diluted 20-fold in deionized water (DI-$H_2O$) and subjected to quantitiative PCR (qPCR) analysis using the above listed primes in technical replicates on a 7900HT Thermocycler (Life Technologies) according to supplier's recommended protocol.

Ct values were obtained from the qPCR run using SDS 2.4 software (Life Technologies), and converted to Relative Expression (RE) values by the comparative Ct method, using the formula RE= $2^{-\Delta\Delta Ct}$, where

$$\Delta Ct = \text{normalized Ct} = \text{gene Ct} - \text{18S Ct}$$

$$\Delta\Delta Ct = \text{normalized sample Ct} - \text{normalized control Ct}$$

An average ± standard error of the mean was determined for RE values amongst technical replicates, and subsequently plotted as the final data representation.

*TR1 culture and flow cytometry analysis.* Human naive CD4+ Tcells were freshly isolated from healthy human donor blood using the naive CD4 Tcell isolation kit II according to suppliers protocol (Miltenyi Biotech) and skewed in culture towards TR1 cells according to protocol described in Voo, *et al.* All human donor blood was obtained under informed consent, and used/disposed according to Astrazeneca's Human Biological Sample policy.

At day 8, TR1 cells were collected from culture, washed in PBS and stained for flow cytometry analysis to confirm a Treg marker phenotype. Cells were resuspended in PBS+2% FBS (Life Technologies) to a concentration of 0.5x$10^6$cells/0.1mL and stained with a multicolor antibody panel consisting of CD3-APC-Cy7, CD25-BV711 (both obtained from Biolegend), CD4-FITC, FOXP3-PE, and CCR6-DL647 (all obtained from BD Biosciences) at suppliers recommended concentrations. Prior to cell surface staining, cells were treated with Zombie-UV (Biolegend) according to supplier's protocol to gate out dead cells from the analysis. Following this, live cells were stained for all cell surface markers for 15 min. on ice. Stained cells were then washed twice with PBS+2% FBS and fixed using BD cytofix/cytoperm kit (BD Biosciences) according to recommended protocol. Following the fixation step, cells were stained for intracellular FOXP3 for 15 min. on ice, followed by an additional 2 washes in PBS+2% FBS. Flow cytometry analysis was performed on a BD Fortessa and analyzed by FlowJo software. Multicolor compensation was performed using compensation beads (eBiosciences), stained and analyzed in parallel to the cells, and Full-minus-one (FMO) controls were set up for each color to determine positive/negative gating.

*TR1 chemotaxis assay.* NCIH508 colon cancer cells were plated to ultra-low attachment plates (Corning) in serum-free defined media as described above to promote CSC-driven sphere growth. After 4 days of undisturbed culture, conditioned medium (CM) from the cells was collected, 0.2μM filtered, and stored at 4°C until further use. CCL20 concentration in the media was determined by ELISA as described above. This CSC CM was used within a week of harvesting for chemotaxis assays.

TR1 cells harvested at day 8 of culture were washed in PBS and resuspended to either PBS+2% FBS or serum free defined media (SCM) at 0.4x$10^6$ cells/mL. Isolated CCL20 (R&D systems) was prepared at 100nM concentration in PBS+2% FBS. Anti-CCL20 monoclonal antibody or isotype control antibody (both from R&D systems) was added to either isolated CCL20 or CSC CM at 667nM final concentration and allowed to incubate for 30 min. at room temperature. Prepared treatments were transferred to the receiving wells of a transwell migration plate with 8 μM pores (Neuroprobe) at n=4 wells/treatment. TR1 cells were plated on top of the filter plate according to suppliers protocol at 50,000 cells/well in matched medium (PBS+2% FBS for isolated CCL20 treatments, and SCM for CSC CM treatments). Plates were allowed to incubate for 60 min. at 37°C, after which non-migrated cells were blotted off the filter plate, and the filter was gently washed with PBS. Plates were centrifuged at 200rpm for 2 min. to collect migrated cells in the receiving wells. Numbers of migrated cells were determined by cell titer glo assay (Promega) according to suppliers protocol, whereby a standard curve of cell number vs. relative luminescence (RLU) was used to convert RLU to % of TR1 cells migrated. Statistical significance was determined by 2-tailed student's T-test between treatment and control, whereby n=4 replicates/treatment.

*Beta-arrestin and cAMP pathway assays.* Beta-arrestin recruitment was measured as a reporter for both human and mouse CCL20-CCR6 pathway activation using the PathHunter® eXpress CCR6 CHO-K1 β-Arrestin GPCR Assay (DiscoverX), whereby cells stably expressed mouse CCR6 for the mouse pathway assay and human CCR6 for the human pathway assay. Assays were run according to supplier's protocol with the adjustment that cells were stimulated with 6 nM recombinant human CCL20 (R&D Systems) or 1.2 nM recombinant mouse CCL20 (R&D systems) for 90 min at 37°C. Changes in cAMP levels were additionally measured in a separate assay as a reporter for human CCL20-CCR6 pathway activation using the LANCE cAMP assay kit (Perkin Elmer) according to supplier's protocol, using Ad293 cells stably transfected with human CCR6. In this assay, cells were stimulated with 4 μM forskolin (Sigma Aldrich) in combination with 1nM recombinant CCL20 for 60 min at room temperature. In both assays, pathway neutralization was measured following treatment with CCL20 IgG or isotype control IgG (both R&D systems).

*In-vivo syngeneic mouse models.* 4T1 mouse breast carcinoma was xenografted subcutaneously into the hind flank of 4-6 week old female Balb/c mice (*BALB/cAnNHsd,* Envigo). When tumors reached approximately 50-100$mm^3$, mice began receiving treatment with either an anti-mouse CCL20 monoclonal antibody (clone 114908) or an isotype control antibody (both R&D systems), dosed at 10mg per kg i.p. twice weekly for 4 consecutive doses. Mice were humanely euthanized at 24 hours following the final dose and spleens and tumors were collected for analysis. Both spleen and tumor tissue was digested to single cell suspension and resulting cells were cryopreserved in Cryostor CS5 (Sigma-Aldrich) prior to flow cytometric analysis of immune cells.

*Mouse immune cell flow cytometric analysis.* Cryopreserved spleen and tumor cells from mice treated with either isotype or anti-mouse CCL20 monoclonal antibody were thawed at 37°C, washed, and stained for mouse immune cell analysis using the following antibody panel: anti cd45-BV21, anti cd4-BV711, anti cd11b-BV510 (all obtained from BD Biosciences), anti cd3-FITC, anti cd8a-APC-Cy7, anti foxp3-PerCP-Cy5.5, anti roryt-PE (all obtained from eBiosciences), anti cd11c-BV605, anti-cd83-PE-Cy7, anti ly6g-BV785 (all obtained from Biolegend). Live cells were first blocked with 4% mouse serum (Jackson Immunoresearch), subsequently stained for live/dead cells using Zombie-UV (Biolegend), then stained for extracellular antigens (all targets except for foxp3 and roryt). Following this, cells were washed and fixed and permeabilized using a Cytofix/Cytoperm kit (BD Biosciences) prior to staining the cells with antibodies against intracellular antigens foxp3 and roryt. Flow cytometry was run on a BD Fortessa equipped with 355nm, 405nm, 488nm, 532nm, and 640nm laser excitation, and analyzed using flowjo V10 software. Statistical significance was determined by 2-tailed student's T-test between treatment and control, whereby n=5 mice/treatment/tissue type. Data points that fell beyond 3x standard error of the mean were identified as outliers and removed from analysis.

*Human CD4+ Tcell chemotaxis assay.* CD4+ Tcells were isolated from healthy human donor blood by negative selection using the Rosettesep CD4+ Tcell enrichment kit (Stemcell Technologies). Cells were washed in PBS and resuspended to PBS+2% FBS. Isolated CCL20 (R&D systems) was prepared at 10 nM concentration in PBS+2% FBS. Anti-CCL20 monoclonal antibody or isotype control antibody (both from R&D Systems) was added to isolated CCL20 at 30 nM final concentration and allowed to incubate for 30 min. at room temperature. Prepared treatments were transferred to the receiving wells of a transwell migration plate with 5 $\mu$M pores (Corning) at n=3 wells/treatment, and CD4+ Tcells were loaded into the insert over a polycarbonate filter at 50,000 cells/well in matched medium (PBS+2% FBS), and plates were allowed to incubate for 3 hours at 37°C. Numbers of migrated cells were determined by cell titer glo assay (Promega) according to supplier's protocol, whereby a standard curve of cell number vs. relative luminescence (RLU) was used to convert RLU to % of CD4+ Tcells migrated. Statistical significance was determined by 2-tailed student's T-test between treatment and control, whereby n=3 replicates/treatment/donor.

**EXAMPLE 1: Expression of CCL20 in cancer stem cells in culture**

As discussed above, although CCL20 is known as a chemo-attractant for the recruitment of immune cells, the results discussed herein demonstrate that CCL20 is a factor produced by CSCs in cancer cell lines, some of which are exemplified herein, including breast, pancreatic, and colorectal cancers. In tissue culture, the majority of cell lines are found to profoundly increase secretion of CCL20 when cultured under CSC-enriching sphere conditions relative to monolayer culture conditions (**FIGs. 1A-1C**). As Figure 1 shows, sphere culture conditions resulted in a significant increase in CCL20 secretion when compared to monolayer culture in many of the breast cancer lines **(FIG. 1A),** pancreatic cancer lines (**FIG. 1B**), and colorectal cancer lines **(FIG. 1C)** that were tested. Typical levels of CCL20 that were secreted by cells under sphere culture conditions ranged from about 1 to about 1000 pg/mL, whereby cell lines that lacked a CCL20 increase in sphere culture generally had low or undetectable protein levels of at or below 1 pg/mL **(FIG.2).**

To confirm that the sphere-associated increase in CCL20 levels are attributable to CSCs, fluorescence activated cell sorting (FACS) was used to isolate CSCs and non-CSCs from breast and pancreatic cancer lines, in order to measure the levels of CCL20 mRNA in each cell population. Isolated $CD44^{high/+}$ $CD24^{low/-}$ CSCs from two breast cell lines (MDAMB468 and HCC1937) have dramatically elevated CCL20 mRNA levels compared with $CD44^{low/-}$ $CD24^{low/-}$ non-CSCs isolated from the same lines (**FIG**. **1D**). CSCs from MDAMB468 and HCC1937 cells produce 23-fold and 10-fold higher CCL20 transcript than non-CSCs, respectively (RE for CSCs vs. non-CSCs = 23.3$\pm$16 vs. 1.0$\pm$0.2 in MDAMB468 and 9.3$\pm$4.6 vs.0.9$\pm$0.3 in HCC1937). To assure clinical relevance of this observation, CCL20 transcript levels were also measured in FACS isolated CSCs versus non-CSCs from primary patient derived tumor xenografts (PDX). Tumors from two pancreatic cancer (PA_PDX_1 and PA_PDX_2) and one breast cancer (BR_PDX_1) PDX model were similarly FACS sorted to isolate $Epcam^+$ $CD44^+$ $CD24^+$ CSCs along with $Epcam^-$ $CD44^-$ $CD24^-$ non-CSCs for pancreatic cancer, and $Epcam^+$ $CD44^+$ $CD24^-$ CSCs along with $Epcam^+$ $CD44^-$ $CD24^-$ non-CSCs for breast cancer. As shown in **FIG. 1E,** all three patient xenograft models showed a significant increase in CCL20 mRNA levels in isolated CSCs over non-CSCs, with relative expression for CSCs vs. non-CSCs = 71.8$\pm$41.6 vs. 1.2$\pm$0.6 (for BR_PDX_1), 48.8$\pm$8.6 vs. 1.0$\pm$0.0 (for PA_PDX_1), and 8.6$\pm$1.3 vs. 1.0$\pm$0.0 (for PA_PDX_2).

Next, we confirmed by flow cytometry that CSCs indeed are marked by elevated CCL20 levels compared to the rest of the tumor cell population. While CSCs are commonly identified as $Epcam^+CD44^+CD24^+$ in pancreatic cancer and $CD44^+CD24^-$ in breast cancer, we find that the stem cell phenotype, as determined by tumorigenicity, sphere formation, and sternness gene expression often tracks better in monolayer cell line models with high expression of CD44 and/or CD24 (thus $Epcam^+CD44^{high}CD24^{high}$ in monolayer cultured pancreatic cancer and $CD44^{high}CD24^-$ in monolayer cultured breast cancer). By this identification, we found that CSCs from the Bxpc3 pancreatic model **(FIG. 3A)** and from the HCC1937 breast cancer model **(FIG. 3D)** both showed a 1.5-fold increased level of CCL20 median fluorescence intensity (MFI) over the total tumor cell population. Bxpc3 CSCs **(FIG.** 3B) exhibited a shift in MFI of 189, while the total tumor cell population averaged a CCL20 MFI of 124. Similarly, HCC1937 CSCs **(FIG. 3E)** also showed an increased shift in CCL20 MFI from 243 in the total tumor cells to 361 specifically for the $CD44^{high}CD24^-$ CSCs. This data corroborates the findings and results depicted in **FIG. 1** which show that elevated CCL20 levels associate predominantly with CSC-enriched spheres and isolated CSCs. CSCs gating was determined by full-minus one staining as depicted in **FIG. 13.**

**EXAMPLE 2: Association of CCR6 and co-localization with CCL20 in cancer stem cells**

Interestingly, when we overlaid the CSC populations with CCL20/CCR6 staining for the tumor cells, we found that the majority of CSCs from both Bxpc3 and HCC1937 lines appear to co-localize with cells that are double positive for both the ligand and the receptor, ie. $CCL20^+CCR6^+$ **(FIGs. 3C & 3E).** Strikingly, we discovered that sphere culture dramatically increases this double positive $CCL20^+CCR6^+$ population over monolayer culture. Figure 4 illustrates this effect in Bxpc3 and Panc1 pancreatic cancer models. When cultured in standard monolayer, a small fraction of cells in both models are identified as $CCL20^+CCR6^+$ **(FIGs. 4A & 4C,** and **FIG 13** for gating controls). However, when cultured as spheres, the majority of cells in both models stain positive for both CCL20 and CCR6 **(FIGs. 4B & 4D).** In the Bxpc3 model, this $CCL20^+CCR6^+$ population increases from 3.76% **(FIG. 4A)** as monolayer to 71.2% **(FIG. 4B)** in spheres. Similarly, Panc1 cells cultured as monolayer have 5.87% $CCL20^+CCR6^+$ cells **(FIG. 4C),** a population that is boosted to 83.5% in sphere culture **(FIG. 4D).** Interestingly, when we gate on the $Epcam^+CD44^+CD24^+$ cells in these spheres, they again overlay almost completely with the $CCL20^+CCR6^+$ population in Bxpc3 **(FIG. 4E)** and Panc1 **(FIG. 4F)** spheres. These observations suggested that CSCs may have an autocrine dependence on the CCL20-CCR6 axis.

**EXAMPLE 3: Correlation of CCL20 levels and cancer stem cell activity**

Following the confirmation that CCL20 is indeed overexpressed by and associated with the CSC population, we sought to confirm the phenotypic relevance of this molecule to CSC activity. **FIG. 5** illustrates how exogenous addition of purified CCL20 boosts sphere growth, which serves as a model for CSC-driven tumorigenicty. The addition of recombinant human CCL20 (rhCCL20) to the CSC culture significantly stimulated sphere growth at day 4 in a dose-dependent manner in three models of cancer, Aspc1 **(FIG. 5A),** Bxpc3 **(FIG. 5B),** and SUM159 **(FIG. 5C).** While the addition of CCL20 causes a dose-dependent increase in sphere growth, the ligand shows no effect on stimulating growth of cells in standard monolayer culture **(FIGs. 5A-5C).** This observation tends to suggest that the effect of CCL20 is directed specifically toward CSCs. The specificity of this effect was confirmed in **FIG. 5D,** which illustrates that a 50 pg/mL dose of CCL20 obtained from two separate commercial sources (CCL20 A and CCL20 B) stimulates sphere growth of BT549 breast cancer cells to the same extent. For example, CCL20 A stimulated growth to 333.3$\pm$ 64.9% compared with 100.0$\pm$16.7% for basal cells, while CCL20 B stimulated growth to 300.0$\pm$54.6% over basal ($p<0.01$ and $p<0.05$, respectively, when compared with basal sphere growth). Moreover, this stimulatory effect can be neutralized or nullified upon addition of anti-CCL20 monoclonal antibody (15 μg/mL), whereby despite the presence of the same 50 pg/mL CCL20 A or CCL20 B, sphere growth was maximally 154$\pm$16.9% for CCL20 A + anti-CCL20 antibody and 125.0$\pm$16.1% for CCL20 B + anti-CCL20 antibody *(see,* **FIG. 5D;** $p<0.05$ compared to CCL20-treated spheres without anti-CCL20 antibody).

Overall the specificity of this response confirms that CCL20 indeed mediates phenotypic CSC activity. However, since sphere growth is an indirect measure of CSC activity, we sought to confirm that CCL20, as an isolated factor, increases CSCs directly. In brief, spheres treated with or without 200 pg/mL CCL20 for 4 days were subjected to flow cytometry analysis to directly measure CCL20-driven increases in the CSC population. **FIG. 6** demonstrates that CCL20 treatment significantly increases the percentage of $CD44^+CD24^-$ breast CSCs from 0.9$\pm$ 0.1% (without CCL20) to 1.3$\pm$ 0.1% (with CCL20) in MDAMB468 cells ($p<0.05$) **(FIG. 6A).** Similarly, Bxpc3 **(FIG. 6B)** and Aspc1 **(FIG. 6C)** pancreatic cancer cells show significant increases in their $Epcam^+CD44^{high}CD24^{high}$ CSC frequencies following CCL20 stimulation, from 0.9$\pm$0.1% to 1.2$\pm$0.1% in Bxpc3 and from 2.7$\pm$0.1% to 3.6$\pm$0.4% in Aspc1 ($p<0.05$ in both cell types). In addition to sphere growth and flow cytometry we, and others, have found that a panel of five sternness-related genes also tracks well with the CSC phenotype. Expression of the transcription factors NANOG, SOX2, and OCT3/4, which are known to be associated with pluripotency, increases and decreases with corresponding increases and decreases in the CSC population. Transcript expression of polycomb proteins EZH2 and BMI1 both relate similarly well with changes in the CSC population. **FIG. 6D** shows that addition of 200 pg/mL CCL20 to Bxpc3 spheres indeed increases expression of all 5 sternness-related genes after 4 days of treatment. Similar results were found in Hs578T, SUM159, and Panc1 cell lines **(FIG.7).** Together with the effects seen in sphere growth and the increases measured in the percentage of cells bearing CSC surface markers, the data indeed suggests that the ligand CCL20 can directly promote CSC activity in the cell lines tested.

**EXAMPLE 4: Effect of CCL20 in cancer stem cells is mediated by its receptor, CCR6**

While the results of the experiments discussed in Examples 1-3 confirmed that CCL20 directly affected CSC activity, it remained to be determined whether CCL20 indeed signaled through its known receptor, CCR6, to exert this activity. The data depicted in **FIG. 5** showed that addition of purified CCL20 stimulated sphere growth. From this an experiment was designed to assess whether the effect of CCL20 was mediated through signaling through its receptor CCR6, reasoning that if CCL20 exerted its function through CCR6 signaling, knockdown of CCR6 would prevent CCL20-stimulated sphere growth.

In **FIG. 8A,** we show that recombinant human CCL20 (rhCCL20) loses the ability to stimulate sphere growth in Lovo colorectal cancer cells following knockdown of CCR6 with 20nM siRNA, while cells transfected with non-target control siRNA (20nM) retained their growth response to CCL20. To confirm that CCR6 was a necessary component to this CCL20 axis on CSCs, we separated $CCR6^+$ from $CCR6^-$ cells in the NCIH508 cell line, and stimulated both fractions with 100 pg/mL CCL20 for 48 hours and subsequently evaluated sternness-related gene expression. **FIG. 8B** shows that $CCR6^+$ cells responded with about a 2-fold increased expression of NANOG, BMI1, OCT3/4, and SOX2 after 48 hours of stimulation with 100 pg/mL, when compared with $CCR6^-$ cells. These results demonstrate that CSC-related activity in response to CCL20 stimulation is dependent on the presence of CCR6 as the receptor. To further support this finding, we observed that inhibition of this CCL20-CCR6 axis on the tumor cells, either by loss of CCL20 or by loss of CCR6, resulted in similar outcome of sphere growth **(FIG. 8C & 8D).** RNAi-mediated knockdown of either CCL20 or CCR6 reduced sphere growth to a similar extent in NCIH508 cells, namely 59.6±2.9% or 70.3±2.2% compared to non-target control siRNA (100.0±2.0%, p<0.001) **(FIG. 8C).** The same observation was made in Hs578T cells where RNAi-mediated knockdown reduced sphere growth to 55.4±3.9% with CCL20 siRNA and 21.8±1.8% with CCR6 siRNA, compared to 100.0±7.0% growth with control siRNA (p<0.001) **(FIG. 8D).** Altogether the results confirm that the CCL20-CCR6 axis is responsible for promoting the effects of CCL20 on CSCs.

**EXAMPLE 5: Neutralizing CCL20 inhibits cancer stem cell activity**

As the above Examples validate, CCL20 is a clear mediator of CSC activity. This example assessed whether neutralization of this target could then in turn inhibit CSCs. To test this, a commercially available monoclonal IgG antibody against CCL20 that had reported neutralizing potential was used in a series of experiments. A single dose administration of 10 μg/mL CCL20 IgG significantly reduced sphere formation at 4 days following treatment in models of all three indications tested, compared with the same 10 μg/mL dose of isotype control IgG **(FIG. 9).** Growth of Hs578T breast cancer spheres was reduced to a mere 11.1±11.1% compared to 100.0±27.2% sphere growth with control IgG (p<0.05, **FIG. 9A).** Similarly, growth of NCIH508 colorectal cancer spheres was reduced to 62.8±5.8% vs. 100.0±4.8% with control IgG (p<0.001, **FIG. 9B),** while growth of Panc1 pancreatic cancer spheres reduced to 18.1±2.1% down from 100.0±13.0% with control IgG treatment (p<0.001, **FIG. 9C**). Gating controls for CSC identification are illustrated in **FIG 15.**

The effect of CCL20 inhibition on CSC reduction was confirmed by flow cytometry, where a similar 4 day treatment of Bxpc3 spheres with a single dose administration of 10 μg/mL CCL20 IgG decreased the percentage of $Epcam^+CD44^+CD24^+$ CSCs to 4.5±0.4%, down from 5.6±0.1% with 10 μg/mL control IgG treatment (p<0.05, **FIG. 9D).** The frequency of Panel pancreatic CSCs was reduced in spheres from 9.3±0.9 with control IgG treatment to 6.6±0.7% after CCL20 IgG (p<0.05, **FIG. 9E).** Altogether, these results confirm that neutralization of CCL20 with a monoclonal antibody can indeed be used as a potential therapeutic to reduce the CSC population, revealing a promising opportunity for CCL20 as a target for anti-CSC therapies.

**EXAMPLE 6: $T_{reg}$ mobility is mediated by CCL20 produced by CSCs.**

CCL20 is known to direct chemotactic migration of CCR6+ immune cells, predominantly in the Bcell, Tcell, and dendritic cell subsets. As a positive control, we confirmed the ability of an anti-CCL20 IgG to block chemotactic migration of a known cell type, namely CD4+ Tcells. **FIG. 10** confirms that anti-CCL20 IgG, dosed at 30 nM, can significantly inhibit chemotactic migration of CD4+ Tcells isolated from peripheral blood from 2 healthy human donors.

To test whether CCL20 produced by CSCs can influence migration of $T_{regs}$, we skewed human naive CD4+ Tcells towards type-1 regulatory Tcells (TR1) in culture as described above in the materials and methods. Flow cytometry analysis of the cells confirmed that TR1 cells resemble $T_{regs}$ by surface phenotype, as they are predominantly CD3+/CD4+/CD25+/FOXP3+ Tcells, and that these TR1 cells expressed CCR6 **(FIG. 11).** A transwell migration assay revealed that these TR1 cells could selectively migrate towards isolated CCL20, whereby 14.7±0.9% of cells migrated towards CCL20, compared with 10.2 ±0.6% of cells that non-specifically migrated (p<0.01, **FIG. 12A).** Addition of the anti-CCL20 antibody at 6-fold molar excess was able to block this migration, seen by a reduction of cell migration to 7.9±0.6% (p<0.01 compared with 100 nM CCL20 treatment, **FIG. 12A).**

Next, we asked whether TR1 cells could migrate towards CCL20 produced by CSCs. We generated CSC-conditioned medium from NCIH508 colon cancer cells cultured under CSC-driven sphere forming conditions for 4 days, which was found to contain CCL20 at an average concentration of 211 pg/mL (= 26.4 pM) as determined by ELISA **(FIG. 12B).** TR1 cells (19.3 ±0.9%) were able to migrate towards CSC CM compared with 10.3 ±0.6% of cells that non-specifically migrated (p<0.01, **FIG. 12C).** The migration was in fact directed towards CCL20 in the conditioned medium since addition of the neutralizing anti-CCL20 antibody significantly reduced migration back down to 13.8 ±0.7% (p<0.01 compared with CSC CM or CSC CM + isotype control IgG, **FIG. 12C).** Addition of an isotype control IgG did not have any effect on reducing TR1 migration towards CSC CM (17.9 ±1.7% migration with isotype control IgG). These results confirm the hypothesis that type-1 regulatory Tcells can migrate towards CCL20 produced by CSCs, and thereby suggests that neutralizing antibodies against CCL20 may reduce the infiltration of $T_{regs}$ into the tumor environment.

**EXAMPLE 7: CCL20 neutralizing antibodies reduce tumorigenesis and immunosuppression *in vivo***

Once CCL20 was established as a clear mediator of CSC activity, we investigated whether neutralization of this target could then in turn inhibit CSCs. To test this, we used a commercially available neutralizing human monoclonal antibody against CCL20 (clone 67310; R&D Systems). Ability of this antibody to inhibit CCL20-stimulated CCR6 pathway activity in both a cAMP assay and a beta-arrestin translocation assay **(FIG. 16A** and 16B, respectively) confirmed the neutralization potential. $IC_{50}$ was measured at 0.8nM in a cAMP assay when cells were stimulated with 1 nM CCL20, while $IC_{50}$ was measured at 2.7 nM when cells were stimulated with 6 nM CCL20.

The reduction of CSC content through CCL20 neutralization *in vitro* translated into a delay in tumor take rate and tumor growth rate. Median time to tumor take of Panc1 tumors (% tumor-bearing animals) was delayed by 56.5 days when Panc1 tumors were pre-treated with 10 ug/mL CCL20 IgG compared to Panc1 tumors that were pre-treated with 10 ug/mL isotype IgG for 4 days *in-vitro* prior to tumor inoculation **(FIG. 17A).** Tumor take in this instance was measured from the first instance of palpable tumor formation. Average tumor volume for CCL20 IgG treated tumors was also significantly smaller than isotype IgG treated tumors over the course of study **(FIG. 17B).**

To evaluate the role of CCL20 neutralization in immune-competent syngeneic mouse models, we identified a commercially available anti-mouse CCL20 monoclonal antibody (clone 114908; R&D Systems) with sufficient blocking/neutralization potential to act as a surrogate for the anti-human CCL20 monoclonal antibody. The anti-mouse CCL20 IgG antibody was able to inhibit the mouse CCL20-mouse CCR6 pathway as measured by a beta-arrestin translocation assay with an $IC_{50}$ of 14.0 nM, when cells were stimulated with 1.2 nM mouse CCL20 **(FIG. 18).** However, potency of the mouse CCL20 IgG is noticeably reduced compared with the human CCL20 IgG (described in **0174**) when the ratio of CCL20 to antibody $IC_{50}$ value is compared between human and mouse.

Syngeneic tumor models were used to evaluate the effect of *in vivo* CCL20 neutralization on peripheral and tumor-infiltrating immune cells. The 4T1 mouse breast carcinoma cell line is known to naturally express CCL20 and therefore chosen for this study. Immune-competent mice were xenografted subcutaneously with HLA-matched 4T1 cells, and dosed with anti-mouse CCL20 IgG or matched isotype control IgG at 10 mg per kg twice weekly for 4 consecutive doses once tumors had reached approximately 50-100mm$^3$. Post-mortem immunophenotyping was performed on spleen and tumor tissue harvested 24 hours following the 4$^{th}$ dose.

Frequency of lymphoid and myeloid immune cell subsets was determined by multicolor flow cytometry according to the gating schematic illustrated in **FIG. 19** for spleen and **FIG. 20** for tumor tissue. Full Minus One (FMO) controls were used for gating to identify cells that were positive vs. negative for any particular marker in the panel.

Data indicates that *in-vivo* treatment with an anti-CCL20 IgG significantly affects the frequency of peripheral and tumor-infiltrating immune cells. In the spleen, CCL20 inhibition caused a significant reduction in foxp3+ $T_{regs}$ **(FIG. 21A)** and ror$\gamma$t+ $Th_{17}$ cells **(FIG.** 21B), whereby the frequency of both populations decreased approximately 2-fold compared with isotype control treated animals (11.5$\pm$0.4% vs. 6.5$\pm$1.2% $T_{regs}$ and 4.7$\pm$0.4% vs. 2.2$\pm$0.5% $Th_{17}$ in isotype IgG vs. anti-CCL20 IgG treated animals, respectively). This same effect was also captured in the tumor, were the frequency of $T_{regs}$ reduced from 1.5$\pm$0.05% following isotype IgG treatment to 1.0$\pm$0.1% in response to anti-CCL20 IgG treatment **(FIG. 22A),** and the frequency of $Th_{17}$ cells also reduced from 0.4$\pm$0.05% in isotype IgG treated tumors to 0.3$\pm$0.02% after CCL20 IgG treatment **(FIG. 22B).** This result suggests that blocking CCL20 *in vivo* leads to both peripheral and tumor-specific reduction of 2 cell types known to promote immunosuppression in the tumor environment, namely $T_{reg}$ and $Th_{17}$, suggesting that therapies inhibiting CCL20 can help relieve immunosuppression as part of an anti-cancer therapeutic strategy.

In addition to the effect CCL20 exerts on $T_{reg}$ and $Th_{17}$ populations, CCL20 neutralization also significantly affected dendritic cells. In the tumor environment, dendritic cell frequency decreased from 18.2$\pm$2.2% in isotype IgG treated mice to 11.9$\pm$2.3% following CCL20 IgG treatment **(FIG. 22C,** not significant). This decrease was mostly attributed to the significant reduction in mature cd83+ dendritic cells **(FIG. 22E),** but not immature cd83-dendritic cells **(FIG. 22D).** Contrary to this, in the spleen, CCL20 neutralization caused a significant increase in dendritic cells, from 6.9$\pm$0.5% in isotype IgG treated mice to 9.8$\pm$0.6% in CCL20 IgG treated mice **(FIG. 21C).** The dendritic cell increase was predominantly to an increase in cd83- immature dendritic cells **(FIG. 21D),** not cd83+ mature dendritic cells **(FIG. 21E).** This result indicates that neutralizing CCL20 *in vivo* inhibits maturation of dendritic cells and consequently infiltration of this cell type into the tumor.

No changes were found in the remaining immune cell populations profiled, namely cd8+ Tcells and neutrophils/granulocytic myeloid derived suppressive cells (gMDSC) in either the spleen or tumors of mice treated with anti-CCL20 compared with isotype control treated mice (data not shown).

## DISCUSSION

The data in the above Examples demonstrates a previously unidentified and unexpected function for the CCL20-CCR6 axis aside from its known role in immune cell recruitment. In particular, the studies presented herein demonstrate that with regard to cancer, CCL20 has a specific role in CSC function, representing a previously unreported finding. In several of the illustrative Examples, the results show that in models of breast, colorectal, and pancreatic cancer, CCL20 is specifically overexpressed by CSCs when compared to non-CSC tumor cells, at both the transcript and protein level. The examples also confirm a functional role for the CCL20 protein in CSC activity, as exogenous administration of CCL20 to cell cultures provide a direct increase in the percentage of CSCs as determined by surface marker analysis. CCL20 also increases CSC self-renewal and proliferative function as seen by its effect in directly increasing the sternness gene activation and sphere growth, respectively. *In vitro,* CCL20 exerts activity on CSCs at physiologically relevant concentrations that are not only similar to the levels secreted into culture medium in our studies, but also similar to the circulating levels detected in colorectal cancer patients with poor prognosis (Iwata et al., 2013).

Although this activity of CCL20 on CSCs was an unexpected finding, other chemokines/cytokines have also been shown to have a direct effect on CSC activity. Interleukin-6 (IL6) and Interleukin-8 (IL8) have both been shown to provide direct effects on breast CSCs (Ginestier et al., 2010; Iliopoulos et al., 2011). Similarly, modulation of CXC-motif ligand 12 (CXCL12) has been shown to modulate glioblastoma CSCs (Wurth et al., 2014), while activity of CCL3 was placed in the context of CML leukemia-initiating cells (Baba et al., 2013). Thus, together with this finding, a growing body of evidence is pointing towards a role for mediators outside of conventional self-renewal pathways such as Notch-, Wnt-, and Hedgehog-signaling to regulate function of CSCs. Given the unique relationship between CCL20 and the receptor CCR6, we hypothesized that CCL20 must exert its activity on CSCs by signaling to its receptor CCR6. By knocking out CCR6, we confirmed that the receptor was indeed required for CCL20's activity in promoting functional CSC activity. But furthermore, when we separated cancer cell lines into fractions that were either positive or negative for the receptor, we found that only the receptor-positive cells showed activation of sternness genes following CCL20 stimulation. Lastly, the observation that CSCs appear to mostly co-localize with a $CCL20^+/CCR6^+$ cell surface phenotype, suggests that the activity of CCL20 on CSCs could be the result of an autocrine loop, although further studies would be needed to confirm this hypothesis. Altogether, this evidence supports the conclusion that CCL20 exerts its activity on CSCs by signaling through its receptor CCR6 on the tumor cells, and that thus the CCL20-CCR6 axis as a whole can be implemented in CSC activity in breast, pancreatic, and colorectal cancer.

A recent publication demonstrated that $APC^{min}$ mice showed reduced intestinal tumorigenesis when CCR6 was knocked out (Nandi et al., 2014). This result implicated the CCL20-CCR6 axis in tumor initiation in the intestine, again strengthening the role for this axis in the events underlying tumor development and progression. Given our finding that this CCL20-CCR6 axis is relevant to the CSC phenotype, we anticipate that therapeutic intervention of this axis could help eradicate CSCs. In fact, we find that treatment with a neutralizing monoclonal antibody against CCL20 significantly decreases the CSC frequency and activity in models of all three tumor types tested. While we see that CCL20 neutralization directly reduces the percentage of surface phenotype positive CSCs in pancreatic cancer models, we also find that CCL20 neutralization significantly inhibits sphere growth in breast, pancreatic, and colorectal cancer models. These results confirm that we can inhibit not just CSC numbers but, more importantly, CSC function.

*In vivo,* CSC function can be measured by their potential for tumorigenesis. We found that treatment of CSC-enriched pancreatic cancer spheres with a CCL20 neutralizing monoclonal antibody dramatically delays the onset of tumor formation in mouse models. Moreover, tumors that do form from CSCs treated with anti-CCL20 IgG show a significant reduction in tumor growth compared to isotype control treated tumors. This finding confirms that the role of CCL20 in driving CSC function, identified in the above examples, translates to an *in vivo* impact on tumorigenesis and rate of tumor growth.

Since the CCL20-CCR6 axis provides a baseline function in immune cell recruitment, we hypothesize that CSC secreted CCL20 could potentially influence tumor-infiltrating lymphocyte recruitment in addition to its direct effect on tumor cells. Chen *et al.* showed evidence that CCL20 levels directly correlated with regulatory T-cell levels in tumor biopsies of hepatocellular carcinoma patients, and that CCL20 significantly promoted regulatory T-cell migration in this indication (Chen et al., 2011). Given the association of regulatory T-cells with poor prognosis in many tumor types (Want et al. 2012), one could envision a mechanism whereby CCL20 promotes tumorigenicity via a one-two punch, by both regulating the CSC fraction, and by stimulating recruitment of immune-suppressive regulatory T-cells to the tumor environment.

When tested *in vivo* using immune-competent tumor-bearing mice, we confirmed indeed that CCL20 neutralization inhibits infiltration of $T_{regs}$ into the tumor. However, CCL20 neutralization also inhibited $Th_{17}$ infiltration into the tumor, a finding that is expected since $Th_{17}$ cells are characterized as CCR6+. Interestingly, we discovered that CCL20 inhibition not only affects the frequency of these two immune cell types in the tumor, but it also reduces the frequency of $T_{regs}$ and $Th_{17}$ cells in spleen, suggesting a peripheral response on inhibiting these two cell types beyond mere tumor infiltration. Contrary to this, we also identified that CCL20 neutralization reduced the frequency of mature dendritic cells in the tumor, however, this finding was countered by the presence of increased immature dendritic cells in the spleen. This result suggests that CCL20 also plays a role in maturation and then invasion of dendritic cells into the tumor environment.

Given the association of $T_{regs}$ and $Th_{17}$ cells with poor prognosis and immunosuppression in many tumor types, and the finding that CCL20 significantly reduces the frequency of these two cell types in the tumor and spleen, our overall findings propose that CCL20 plays a dual role in the tumor environment: 1) by driving activity and tumorigenic ability of CSCs, and 2) by promoting an immunosuppressive phenotype.

An abundance of pre-clinical and clinical evidence points to a critical function for CSCs in cancer treatment, thus treatments targeting this population of tumor cells can be of high value. The work described herein points not only to a novel and important role for the CCL20-CCR6 axis in CSC maintenance, but also to a critical role for CCL20 in supporting an immunosuppressive phenotype. This finding supports the hypothesis that CCL20 acts in a tumor-promoting manner on two fronts, by promoting CSC function and hindering the antitumor immune response. Our data indicates that neutralization of CCL20 can be a useful strategy to simultaneously inhibit CSC function and relieve the immunosuppressive phenotype. Overall we hypothesize that, by this dual mechanism, inhibition of CSCs by blocking CCL20 could allow for unique and effective therapies to treat cancer and improve patient lives.

References

Al-Hajj, M., Wicha, M.S., Benito-Hernandez, A., Morrison, S.J., and Clarke, M.F. (2003). Prospective identification of tumorigenic breast cancer cells. Proceedings of the National Academy of Sciences of the United States of America 100, 3983-3988.

Baba, M., Imai, T., Nishimura, M., Kakizaki, M., Takagi, S., Hieshima, K., Nomiyama, H., and Yoshie, O. (1997). Identification of CCR6, the specific receptor for a novel lymphocyte-directed CC chemokine LARC. The Journal of biological chemistry 272, 14893-14898. Baba, T., Naka, K., Morishita, S., Komatsu, N., Hirao, A., and Mukaida, N. (2013). MIP-1alpha/CCL3-mediated maintenance of leukemia-initiating cells in the initiation process of chronic myeloid leukemia. The Journal of experimental medicine 210, 2661-2673.

Barker, N., Ridgway, R.A., van Es, J.H., van de Wetering, M., Begthel, H., van den Born, M., Danenberg, E., Clarke, A.R., Sansom, O.J., and Clevers, H. (2009). Crypt stem cells as the cells-of-origin of intestinal cancer. Nature 457, 608-611.

Chen, K.-J., Lin, S.-Z., Zhou, L., Xie, H.-Y., Zhou, W.-H., Taki-Eldin, A., and Zheng, S.-S. (2011). Selective recruitment of regulatory T cell through CCR6-CCL20 in hepatocellular carcinoma fosters tumor progression and predicts poor prognosis. PloS one 6, e24671.

Creighton, C.J., Li, X., Landis, M., Dixon, J.M., Neumeister, V.M., Sjolund, A., Rimm, D.L., Wong, H., Rodriguez, A., Herschkowitz, J.I., et al. (2009). Residual breast cancers after conventional therapy display mesenchymal as well as tumor-initiating features. Proceedings of the National Academy of Sciences 106, 13820-13825.

Gemei, M., Mirabelli, P., Di Noto, R., Corbo, C., Iaccarino, A., Zamboli, A., Troncone, G., Galizia, G., Lieto, E., Del Vecchio, L., et al. (2013). CD66c is a novel marker for colorectal cancer stem cell isolation, and its silencing halts tumor growth in vivo. Cancer 119, 729-738.

Ghadjar, P., Rubie, C., Aebersold, D.M., and Keilholz, U. (2009). The chemokine CCL20 and its receptor CCR6 in human malignancy with focus on colorectal cancer. International journal of cancer Journal international du cancer 125, 741-745.

Ginestier, C., Liu, S., Diebel, M.E., Korkaya, H., Luo, M., Brown, M., Wicinski, J., Cabaud, O., Charafe-Jauffret, E., Birnbaum, D., et al. (2010). CXCR1 blockade selectively targets human breast cancer stem cells in vitro and in xenografts. The Journal of clinical investigation 120, 485-497.

Hermann, P.C., Huber, S.L., Herrler, T., Aicher, A., Ellwart, J.W., Guba, M., Bruns, C.J., and Heeschen, C. (2007). Distinct populations of cancer stem cells determine tumor growth and metastatic activity in human pancreatic cancer. Cell stem cell 1, 313-323.

Hirsch, D., Barker, N., McNeil, N., Hu, Y., Camps, J., McKinnon, K., Clevers, H., Ried, T., and Gaiser, T. (2014). LGR5 positivity defines stem-like cells in colorectal cancer. Carcinogenesis 35, 849-858.

Iliopoulos, D., Hirsch, H.A., Wang, G., and Struhl, K. (2011). Inducible formation of breast cancer stem cells and their dynamic equilibrium with non-stem cancer cells via IL6 secretion. Proceedings of the National Academy of Sciences of the United States of America 108, 1397-1402.

Iwata, T., Tanaka, K., Inoue, Y., Toiyama, Y., Hiro, J., Fujikawa, H., Okugawa, Y., Uchida, K., Mohri, Y., and Kusunoki, M. (2013). Macrophage inflammatory protein-3 alpha (MIP-3a) is a novel serum prognostic marker in patients with colorectal cancer. Journal of surgical oncology 107, 160-166.

Kleeff, J., Kusama, T., Rossi, D.L., Ishiwata, T., Maruyama, H., Friess, H., Buchler, M.W., Zlotnik, A., and Korc, M. (1999). Detection and localization of Mip-3alpha/LARC/Exodus, a macrophage proinflammatory chemokine, and its CCR6 receptor in human pancreatic cancer. International journal of cancer Journal international du cancer 81, 650-657.

Lapidot, T., Sirard, C., Vormoor, J., Murdoch, B., Hoang, T., Caceres-Cortes, J., Minden, M., Paterson, B., Caligiuri, M.A., and Dick, J.E. (1994). A cell initiating human acute myeloid leukaemia after transplantation into SCID mice. Nature 367, 645-648.

Li, C., Heidt, D.G., Dalerba, P., Burant, C.F., Zhang, L., Adsay, V., Wicha, M., Clarke, M.F., and Simeone, D.M. (2007). Identification of pancreatic cancer stem cells. Cancer research 67, 1030-1037.

Liu, J., Zhang, N., Li, Q., Zhang, W., Ke, F., Leng, Q., Wang, H., Chen, J., and Wang, H. Tumor-associated macrophages recruit CCR6+ regulatory T cells and promote the development of colorectal cancer via enhancing CCL20 production in mice. PLoS One 2011 Apr 29;6(4):e19495.

Marsigliante, S., Vetrugno, C., and Muscella, A. (2013). CCL20 induces migration and proliferation on breast epithelial cells. Journal of cellular physiology 228, 1873-1883.

Nandi, B., Pai, C., Huang, Q., Prabhala, R.H., Munshi, N.C., and Gold, J.S. (2014). CCR6, the sole receptor for the chemokine CCL20, promotes spontaneous intestinal tumorigenesis. PloS one 9, e97566.

Schutyser, E., Struyf, S., and Van Damme, J. (2003). The CC chemokine CCL20 and its receptor CCR6. Cytokine & growth factor reviews 14, 409-426.

Tehranchi, R., Woll, P.S., Anderson, K., Buza-Vidas, N., Mizukami, T., Mead, A.J., Åstrand-Grundstrom, I., Strömbeck, B., Horvat, A., Ferry, H., et al. (2010). Persistent Malignant Stem Cells in del(5q) Myelodysplasia in Remission. New England Journal of Medicine 363, 1025-1037.

van Vlerken, L.E., Kiefer, C.M., Morehouse, C., Li, Y., Groves, C., Wilson, S.D., Yao, Y., Hollingsworth, R.E., and Hurt, E.M. (2013). EZH2 is required for breast and pancreatic cancer stem cell maintenance and can be used as a functional cancer stem cell reporter. Stem cells translational medicine 2, 43-52.

Voo, KS, Bover, L, Harline, ML, Vien, LT, Facchinetti V, Arima, K, Kwak, LW, and Liu, YJ. Antibodies targeting human OX40 expand effector T cells and block inducible and natural regulatory T cell function. J. Immunol. 2013 Oct 1; 191(7): 3641-3650.

Wang, C., Xie, J., Guo, J., Manning, H.C., Gore, J.C., and Guo, N. (2012). Evaluation of CD44 and CD133 as cancer stem cell markers for colorectal cancer. Oncology reports 28, 1301-1308.

Wang, Y., Ma, Y., Fang, Y., Wu, S., Liu, L., Fu, D., and Shen, X. (2012). Regulatory T cell: a protection for tumour cells. Journal of Cellular and Molecular Medicine 16, 425-436.

Wurth, R., Bajetto, A., Harrison, J.K., Barbieri, F., and Florio, T. (2014). CXCL12 modulation of CXCR4 and CXCR7 activity in human glioblastoma stem-like cells and regulation of the tumor microenvironment. Frontiers in cellular neuroscience 8, 144.

Yu, Q., Lou, X.M., and He, Y. Preferential Recruitment of Th17 Cells to Cervical Cancer via CCR6-CCL20 Pathway. PLoS One 2015 Mar 13;10(3):e0120855.

Zhang, C.Y., Qi, Y., Li, X.N., Yang, Y., Liu, D.L., Zhao, J., Zhu, D.Y., Wu, K., Zhou, X.D., and Zhao, S. The role of CCL20/CCR6 axis in recruiting Treg cells to tumor sites of NSCLC patients. Biomed Pharmacother 2015 Feb;69:242-8.

## Claims

1. A method of determining the presence of a cancer stem cell (CSC) in a sample comprising cancer cells, the method comprising:

   - contacting the sample with an agent that binds to a CCL20 nucleic acid sequence or a CCL20 amino acid sequence;
   - detecting the presence or absence of binding between the agent and the CCL20 nucleic acid sequence or the CCL20 amino acid sequence; and
   - identifying the presence of the CSC in the sample upon detection of binding between the agent and the CCL20 nucleic acid sequence or the CCL20 amino acid sequence.

2. The method of claim 1 wherein the agent further comprises a detectable moiety.

3. The method of claim 1 or 2 , further comprising quantifying the amount of the CCL20 nucleic acid sequence or the CCL20 amino acid sequence in the sample, comparing the amount of the CCL20 nucleic acid sequence or the CCL20 amino acid sequence to a reference level of CCL20 nucleic acid sequence or CCL20 amino acid sequence, and identifying the presence of the CSC in the sample when the detected amount is greater than the reference level.

4. The method of any one of claims 1 to 3, wherein the agent comprises (i) a nucleic acid sequence that hybridizes to the CCL20 nucleic acid sequence under stringent hybridization conditions, or (ii) an antibody that specifically binds to the CCL20 amino acid sequence.

## Patentansprüche

1. Verfahren zum Bestimmen des Vorhandenseins einer Krebsstammzelle (CSC) in einer Probe, die Krebszellen umfasst, wobei das Verfahren umfasst:

- Inkontaktbringen der Probe mit einem Mittel, das an eine CCL20-Nukleinsäuresequenz oder eine CCL20-Aminosäuresequenz bindet;
- Nachweisen des Vorhandenseins oder Fehlens einer Bindung zwischen dem Mittel und der CCL20-Nukleinsäuresequenz oder der CCL20-Aminosäuresequenz; und
- Identifizieren des Vorhandenseins der CSC in der Probe beim Nachweis der Bindung zwischen dem Mittel und der CCL20-Nukleinsäuresequenz oder der CCL20-Aminosäuresequenz.

2. Verfahren nach Anspruch 1, wobei das Mittel zudem eine nachweisbare Einheit umfasst.

3. Verfahren nach Anspruch 1 oder 2, zudem umfassend das Quantifizieren der Menge der CCL20-Nukleinsäuresequenz oder der CCL20-Aminosäuresequenz in der Probe, Vergleichen der Menge der CCL20-Nukleinsäuresequenz oder der CCL20-Aminosäuresequenz mit einem Referenzwert der CCL20-Nukleinsäuresequenz oder CCL20-Aminosäuresequenz und Identifizieren des Vorhandenseins der CSC in der Probe, wenn die nachgewiesene Menge größer als der Referenzwert ist.

4. Verfahren nach einem der Ansprüche 1 bis 3, wobei das Mittel (i) eine Nukleinsäuresequenz, die unter stringenten Hybridisierungsbedingungen an die CCL20-Nukleinsäuresequenz bindet, oder (ii) einen Antikörper, der spezifisch an die CCL20-Aminosäuresequenz bindet, umfasst.

## Revendications

1. Procédé de détermination de la présence d’une cellule souche cancéreuse (CSC) dans un échantillon comprenant des cellules cancéreuses, le procédé comprenant :

- la mise en contact de l’échantillon avec un agent qui se lie à une séquence d’acides nucléiques du CCL20 ou à une séquence d’acides aminés du CCL20 ;
- la détection de la présence ou de l’absence de liaison entre l’agent et la séquence d’acides nucléiques du CCL20 ou la séquence d’acides aminés du CCL20 ; et
- l’identification de la présence de la CSC dans l’échantillon lors de la détection de la liaison entre l’agent et la séquence d’acides nucléiques du CCL20 ou la séquence d’acides aminés du CCL20.

2. Procédé selon la revendication 1, l’agent comprenant en outre un fragment détectable.

3. Procédé selon la revendication 1 ou 2, comprenant en outre la quantification de la quantité de la séquence d’acides nucléiques du CCL20 ou de la séquence d’acides aminés du CCL20 dans l’échantillon, la comparaison de la quantité de la séquence d’acides nucléiques du CCL20 ou de la séquence d’acides aminés du CCL20 à un niveau de référence de séquence d’acides nucléiques du CCL20 ou de séquence d’acides aminés du CCL20, et l’identification de la présence de la CSC dans l’échantillon lorsque la quantité détectée est supérieure au niveau de référence.

4. Procédé selon l’une quelconque des revendications 1 à 3, l’agent comprenant (i) une séquence d’acides nucléiques qui est hybridée à la séquence d’acides nucléiques du CCL20 dans des conditions d’hybridation rigoureuses, ou (ii) un anticorps qui se lie spécifiquement à la séquence d’acides aminés du CCL20.

Figure 1

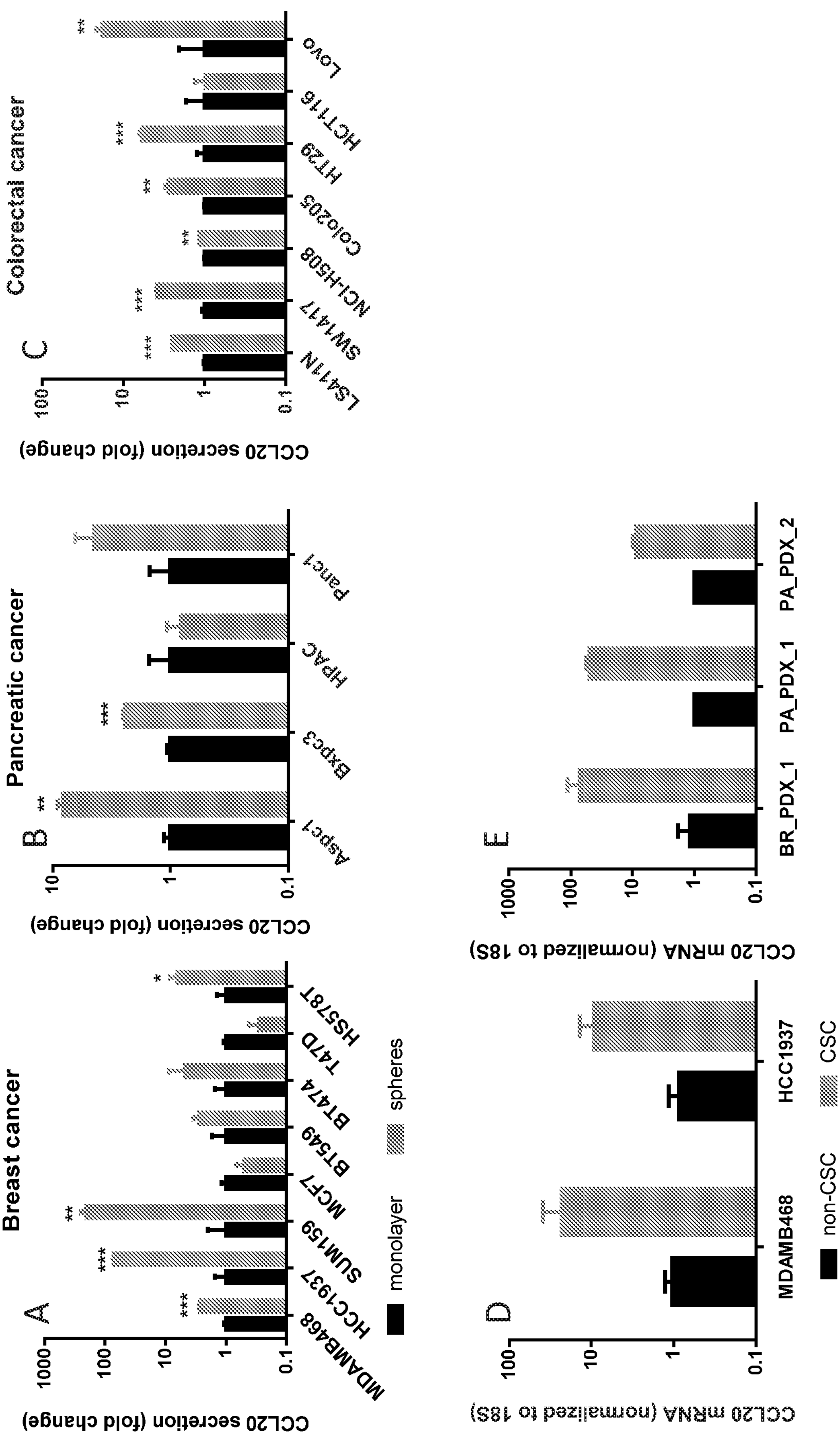
A
Breast cancer
CCL20 secretion (fold change)
1000
100
10
1
0.1
MDAMB468
HCC1937
SUM159
MCF7
BT549
BT474
T47D
HS578T
***
**
*
monolayer
spheres
B
Pancreatic cancer
CCL20 secretion (fold change)
10
1
0.1
Aspc1
Bxpc3
HPAC
Panc1
C
Colorectal cancer
CCL20 secretion (fold change)
100
10
1
0.1
LS411N
SW1417
NCI-H508
Colo205
HT29
HCT116
Lovo
D
CCL20 mRNA (normalized to 18S)
100
10
1
0.1
MDAMB468
HCC1937
non-CSC
CSC
E
CCL20 mRNA (normalized to 18S)
1000
100
10
1
0.1
BR_PDX_1
PA_PDX_1
PA_PDX_2

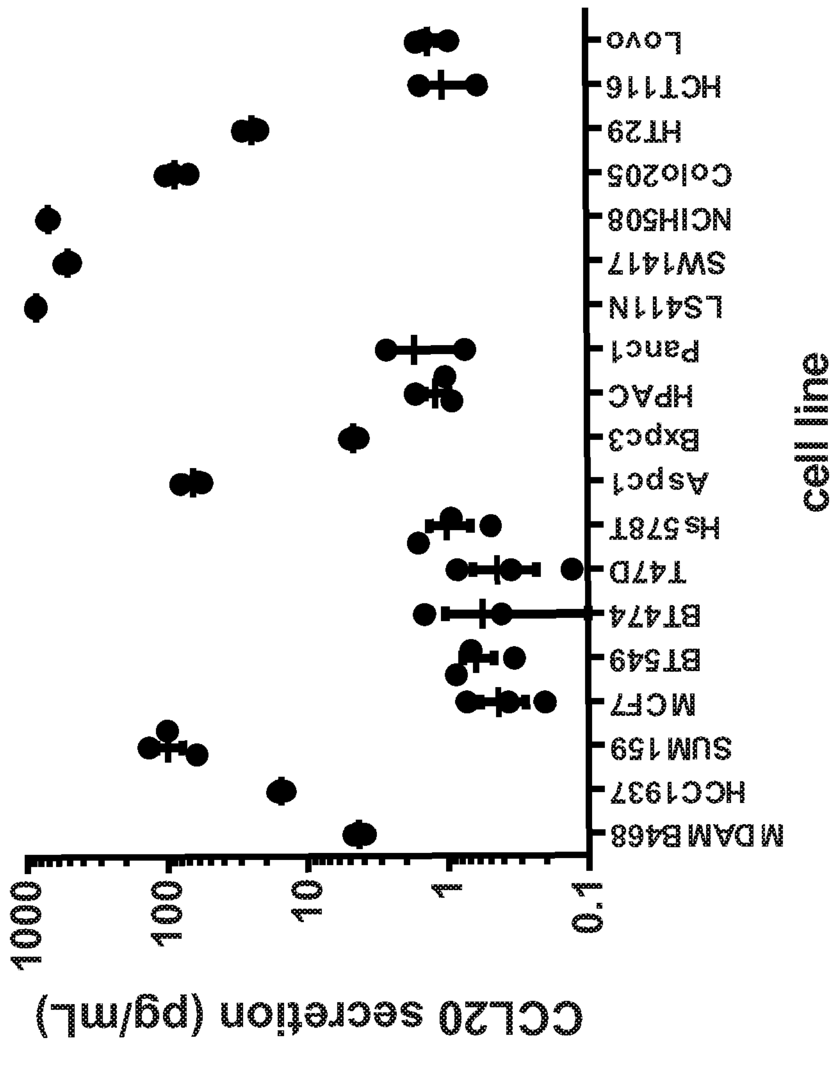
CCL20 secretion (pg/mL)
1000
100
10
1
0.1
MDAMB468
HCC1937
SUM159
MCF7
BT549
BT474
T47D
Hs578T
Aspc1
Bxpc3
HPAC
Panc1
LS411N
SW1417
NCIH508
Colo205
HT29
HCT116
Lovo
cell line

Figure 2

Figure 3

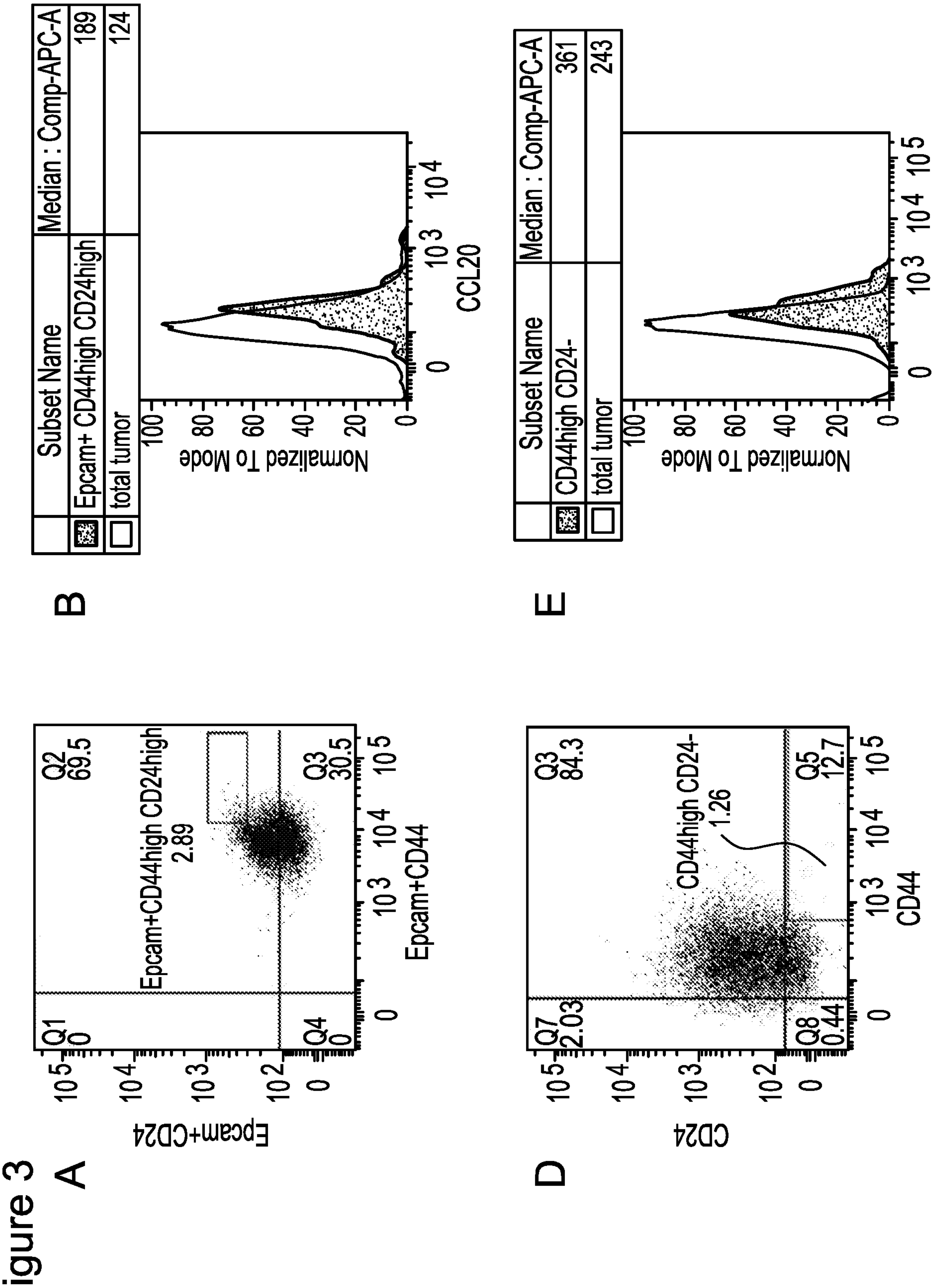
A
Epcam+CD24
Epcam+CD44
Q1
0
Q2
69.5
Q3
30.5
Q4
0
Epcam+CD44high CD24high
2.89
B
Subset Name
Median : Comp-APC-A
Epcam+ CD44high CD24high
189
total tumor
124
Normalized To Mode
CCL20
D
CD24
CD44
Q7
2.03
Q3
84.3
Q5
12.7
Q8
0.44
CD44high CD24-
1.26
E
Subset Name
Median : Comp-APC-A
CD44high CD24-
361
total tumor
243
Normalized To Mode
CCL20

Figure 3 Cont'd.

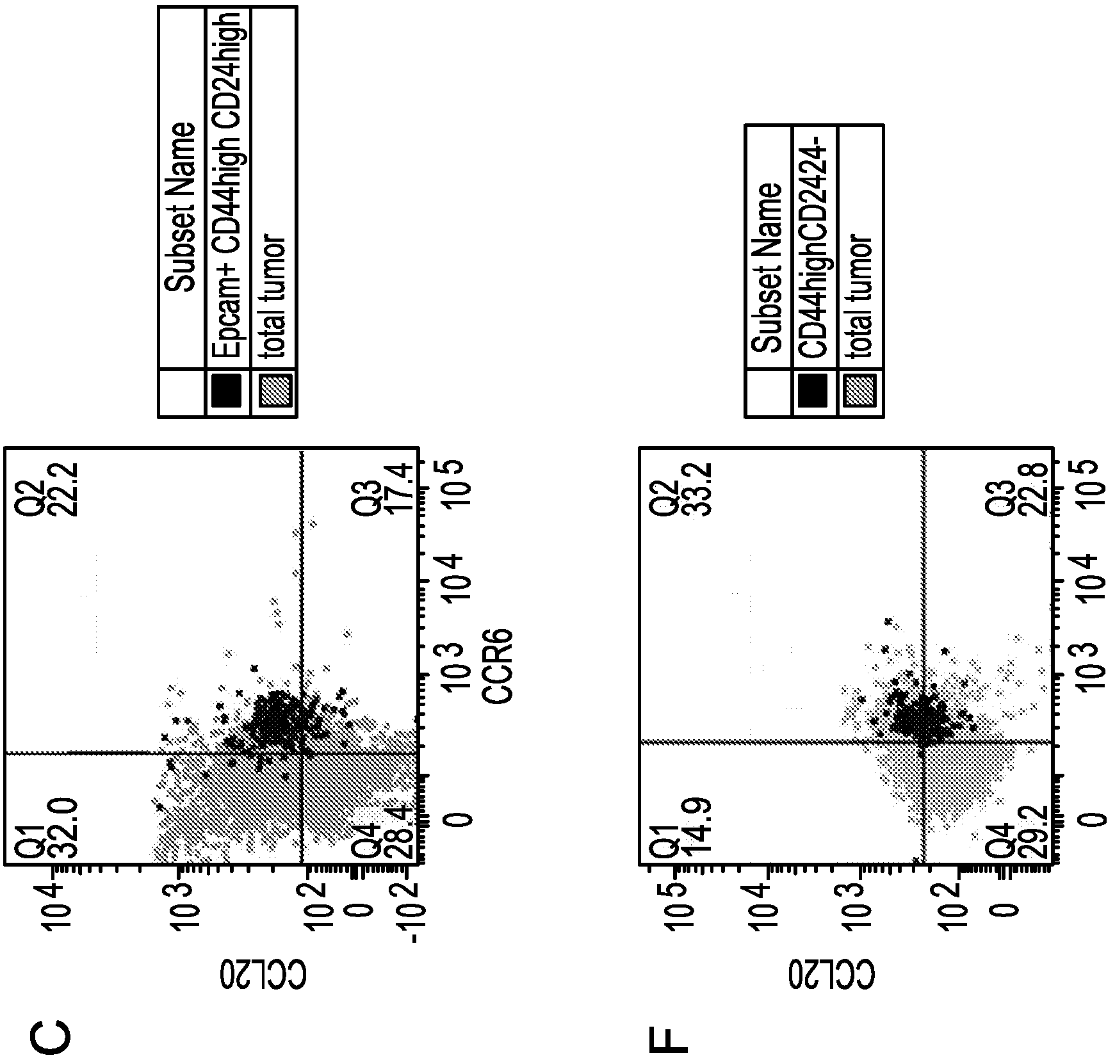

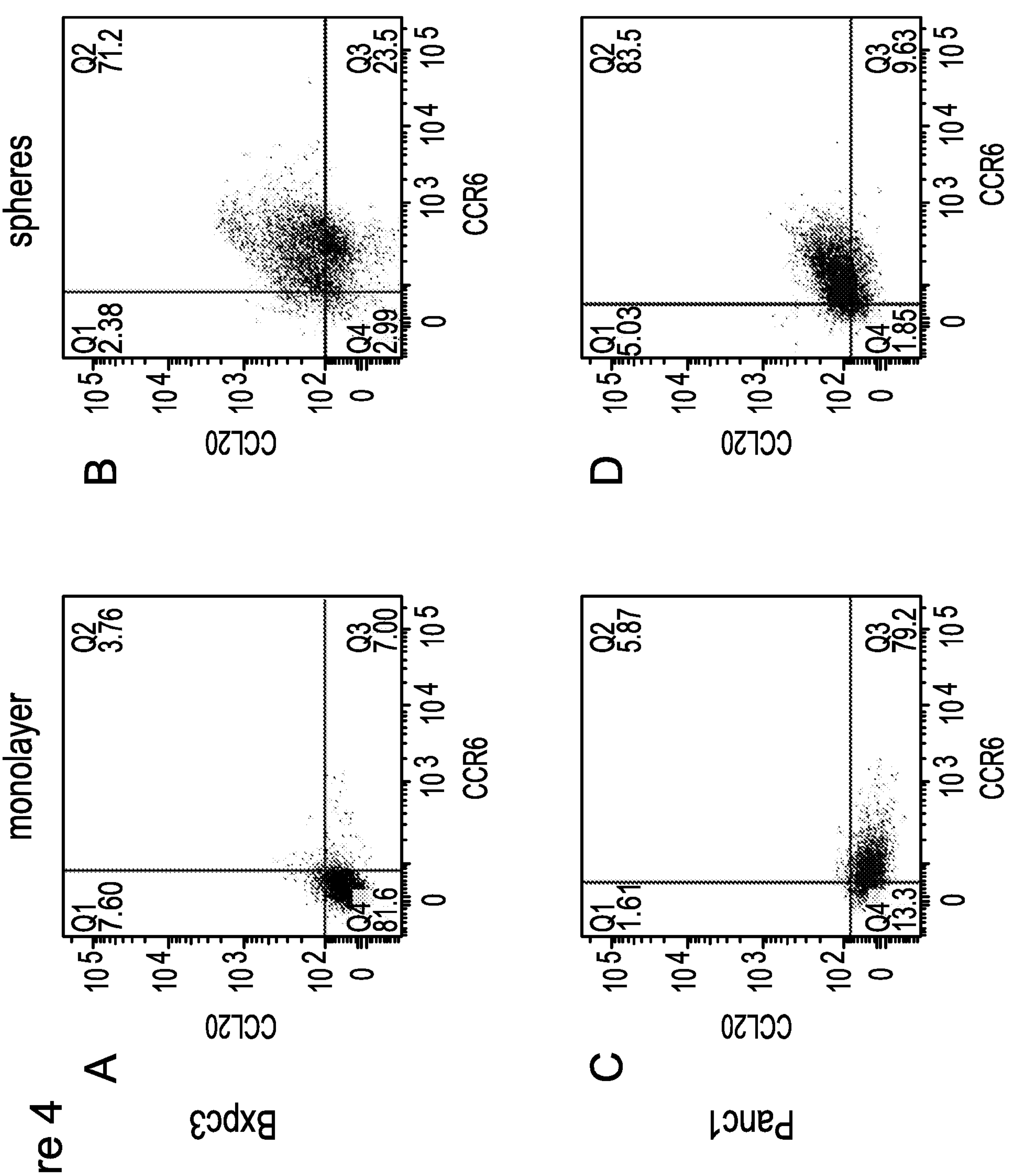
monolayer
spheres
Bxpc3
Panc1
A
B
C
D
CCL20
CCR6
Q1 7.60
Q2 3.76
Q3 7.00
Q4 81.6
Q1 2.38
Q2 71.2
Q3 23.5
Q4 2.99
Q1 1.61
Q2 5.87
Q3 79.2
Q4 13.3
Q1 5.03
Q2 83.5
Q3 9.63
Q4 1.85

Figure 4

Figure 4 Cont'd.

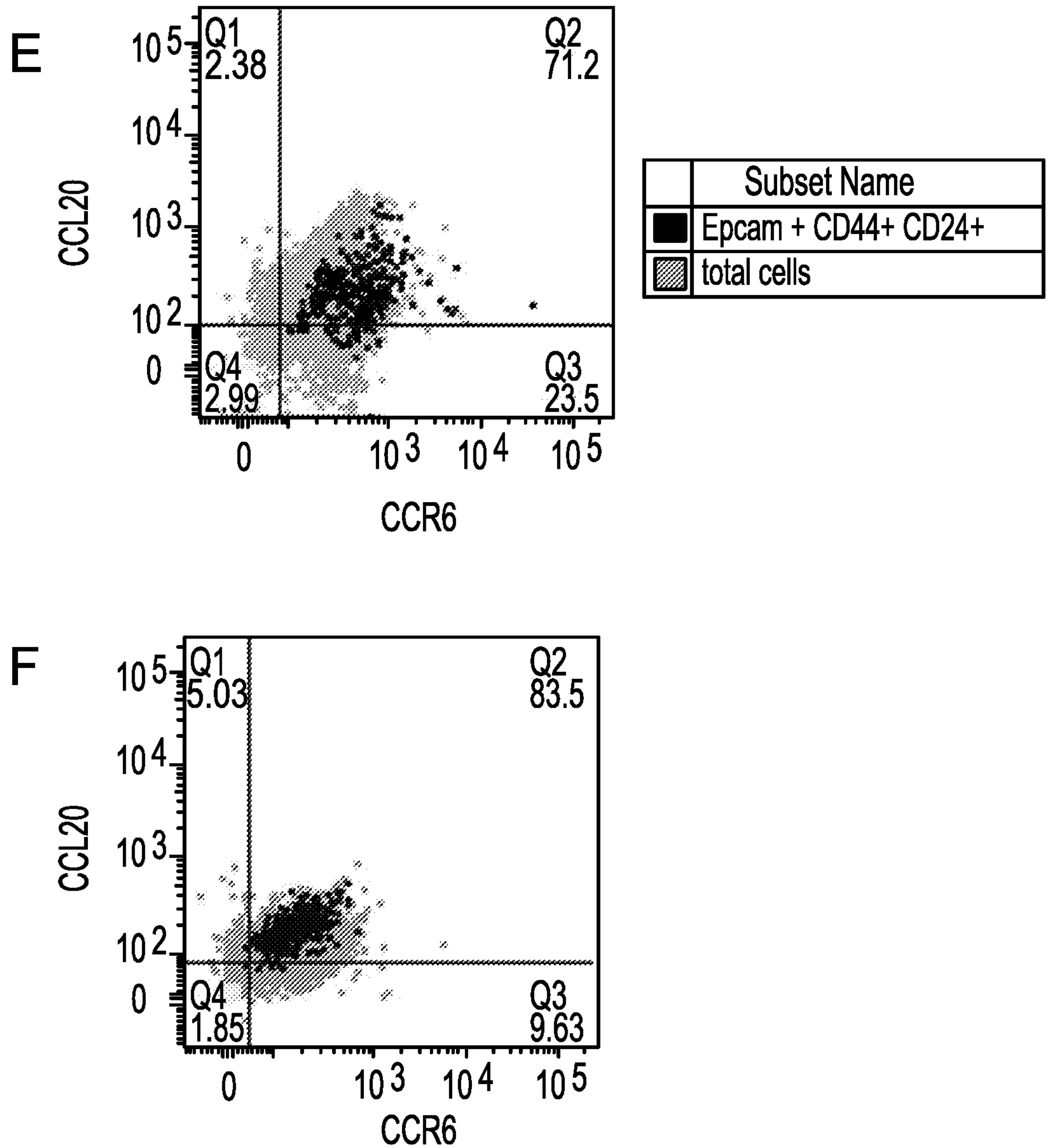

Figure 5

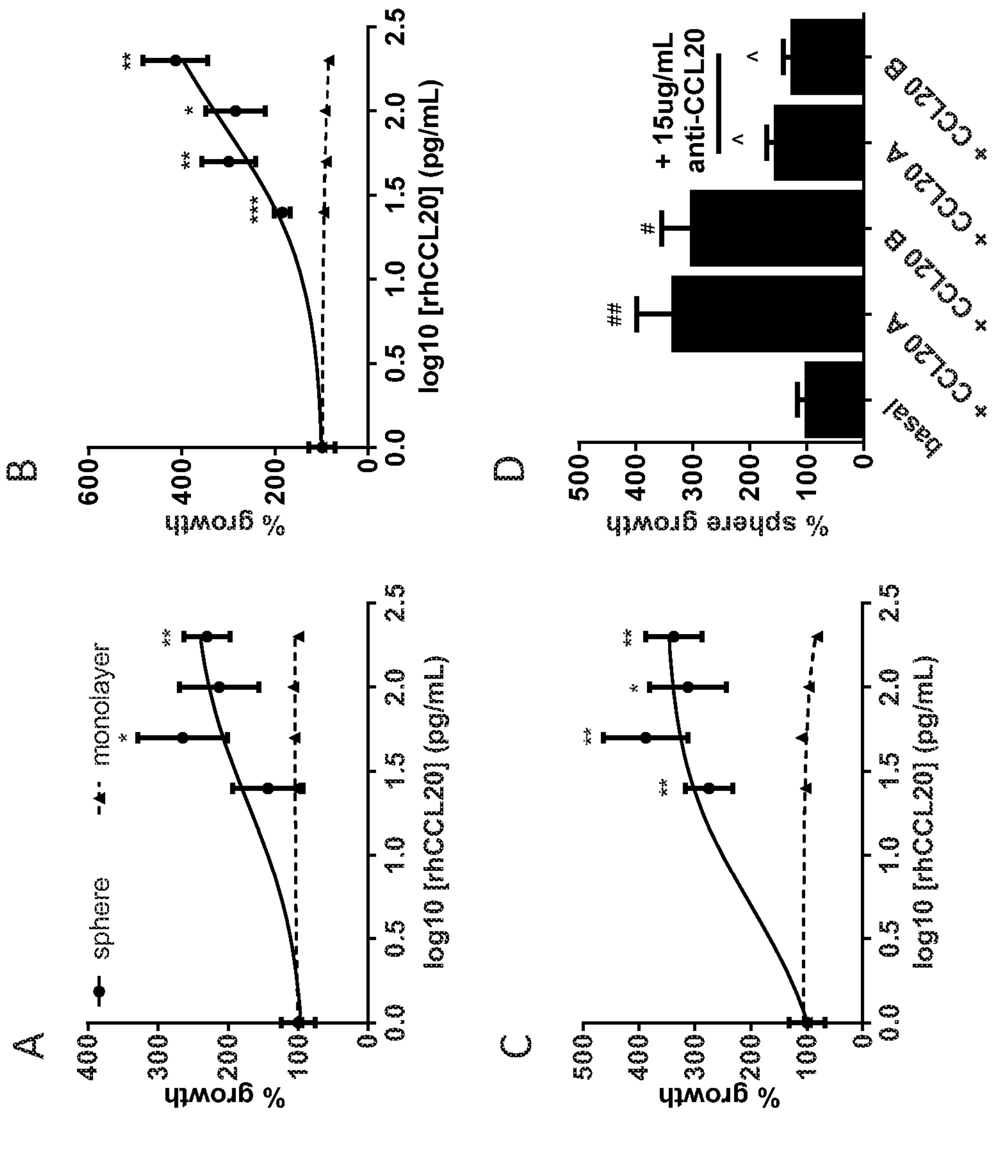
A
B
C
D
sphere
monolayer
% growth
% sphere growth
log10 [rhCCL20] (pg/mL)
+ 15ug/mL
anti-CCL20
basal
+ CCL20 A
+ CCL20 B

Figure 6

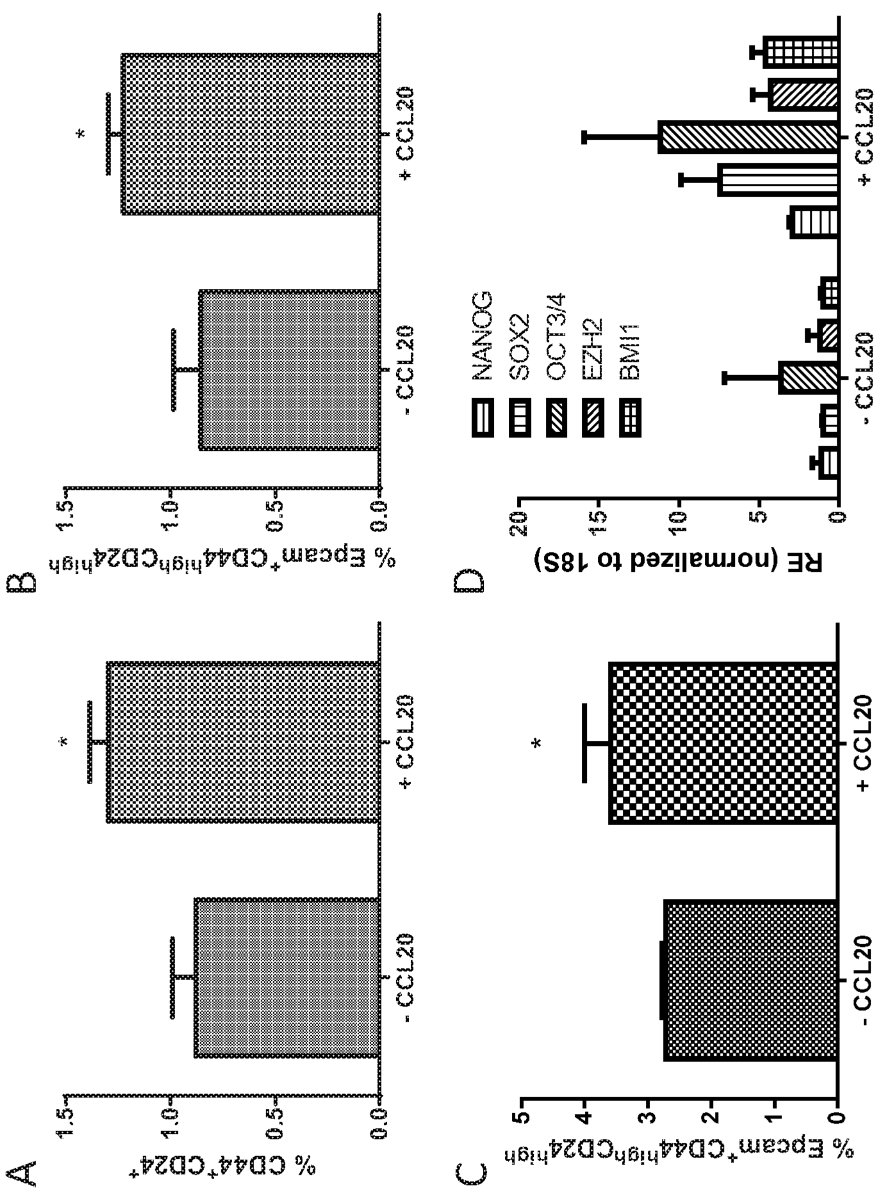
A
% CD44+CD24+
- CCL20
+ CCL20
B
% Epcam+CD44highCD24high
C
D
RE (normalized to 18S)
NANOG
SOX2
OCT3/4
EZH2
BMI1

Figure 7

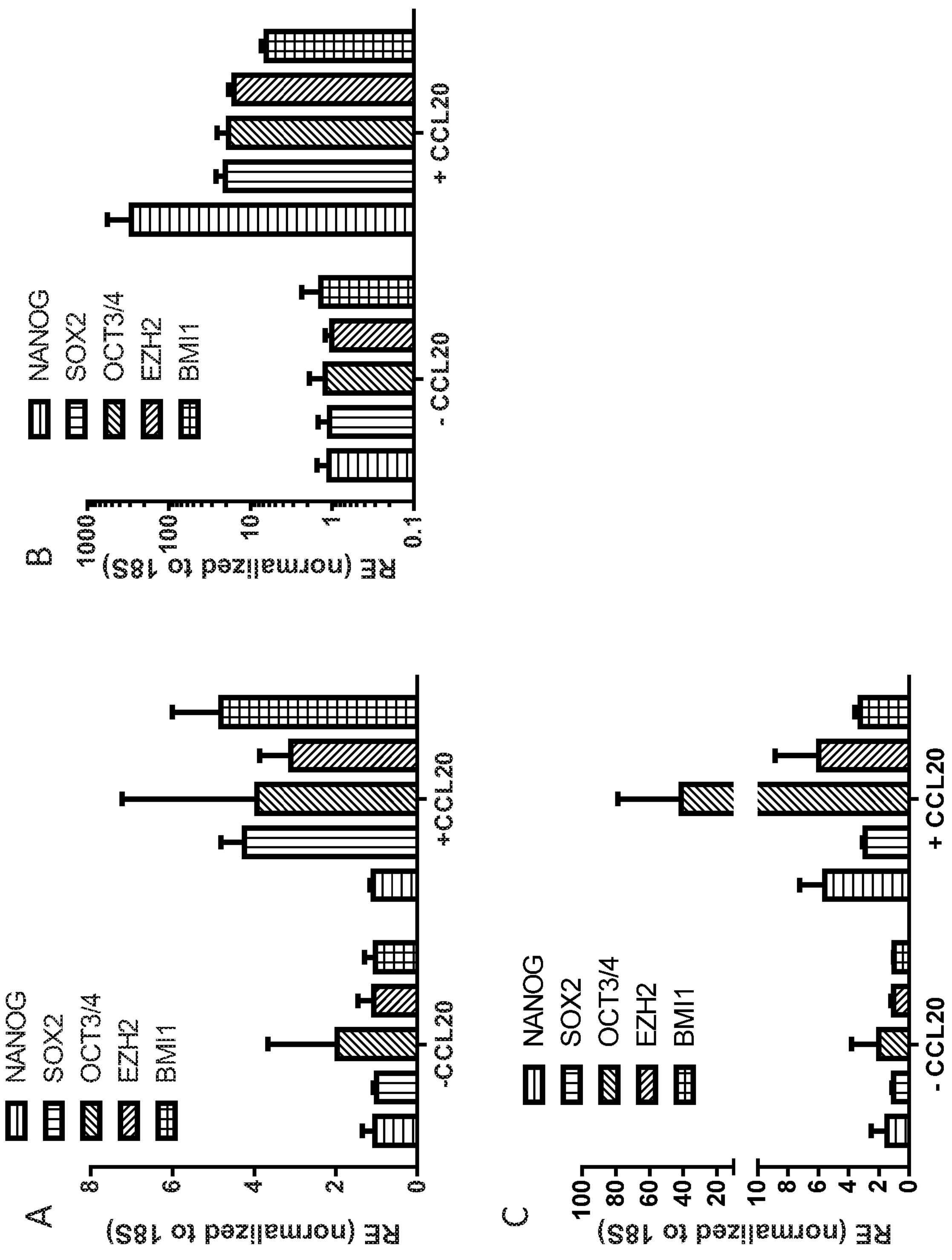
A
RE (normalized to 18S)
0
2
4
6
8
NANOG
SOX2
OCT3/4
EZH2
BMI1
-CCL20
+CCL20
B
RE (normalized to 18S)
0.1
1
10
100
1000
NANOG
SOX2
OCT3/4
EZH2
BMI1
- CCL20
+ CCL20
C
RE (normalized to 18S)
0
2
4
6
8
10
20
40
60
80
100
NANOG
SOX2
OCT3/4
EZH2
BMI1
- CCL20
+ CCL20

Figure 8

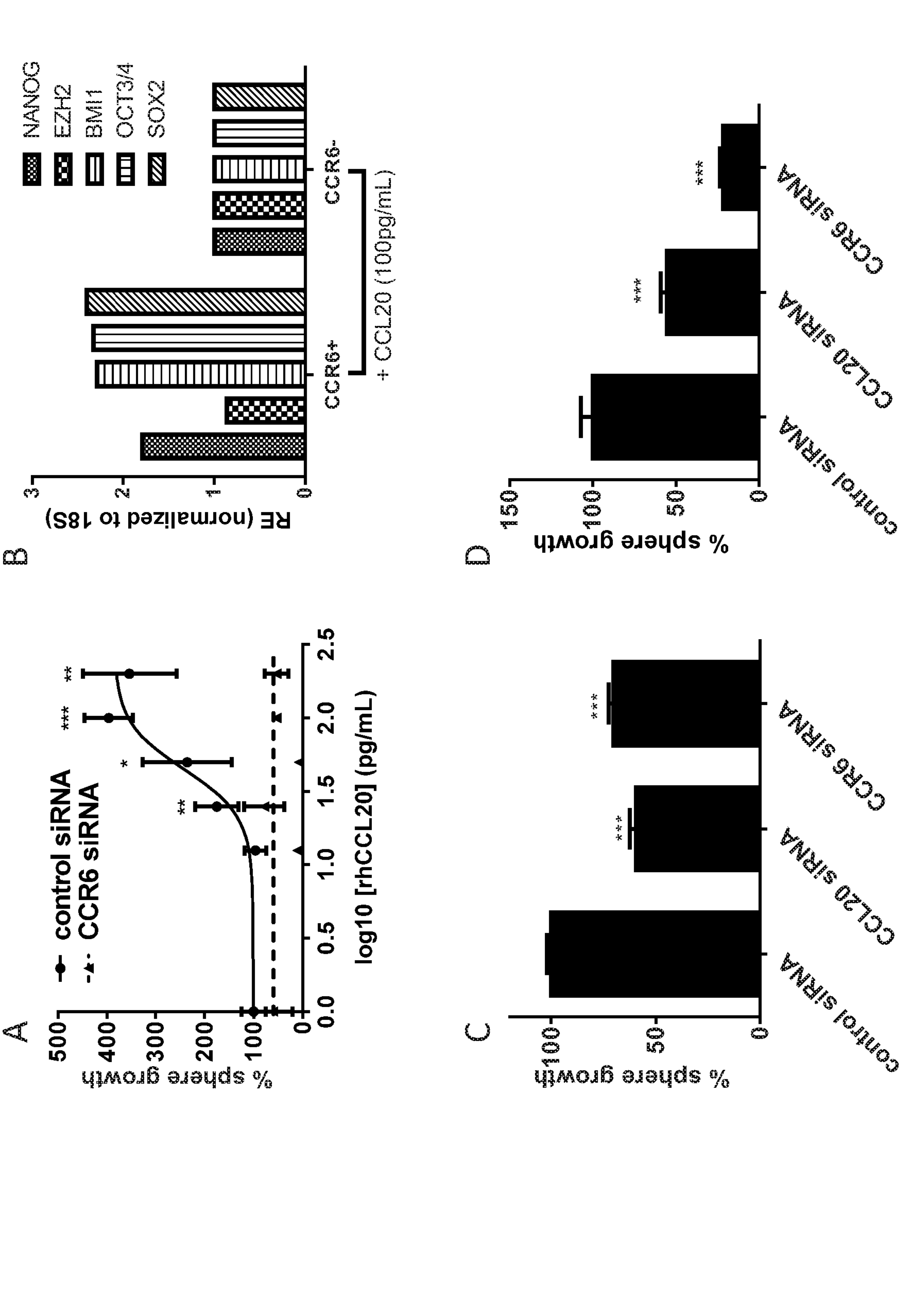
A
% sphere growth
control siRNA
CCR6 siRNA
log10 [rhCCL20] (pg/mL)
B
RE (normalized to 18S)
NANOG
EZH2
BMI1
OCT3/4
SOX2
CCR6+
CCR6-
+ CCL20 (100pg/mL)
C
% sphere growth
control siRNA
CCL20 siRNA
CCR6 siRNA
D
% sphere growth
control siRNA
CCL20 siRNA
CCR6 siRNA

Figure 9

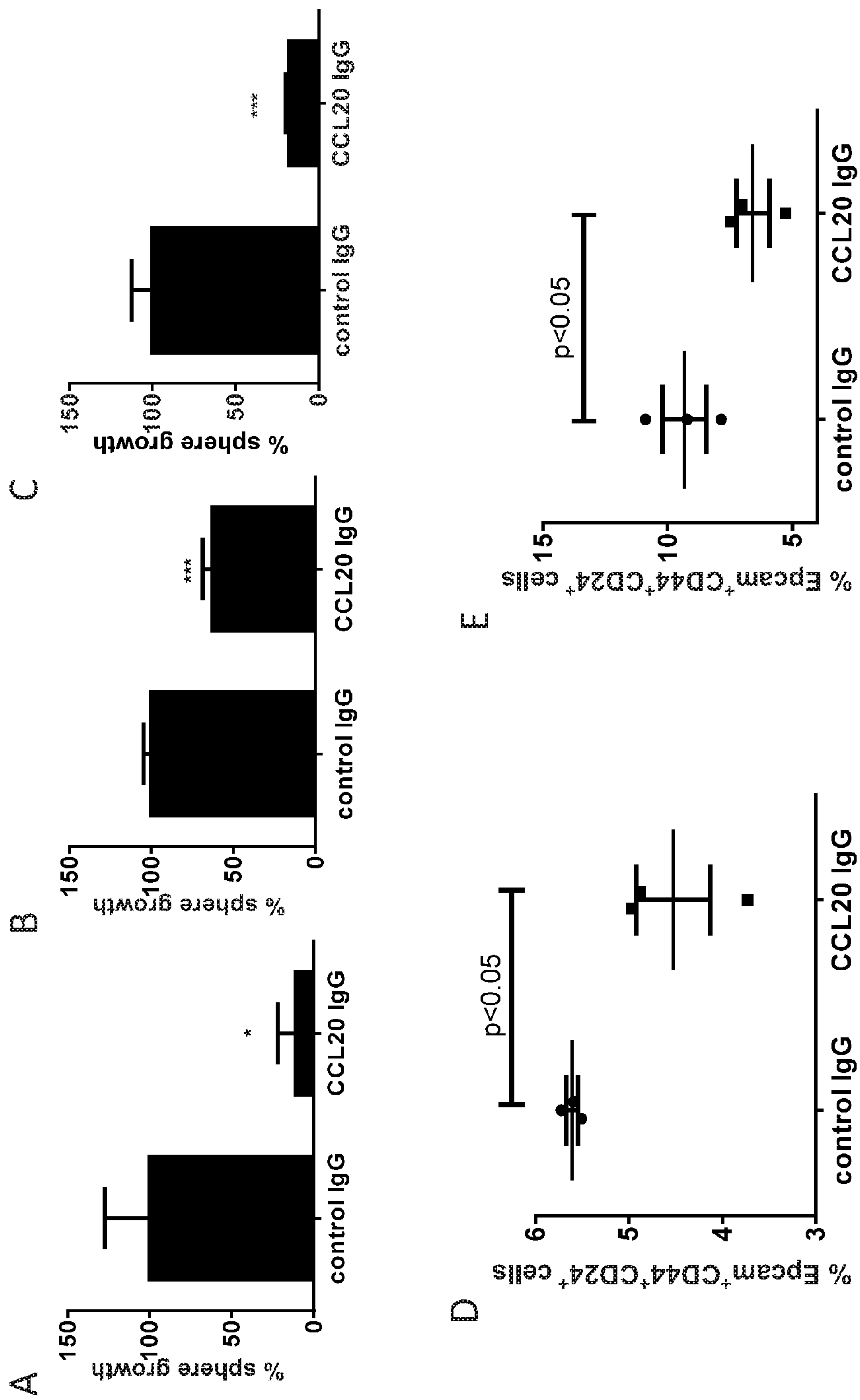
A
B
C
D
E
% sphere growth
150
100
50
0
control IgG
CCL20 IgG
*
***
% Epcam+CD44+CD24+ cells
6
5
4
3
15
10
p<0.05

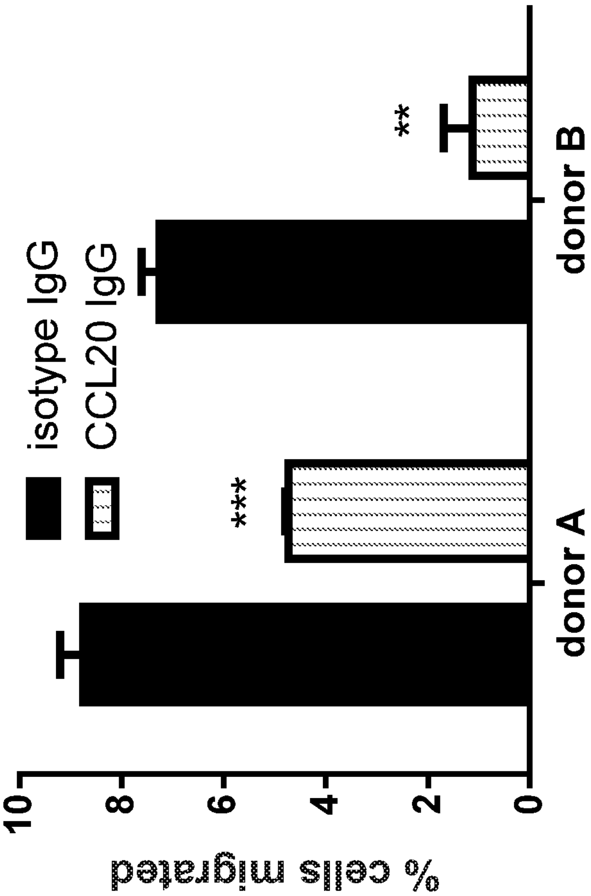
isotype IgG
CCL20 IgG
***
**
% cells migrated
10
8
6
4
2
0
donor A
donor B

Figure 10

Figure 11

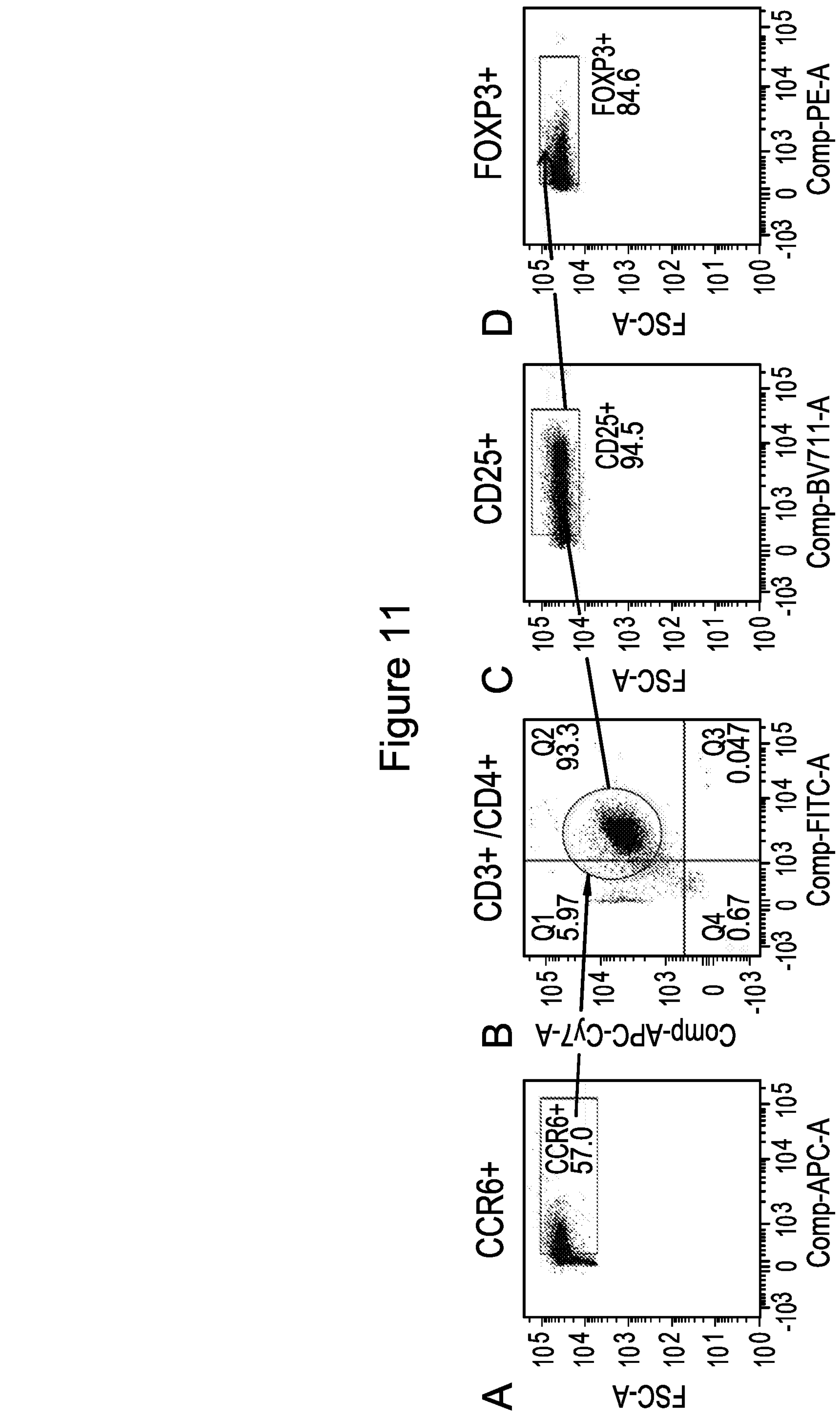

A
CCR6+
CCR6+
57.0
FSC-A
Comp-APC-A
B
CD3+ /CD4+
Q1
5.97
Q2
93.3
Q3
0.047
Q4
0.67
Comp-APC-Cy7-A
Comp-FITC-A
C
CD25+
CD25+
94.5
FSC-A
Comp-BV711-A
D
FOXP3+
FOXP3+
84.6
FSC-A
Comp-PE-A

Figure 11 Cont'd.
Gating controls:

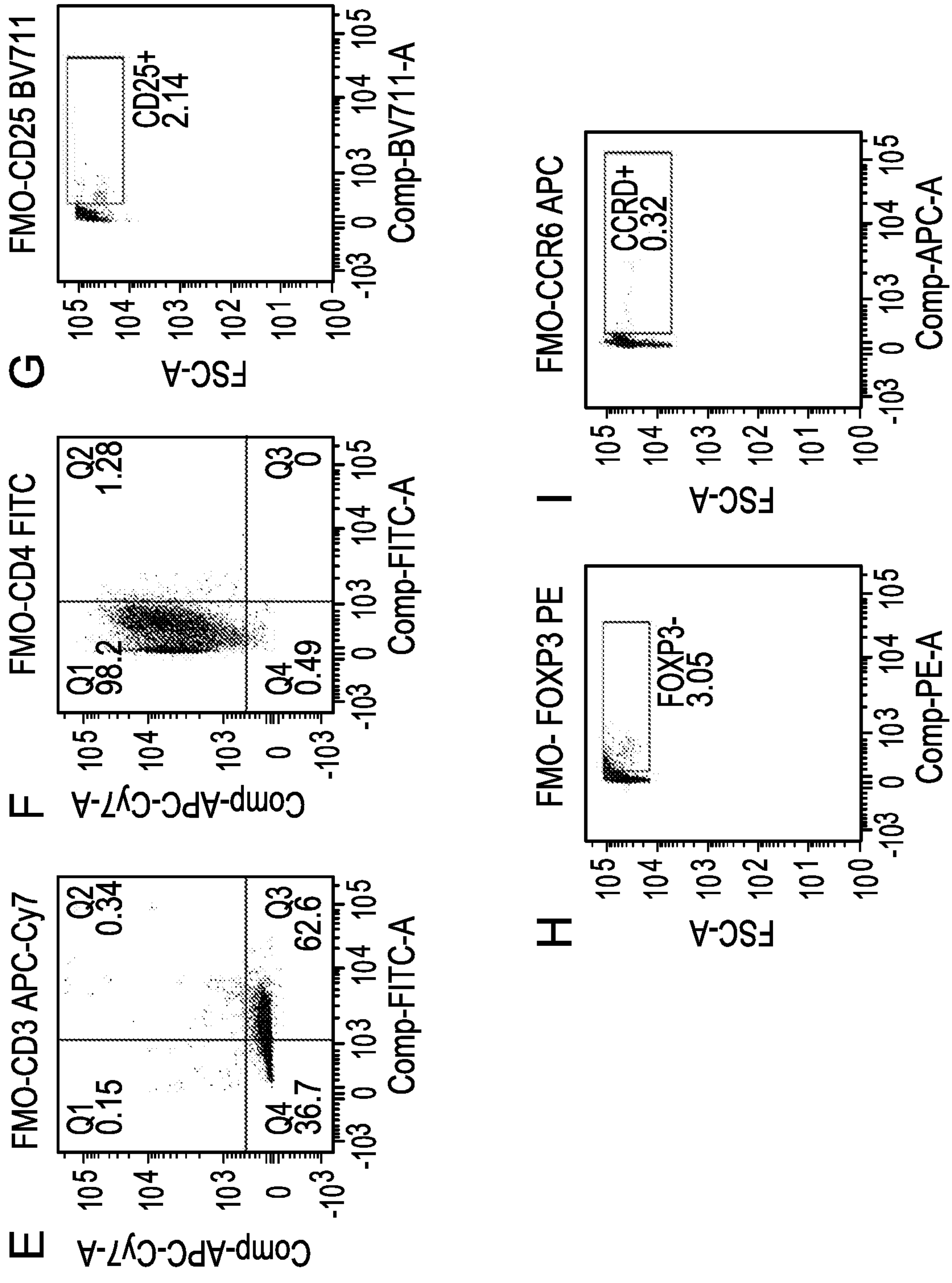

Figure 12

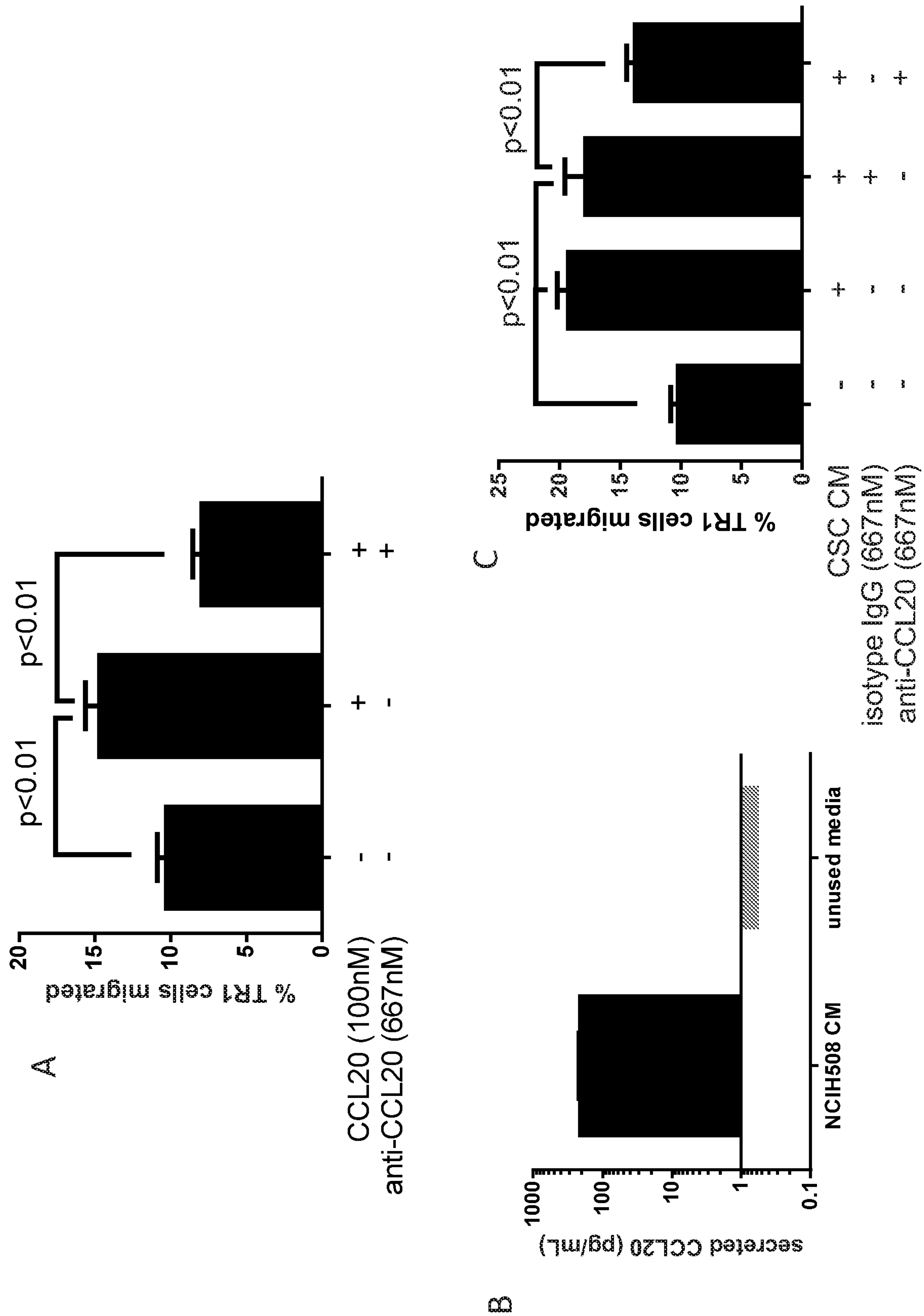
A
% TR1 cells migrated
20
15
10
5
0
p<0.01
p<0.01
CCL20 (100nM)
anti-CCL20 (667nM)
- -
+ -
+ +
B
secreted CCL20 (pg/mL)
1000
100
10
1
0.1
NCIH508 CM
unused media
C
% TR1 cells migrated
25
20
15
10
5
0
p<0.01
p<0.01
CSC CM
isotype IgG (667nM)
anti-CCL20 (667nM)
- - -
+ - -
+ + -
+ - +

Figure 13

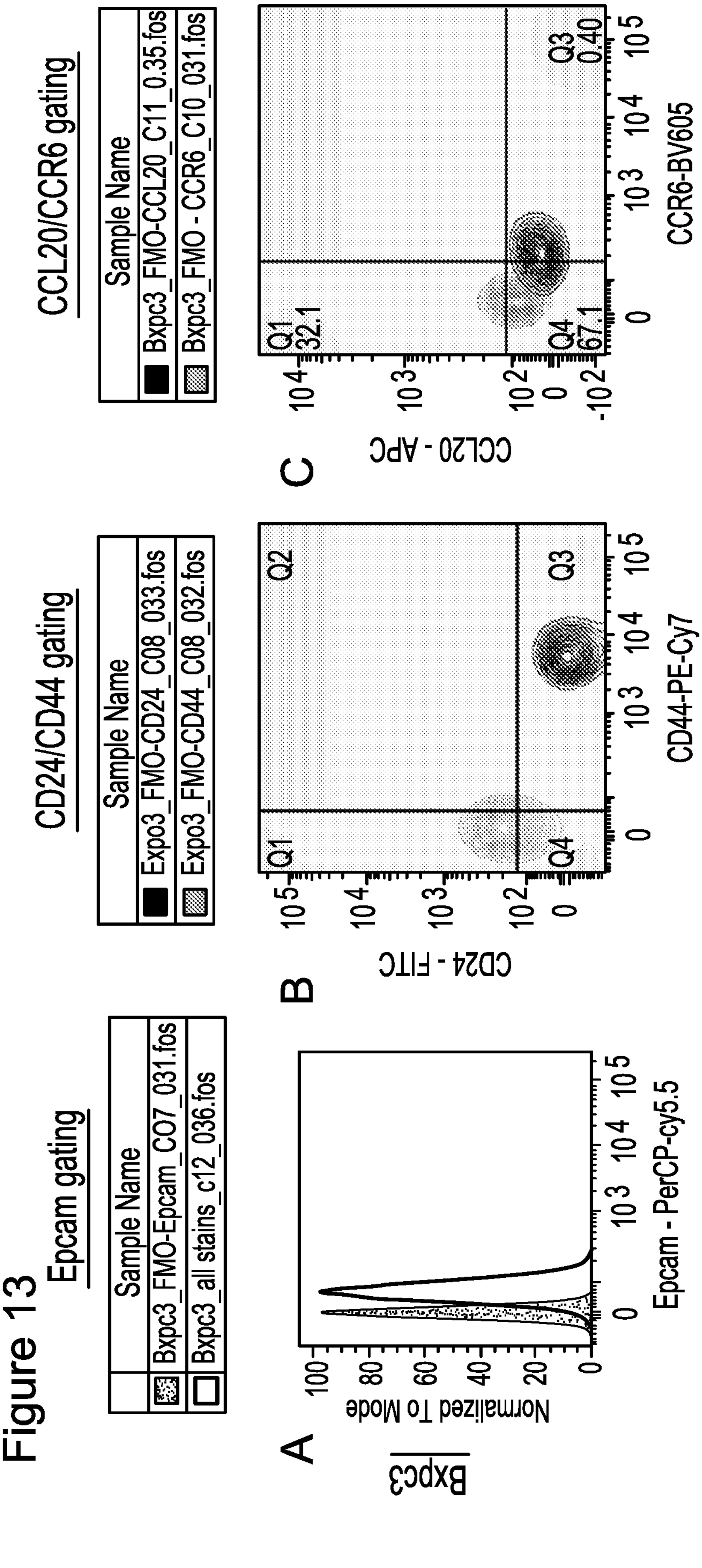

Epcam gating
Sample Name
Bxpc3_FMO-Epcam_CO7_031.fos
Bxpc3_all stains_c12_036.fos
A
Bxpc3
Normalized To Mode
Epcam - PerCP-cy5.5
CD24/CD44 gating
Sample Name
Expo3_FMO-CD24_C08_033.fos
Expo3_FMO-CD44_C08_032.fos
B
CD24 - FITC
CD44-PE-Cy7
Q1
Q2
Q3
Q4
CCL20/CCR6 gating
Sample Name
Bxpc3_FMO-CCL20_C11_0.35.fos
Bxpc3_FMO - CCR6_C10_031.fos
C
CCL20 - APC
CCR6-BV605
Q1
32.1
Q3
0.40
Q4
67.1

Figure 13 Cont'd.

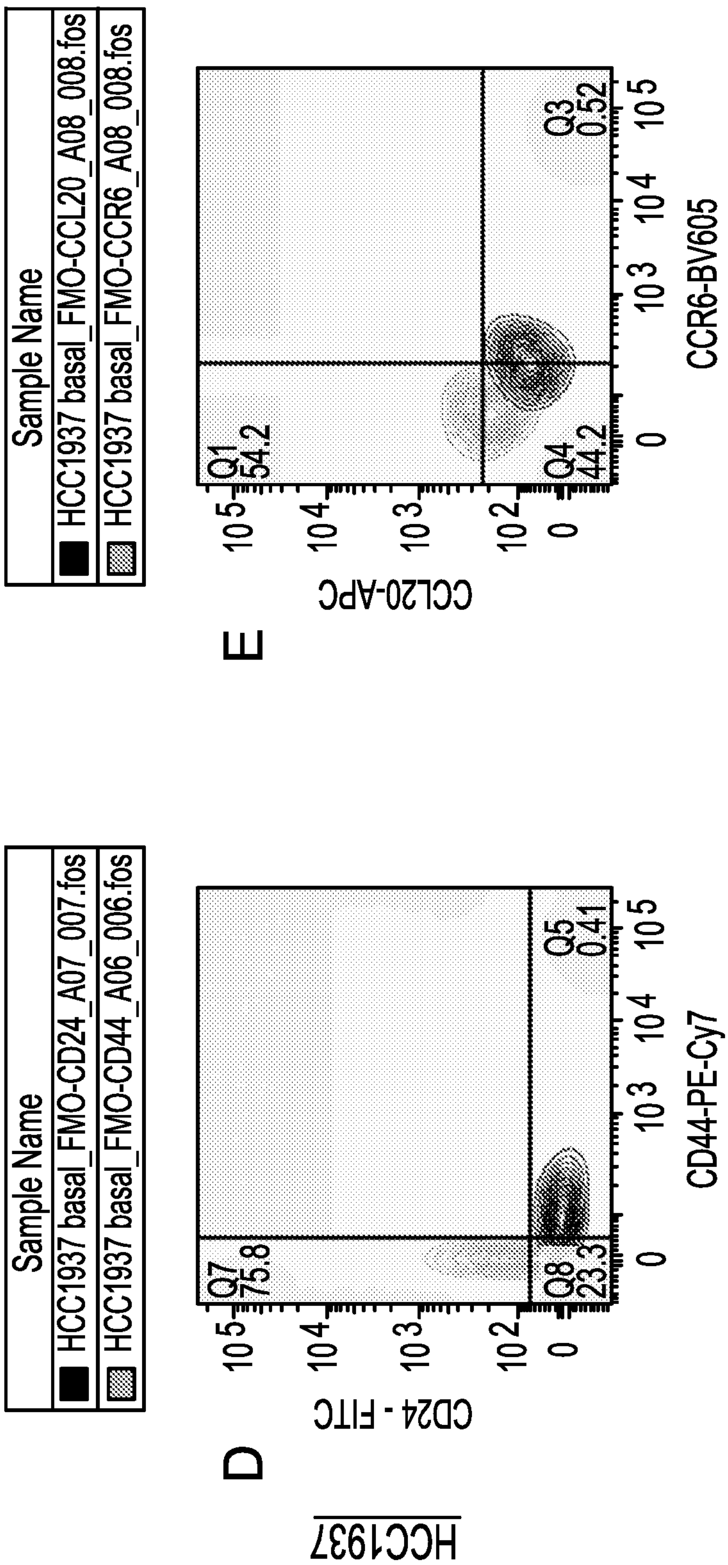

Figure 14

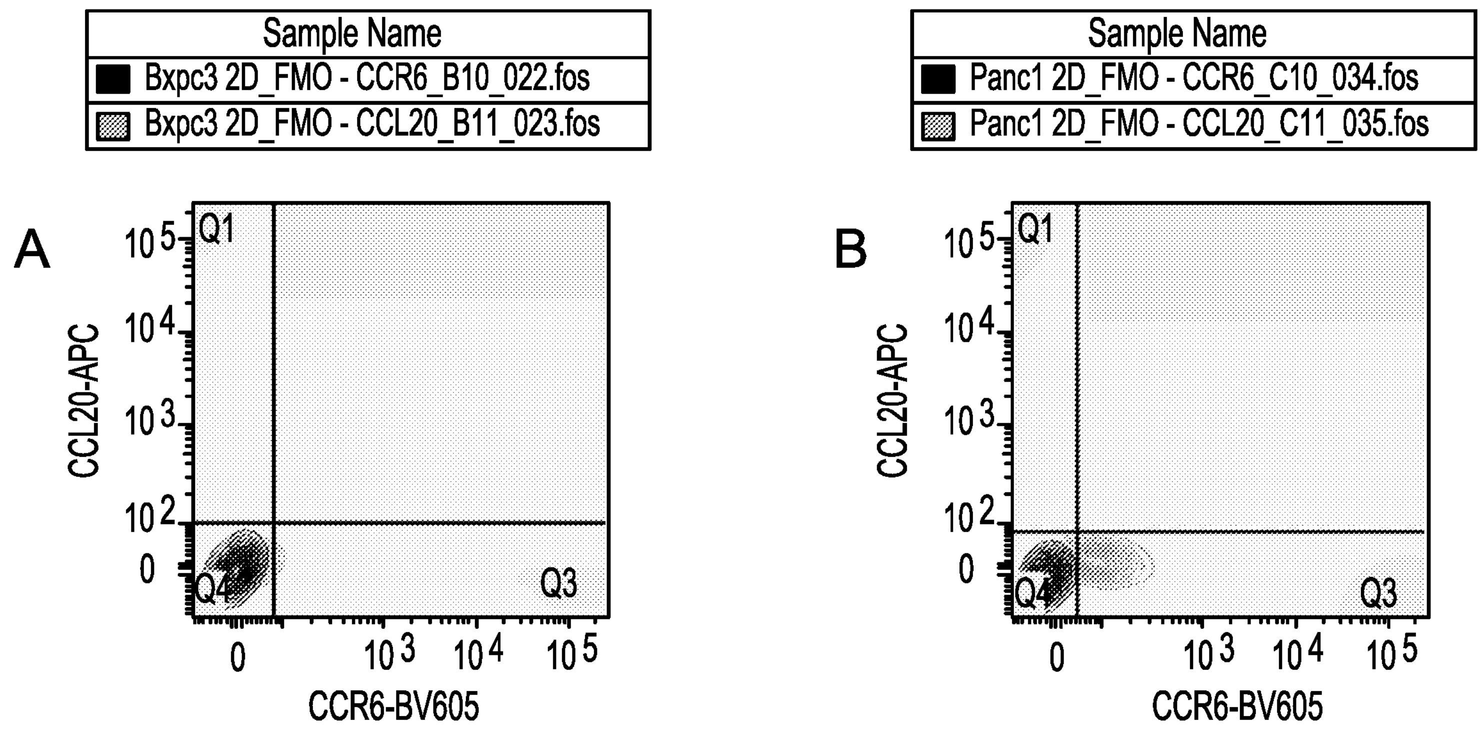
Sample Name
Bxpc3 2D_FMO - CCR6_B10_022.fos
Bxpc3 2D_FMO - CCL20_B11_023.fos
A
CCL20-APC
CCR6-BV605
Q1
Q3
Q4
Sample Name
Panc1 2D_FMO - CCR6_C10_034.fos
Panc1 2D_FMO - CCL20_C11_035.fos
B
CCL20-APC
CCR6-BV605
Q1
Q3
Q4

# Figure 15

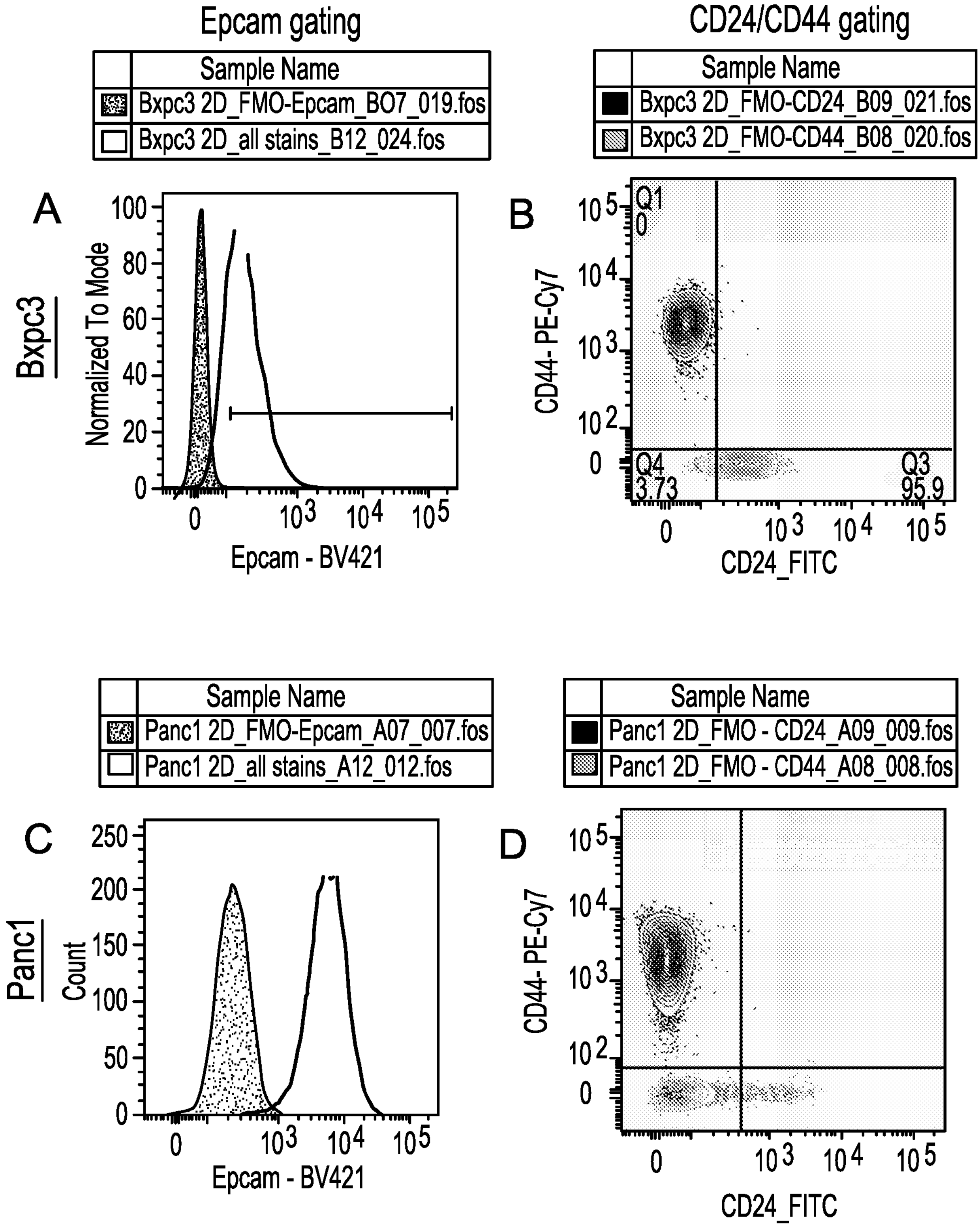
Epcam gating
Sample Name
Bxpc3 2D_FMO-Epcam_BO7_019.fos
Bxpc3 2D_all stains_B12_024.fos
CD24/CD44 gating
Sample Name
Bxpc3 2D_FMO-CD24_B09_021.fos
Bxpc3 2D_FMO-CD44_B08_020.fos
A
Bxpc3
Normalized To Mode
Epcam - BV421
B
CD44- PE-Cy7
CD24_FITC
Q1
0
Q4
3.73
Q3
95.9
Sample Name
Panc1 2D_FMO-Epcam_A07_007.fos
Panc1 2D_all stains_A12_012.fos
Sample Name
Panc1 2D_FMO - CD24_A09_009.fos
Panc1 2D_FMO - CD44_A08_008.fos
C
Panc1
Count
Epcam - BV421
D
CD44- PE-Cy7
CD24_FITC

Figure 16

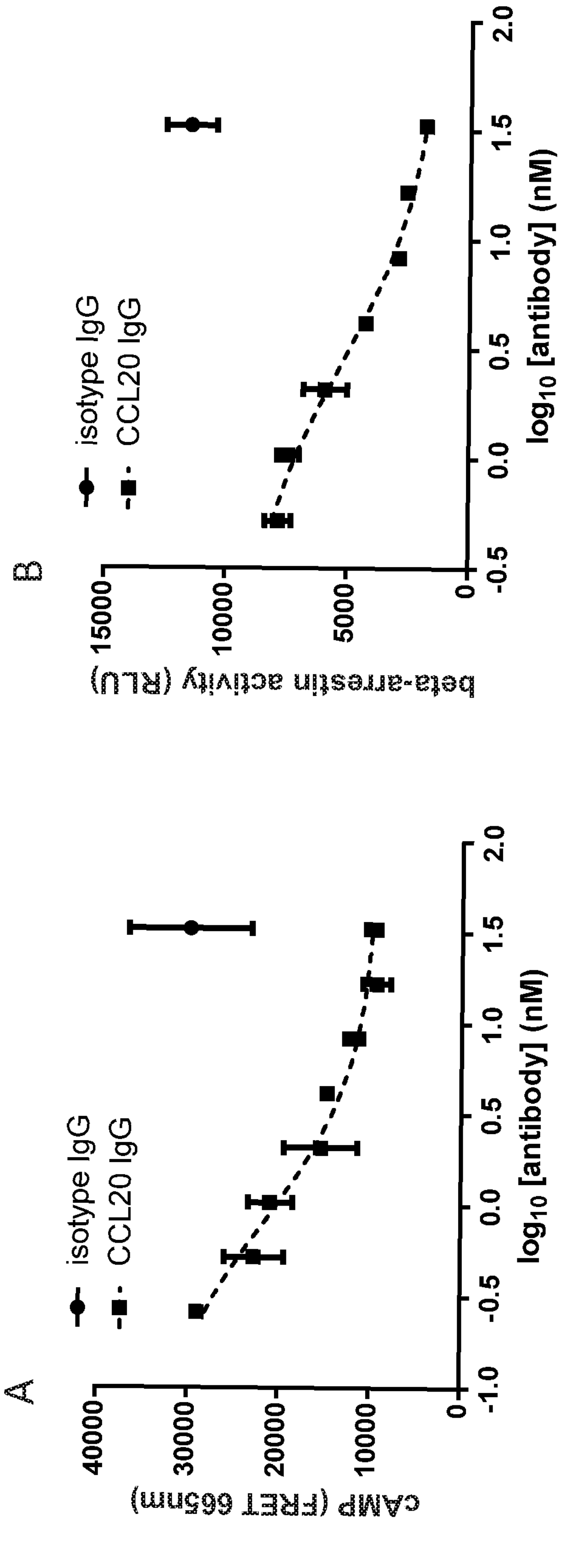
A
isotype IgG
CCL20 IgG
cAMP (FRET 665nm)
40000
30000
20000
10000
0
-1.0
-0.5
0.0
0.5
1.0
1.5
2.0
$log_{10}$ [antibody] (nM)
B
isotype IgG
CCL20 IgG
beta-arrestin activity (RLU)
15000
10000
5000
0
-0.5
0.0
0.5
1.0
1.5
2.0
$log_{10}$ [antibody] (nM)

Figure 17

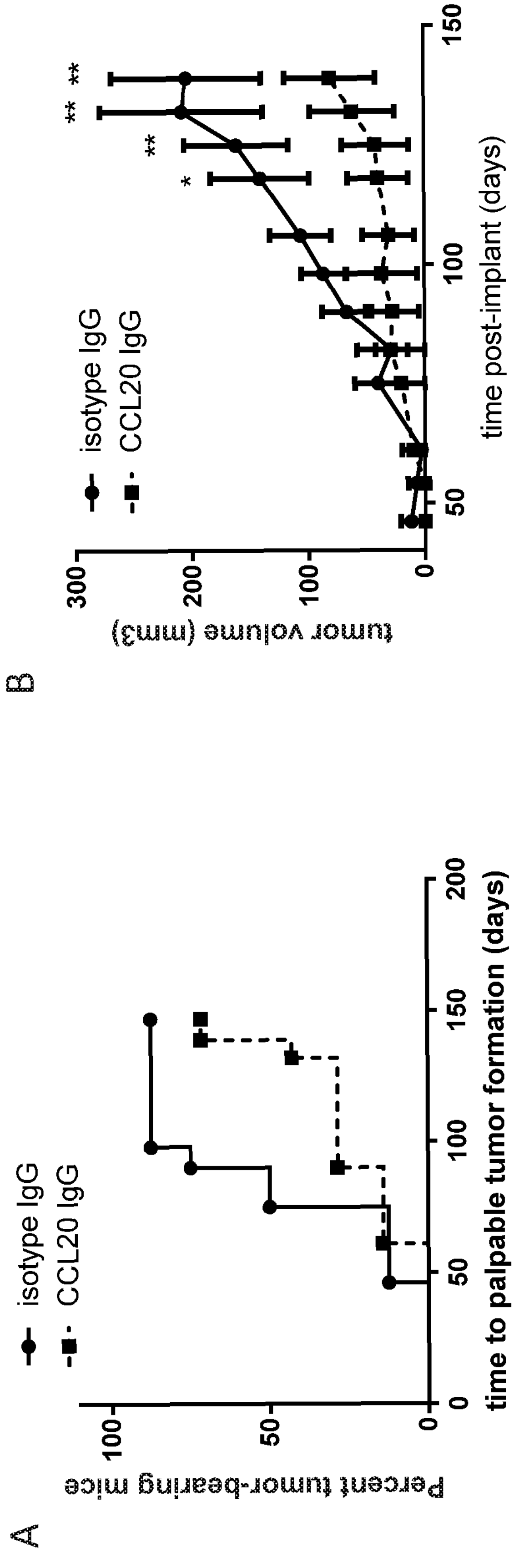
A
isotype IgG
CCL20 IgG
Percent tumor-bearing mice
100
50
0
0
50
100
150
200
time to palpable tumor formation (days)
B
isotype IgG
CCL20 IgG
tumor volume (mm3)
300
200
100
0
50
100
150
time post-implant (days)
*
**
**
**

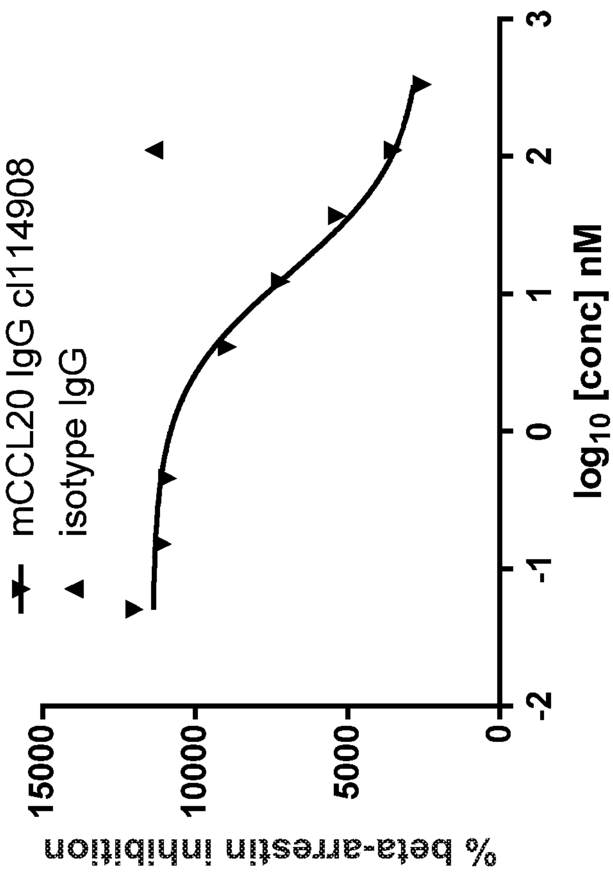
mCCL20 IgG cl114908
isotype IgG
% beta-arrestin inhibition
15000
10000
5000
0
-2
-1
0
1
2
3
$log_{10}$ [conc] nM

Figure 18

Figure 19

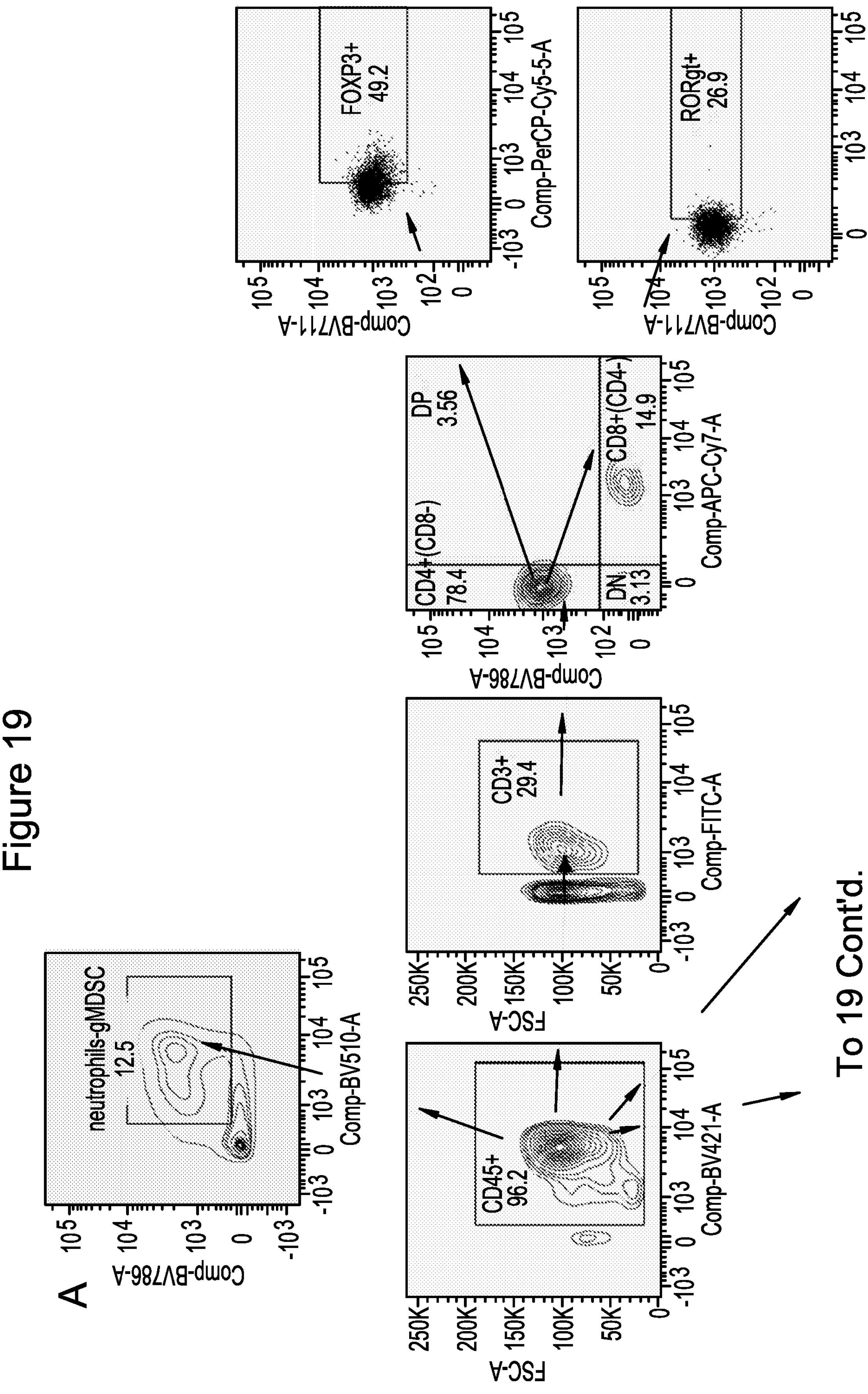


To 19 Cont'd.

Figure 19 Cont'd.

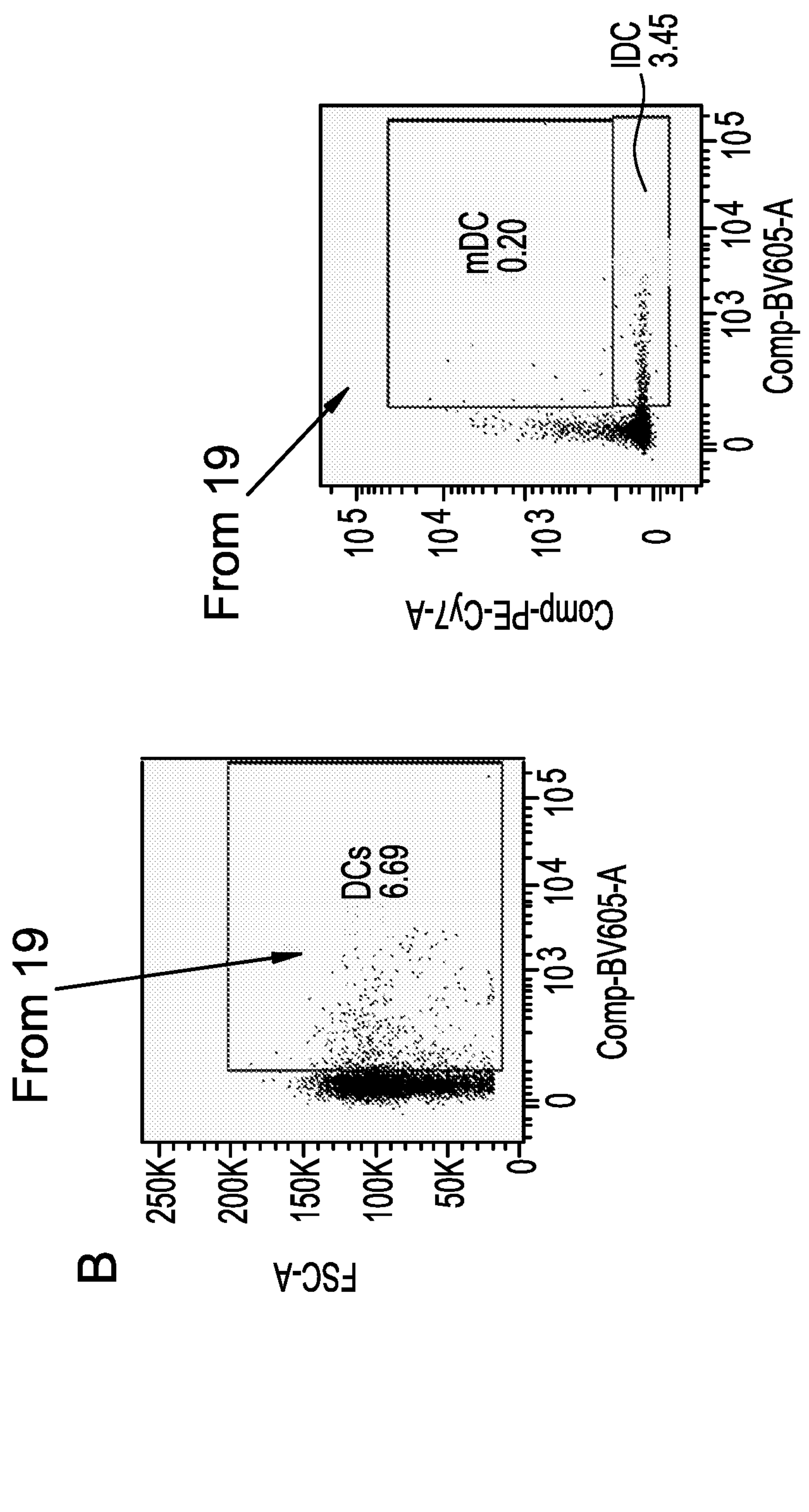

Figure 20

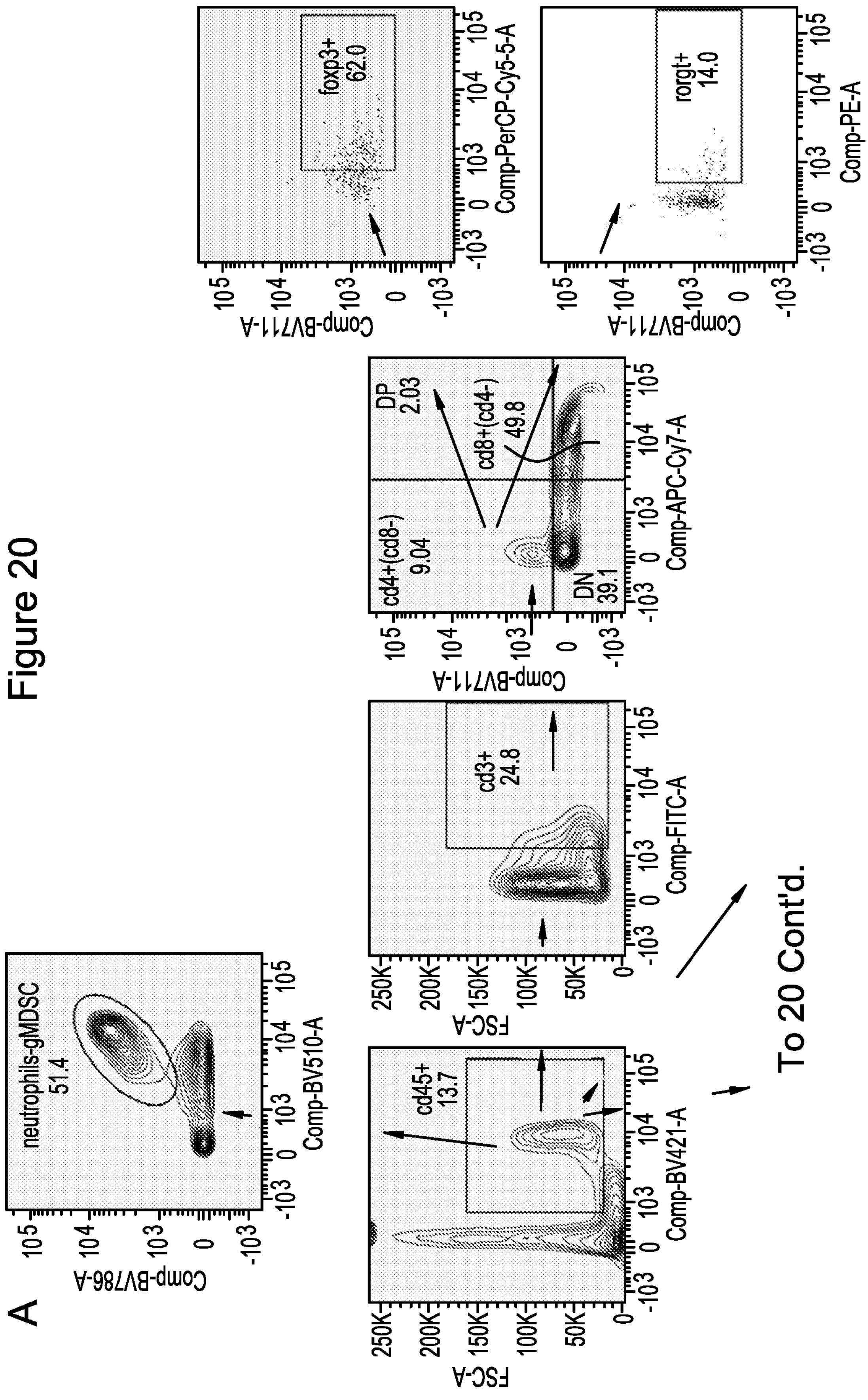
A
neutrophils-gMDSC
51.4
Comp-BV786-A
Comp-BV510-A
FSC-A
cd45+
13.7
Comp-BV421-A
cd3+
24.8
Comp-FITC-A
cd4+(cd8-)
9.04
DP
2.03
cd8+(cd4-)
49.8
DN
39.1
Comp-BV711-A
Comp-APC-Cy7-A
foxp3+
62.0
Comp-PerCP-Cy5-5-A
rorgt+
14.0
Comp-PE-A
To 20 Cont'd.

Figure 20 Cont'd.

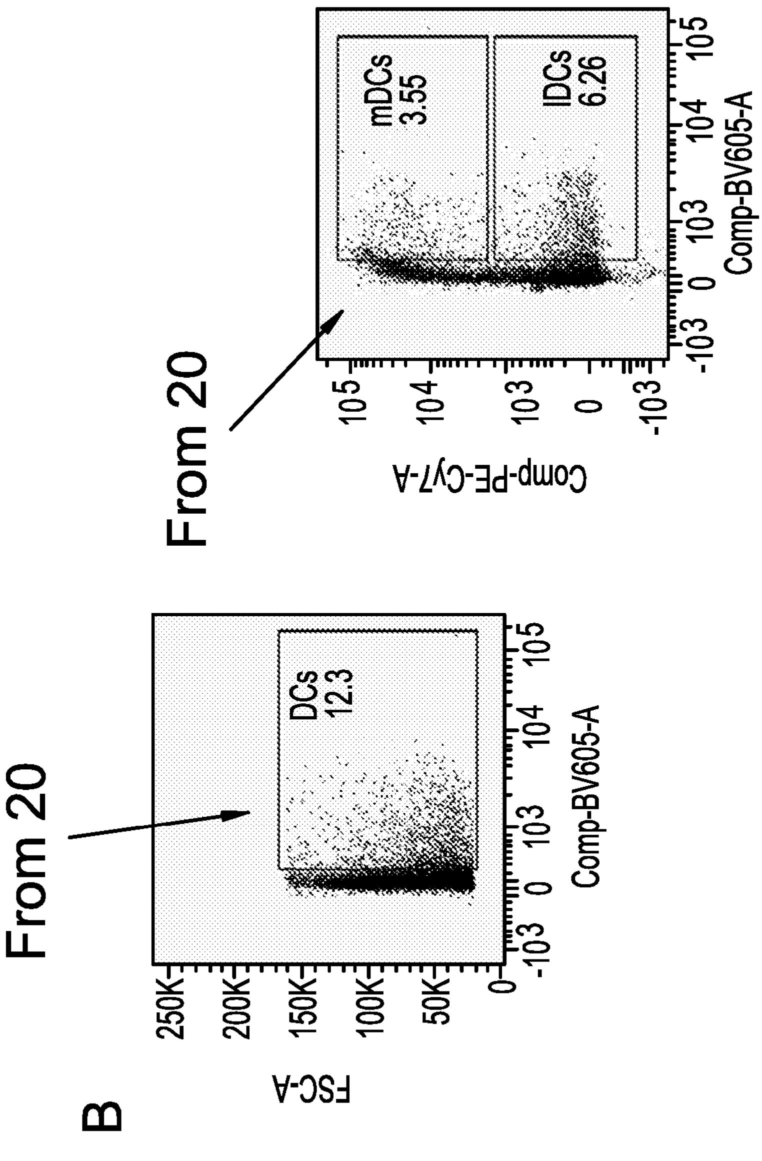

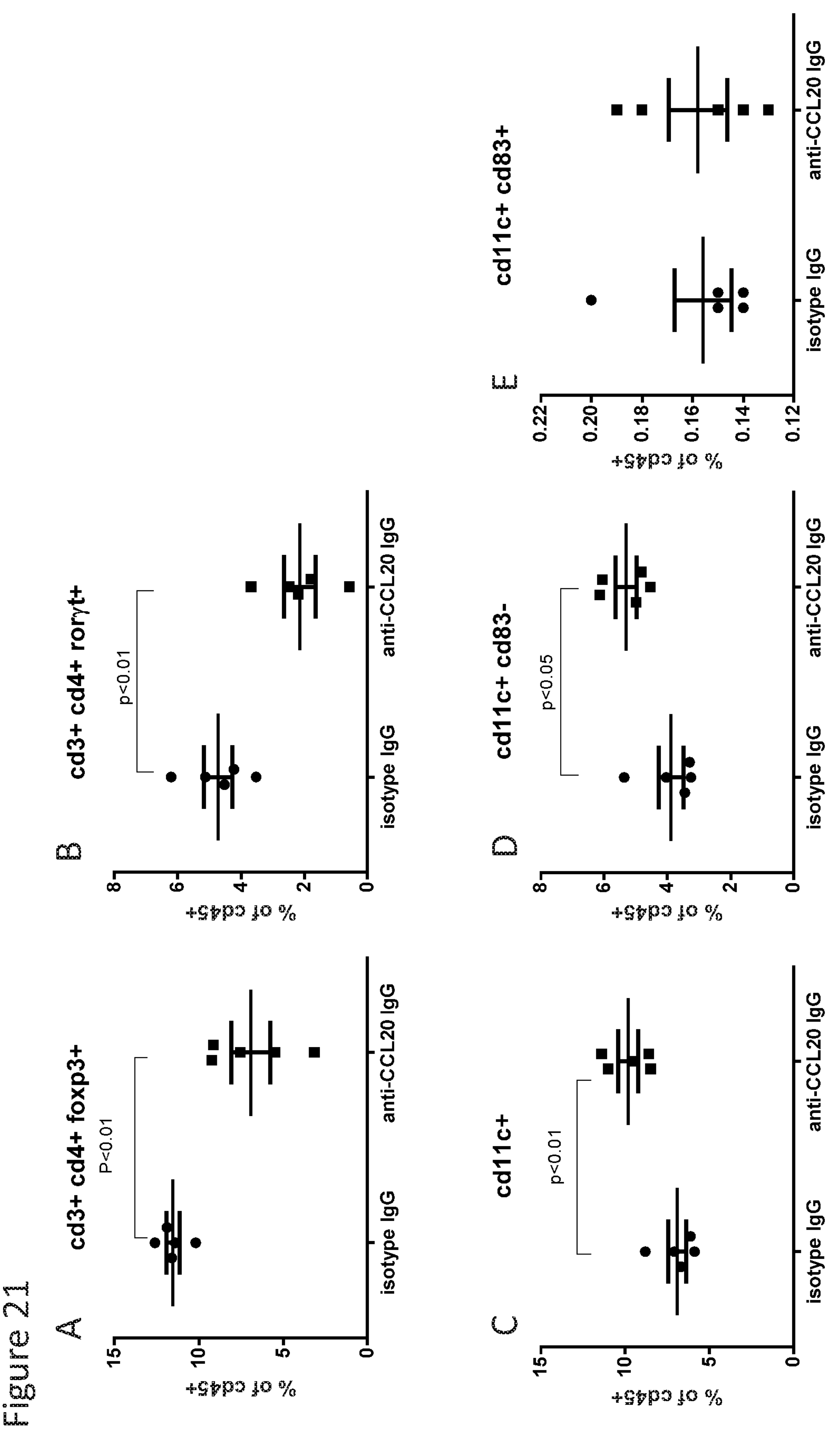
Figure 21
A
cd3+ cd4+ foxp3+
P<0.01
% of cd45+
15
10
5
0
isotype IgG
anti-CCL20 IgG
B
cd3+ cd4+ rorγt+
p<0.01
% of cd45+
8
6
4
2
0
isotype IgG
anti-CCL20 IgG
C
cd11c+
p<0.01
% of cd45+
15
10
5
0
isotype IgG
anti-CCL20 IgG
D
cd11c+ cd83-
p<0.05
% of cd45+
8
6
4
2
0
isotype IgG
anti-CCL20 IgG
E
cd11c+ cd83+
% of cd45+
0.22
0.20
0.18
0.16
0.14
0.12
isotype IgG
anti-CCL20 IgG

Figure 22

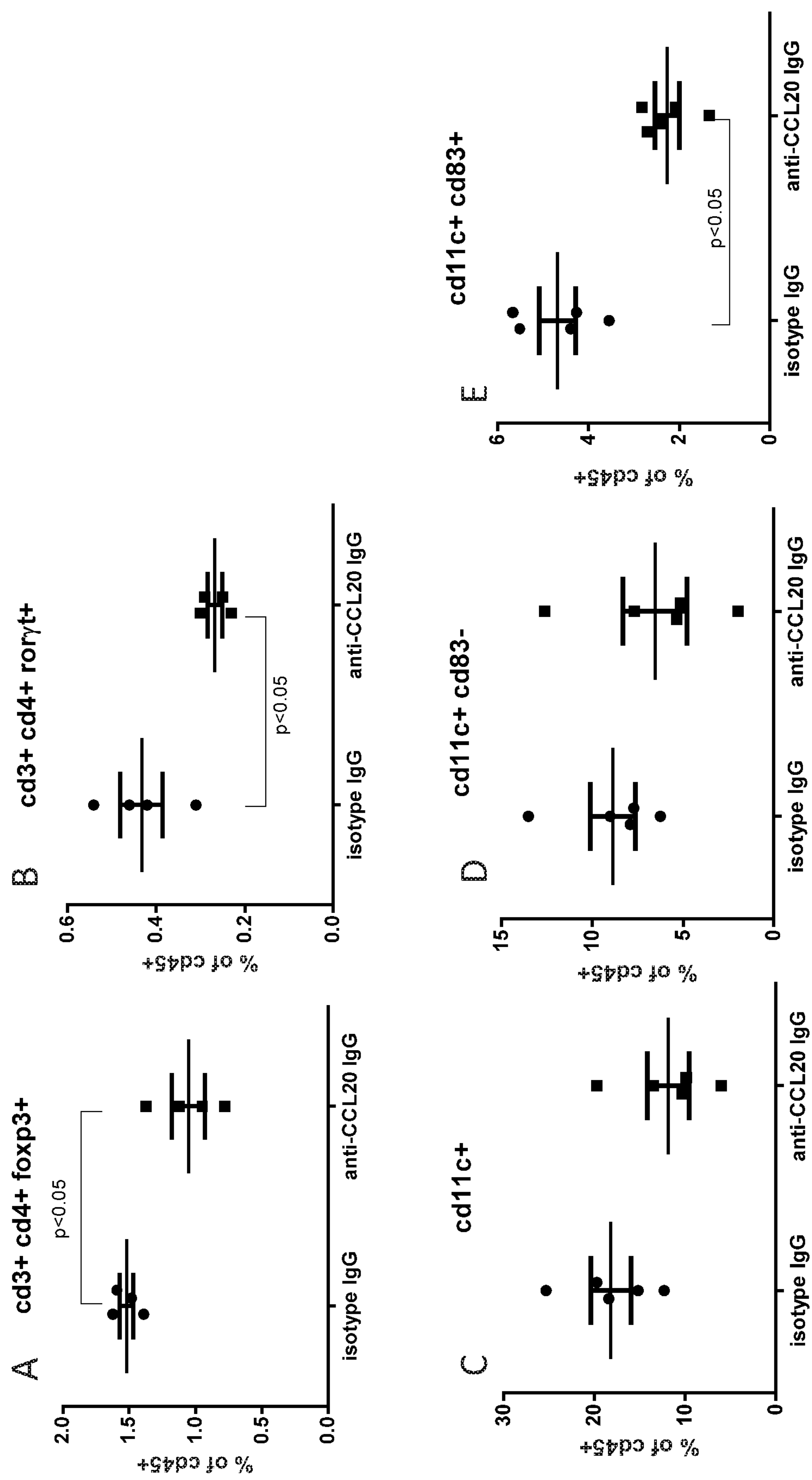
A
cd3+ cd4+ foxp3+
p<0.05
% of cd45+
2.0
1.5
1.0
0.5
0.0
isotype IgG
anti-CCL20 IgG
B
cd3+ cd4+ rorγt+
p<0.05
% of cd45+
0.6
0.4
0.2
0.0
isotype IgG
anti-CCL20 IgG
C
cd11c+
% of cd45+
30
20
10
0
isotype IgG
anti-CCL20 IgG
D
cd11c+ cd83-
% of cd45+
15
10
5
0
isotype IgG
anti-CCL20 IgG
E
cd11c+ cd83+
p<0.05
% of cd45+
6
4
2
0
isotype IgG
anti-CCL20 IgG

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20070099209 A
- WO 2009018386 A
- US 2012045229 W
- WO 2013006544 A


### Non-patent literature cited in the description


- **JUO, PEI-SHOW.** Concise Dictionary of Biomedicine and Molecular Biology. CRC Press, 2002
- The Dictionary of Cell and Molecular Biology. Academic Press, 1999
- Oxford Dictionary Of Biochemistry And Molecular Biology, Revised. Oxford University Press, 2000
- **KARLIN ; ALTSCHUL.** *Proc. Natl. Acad. Sci USA,* 1993, vol. 90, 5873-5877
- **ALTSCHUL et al.** *J. Mol. Biol.,* 1990, vol. 215, 403-410
- **MOSERLE, L. et al.** *Cancer Res.,* 2008, vol. 68, 5658-5668
- **RYBACK, A.P. et al.** *Biochim. Biophys. Acta,* 2011, vol. 1813, 683-694
- **XIA, P.** *Curr Stem Cell Res Ther.,* March 2014, vol. 9 (2), 102-11
- **SHIMAMURA, M. et al.** *Endocr. J.,* 2014, vol. 61 (5), 481-90
- **TIRINO, V. et al.** *FASEB J.,* January 2013, vol. 27 (1), 13-24
- **AL-HAJJ, M. ; WICHA, M.S. ; BENITO-HERNANDEZ, A. ; MORRISON, S.J. ; CLARKE, M.F.** Prospective identification of tumorigenic breast cancer cells. *Proceedings of the National Academy of Sciences of the United States of America,* 2003, vol. 100, 3983-3988
- **BABA, M. ; IMAI, T. ; NISHIMURA, M. ; KAKIZAKI, M. ; TAKAGI, S. ; HIESHIMA, K. ; NOMIYAMA, H. ; YOSHIE, O.** Identification of CCR6, the specific receptor for a novel lymphocyte-directed CC chemokine LARC. *The Journal of biological chemistry,* 1997, vol. 272, 14893-14898
- **BABA, T. ; NAKA, K. ; MORISHITA, S. ; KOMATSU, N. ; HIRAO, A. ; MUKAIDA, N.** MIP-1alpha/CCL3-mediated maintenance of leukemia-initiating cells in the initiation process of chronic myeloid leukemia. *The Journal of experimental medicine,* 2013, vol. 210, 2661-2673
- **BARKER, N. ; RIDGWAY, R.A. ; VAN ES, J.H. ; VAN DE WETERING, M. ; BEGTHEL, H. ; VAN DEN BORN, M. ; DANENBERG, E. ; CLARKE, A.R. ; SANSOM, O.J. ; CLEVERS, H.** Crypt stem cells as the cells-of-origin of intestinal cancer. *Nature,* 2009, vol. 457, 608-611
- **CHEN, K.-J. ; LIN, S.-Z. ; ZHOU, L. ; XIE, H.-Y. ; ZHOU, W.-H. ; TAKI-ELDIN, A. ; ZHENG, S.-S.** Selective recruitment of regulatory T cell through CCR6-CCL20 in hepatocellular carcinoma fosters tumor progression and predicts poor prognosis. *PloS one,* 2011, vol. 6, e24671
- **CREIGHTON, C.J. ; LI, X. ; LANDIS, M. ; DIXON, J.M. ; NEUMEISTER, V.M. ; SJOLUND, A. ; RIMM, D.L. ; WONG, H. ; RODRIGUEZ, A. ; HERSCHKOWITZ, J.I. et al.** Residual breast cancers after conventional therapy display mesenchymal as well as tumor-initiating features. *Proceedings of the National Academy of Sciences,* 2009, vol. 106, 13820-13825
- **GEMEI, M. ; MIRABELLI, P. ; DI NOTO, R. ; CORBO, C. ; IACCARINO, A. ; ZAMBOLI, A. ; TRONCONE, G. ; GALIZIA, G. ; LIETO, E. ; DEL VECCHIO, L. et al.** CD66c is a novel marker for colorectal cancer stem cell isolation, and its silencing halts tumor growth in vivo. *Cancer,* 2013, vol. 119, 729-738
- **GHADJAR, P. ; RUBIE, C. ; AEBERSOLD, D.M. ; KEILHOLZ, U.** The chemokine CCL20 and its receptor CCR6 in human malignancy with focus on colorectal cancer. *International journal of cancer Journal international du cancer,* 2009, vol. 125, 741-745
- **GINESTIER, C. ; LIU, S. ; DIEBEL, M.E. ; KORKAYA, H. ; LUO, M. ; BROWN, M. ; WICINSKI, J. ; CABAUD, O. ; CHARAFE-JAUFFRET, E. ; BIRNBAUM, D. et al.** CXCR1 blockade selectively targets human breast cancer stem cells in vitro and in xenografts. *The Journal of clinical investigation,* 2010, vol. 120, 485-497

- **HERMANN, P.C. ; HUBER, S.L. ; HERRLER, T. ; AICHER, A. ; ELLWART, J.W. ; GUBA, M. ; BRUNS, C.J. ; HEESCHEN, C.** Distinct populations of cancer stem cells determine tumor growth and metastatic activity in human pancreatic cancer. *Cell stem cell,* 2007, vol. 1, 313-323
- **HIRSCH, D. ; BARKER, N. ; MCNEIL, N. ; HU, Y. ; CAMPS, J. ; MCKINNON, K. ; CLEVERS, H. ; RIED, T. ; GAISER, T.** LGR5 positivity defines stem-like cells in colorectal cancer. *Carcinogenesis,* 2014, vol. 35, 849-858
- **ILIOPOULOS, D. ; HIRSCH, H.A. ; WANG, G. ; STRUHL, K.** Inducible formation of breast cancer stem cells and their dynamic equilibrium with non-stem cancer cells via IL6 secretion. *Proceedings of the National Academy of Sciences of the United States of America,* 2011, vol. 108, 1397-1402
- **IWATA, T. ; TANAKA, K. ; INOUE, Y. ; TOIYAMA, Y. ; HIRO, J. ; FUJIKAWA, H. ; OKUGAWA, Y. ; UCHIDA, K. ; MOHRI, Y. ; KUSUNOKI, M.** Macrophage inflammatory protein-3 alpha (MIP-3a) is a novel serum prognostic marker in patients with colorectal cancer. *Journal of surgical oncology,* 2013, vol. 107, 160-166
- **KLEEFF, J. ; KUSAMA, T. ; ROSSI, D.L. ; ISHIWATA, T. ; MARUYAMA, H. ; FRIESS, H. ; BUCHLER, M.W. ; ZLOTNIK, A. ; KORC, M.** Detection and localization of Mip-3alpha/LARC/Exodus, a macrophage proinflammatory chemokine, and its CCR6 receptor in human pancreatic cancer. *International journal of cancer Journal international du cancer,* 1999, vol. 81, 650-657
- **LAPIDOT, T. ; SIRARD, C. ; VORMOOR, J. ; MURDOCH, B. ; HOANG, T. ; CACERES-CORTES, J. ; MINDEN, M. ; PATERSON, B. ; CALIGIURI, M.A. ; DICK, J.E.** A cell initiating human acute myeloid leukaemia after transplantation into SCID mice. *Nature,* 1994, vol. 367, 645-648
- **LI, C. ; HEIDT, D.G. ; DALERBA, P. ; BURANT, C.F. ; ZHANG, L. ; ADSAY, V. ; WICHA, M. ; CLARKE, M.F. ; SIMEONE, D.M.** Identification of pancreatic cancer stem cells. *Cancer research,* 2007, vol. 67, 1030-1037
- **LIU, J. ; ZHANG, N. ; LI, Q. ; ZHANG, W. ; KE, F. ; LENG, Q. ; WANG, H. ; CHEN, J. ; WANG, H.** Tumor-associated macrophages recruit CCR6+ regulatory T cells and promote the development of colorectal cancer via enhancing CCL20 production in mice. *PLoS One,* 29 April 2011, vol. 6 (4), e19495
- **MARSIGLIANTE, S. ; VETRUGNO, C. ; MUSCELLA, A.** CCL20 induces migration and proliferation on breast epithelial cells. *Journal of cellular physiology,* 2013, vol. 228, 1873-1883
- **NANDI, B. ; PAI, C. ; HUANG, Q. ; PRABHALA, R.H. ; MUNSHI, N.C. ; GOLD, J.S.** CCR6, the sole receptor for the chemokine CCL20, promotes spontaneous intestinal tumorigenesis. *PloS one,* 2014, vol. 9, e97566
- **SCHUTYSER, E. ; STRUYF, S. ; VAN DAMME, J.** The CC chemokine CCL20 and its receptor CCR6. *Cytokine & growth factor reviews,* 2003, vol. 14, 409-426
- **TEHRANCHI, R. ; WOLL, P.S. ; ANDERSON, K. ; BUZA-VIDAS, N. ; MIZUKAMI, T. ; MEAD, A.J. ; ÅSTRAND-GRUNDSTROM, I. ; STRÖMBECK, B. ; HORVAT, A. ; FERRY, H. et al.** Persistent Malignant Stem Cells in del(5q) Myelodysplasia in Remission. *New England Journal of Medicine,* 2010, vol. 363, 1025-1037
- **VAN VLERKEN, L.E. ; KIEFER, C.M. ; MOREHOUSE, C. ; LI, Y. ; GROVES, C. ; WILSON, S.D. ; YAO, Y. ; HOLLINGSWORTH, R.E. ; HURT, E.M.** EZH2 is required for breast and pancreatic cancer stem cell maintenance and can be used as a functional cancer stem cell reporter. *Stem cells translational medicine,* 2013, vol. 2, 43-52
- **VOO, KS ; BOVER, L ; HARLINE, ML ; VIEN, LT ; FACCHINETTI V ; ARIMA, K ; KWAK, LW ; LIU, YJ.** Antibodies targeting human OX40 expand effector T cells and block inducible and natural regulatory T cell function. *J. Immunol.,* 01 October 2013, vol. 191 (7), 3641-3650
- **WANG, C. ; XIE, J. ; GUO, J. ; MANNING, H.C. ; GORE, J.C. ; GUO, N.** Evaluation of CD44 and CD133 as cancer stem cell markers for colorectal cancer. *Oncology reports,* 2012, vol. 28, 1301-1308
- **WANG, Y. ; MA, Y. ; FANG, Y. ; WU, S. ; LIU, L. ; FU, D. ; SHEN, X.** Regulatory T cell: a protection for tumour cells. *Journal of Cellular and Molecular Medicine,* 2012, vol. 16, 425-436
- **WURTH, R. ; BAJETTO, A. ; HARRISON, J.K. ; BARBIERI, F. ; FLORIO, T.** CXCL12 modulation of CXCR4 and CXCR7 activity in human glioblastoma stem-like cells and regulation of the tumor microenvironment. *Frontiers in cellular neuroscience,* 2014, vol. 8, 144
- **YU, Q. ; LOU, X.M. ; HE, Y.** Preferential Recruitment of Th17 Cells to Cervical Cancer via CCR6-CCL20 Pathway. *PLoS One,* 13 March 2015, vol. 10 (3), e0120855
- **ZHANG, C.Y. ; QI, Y. ; LI, X.N. ; YANG, Y. ; LIU, D.L. ; ZHAO, J. ; ZHU, D.Y. ; WU, K. ; ZHOU, X.D. ; ZHAO, S.** The role of CCL20/CCR6 axis in recruiting Treg cells to tumor sites of NSCLC patients. *Biomed Pharmacother,* February 2015, vol. 69, 242-8

SEQUENCE LISTING

<110> van Vlerken-Ysla, Lilian Emilia
Hurt, Elaine Marie

<120> Compositions and Methods for Treating Cancer

<130> CCL20-100P1

<160> 8

<170> PatentIn version 3.5

<210> 1
<211> 3156
<212> DNA
<213> Homo sapiens

<400> 1

```
gaaggtcccc aggactctgt ggtcatcagt aagagagggc ccacgtgtat atgctggtga      60
acagaaatgt caaccttttc aaagtctgac atttaagaga aaaaactgtg gctgttggtt     120
tgtggaacag acagctcctt ctttattgag tcacctctac tttcctgcta ccgctgcctg     180
tgagctgaag ggctgaacc atacactcct ttttctacaa ccagcttgca ttttttctgc      240
ccacaatgag cggggaatca atgaatttca gcgatgtttt cgactccagt gaagattatt     300
ttgtgtcagt caatacttca tattactcag ttgattctga gatgttactg tgctccttgc     360
aggaggtcag gcagttctcc aggctatttg taccgattgc ctactccttg atctgtgtct     420
ttggcctcct ggggaatatt ctggtggtga tcacctttgc tttttataag aaggccaggt     480
ctatgacaga cgtctatctc ttgaacatgg ccattgcaga catcctcttt gttcttactc     540
tcccattctg ggcagtgagt catgccaccg gtgcgtgggt tttcagcaat gccacgtgca     600
agttgctaaa aggcatctat gccatcaact ttaactgcgg gatgctgctc ctgacttgca     660
ttagcatgga ccggtacatc gccattgtac aggcgactaa gtcattccgg ctccgatcca     720
gaacactacc gcgcagcaaa atcatctgcc ttgttgtgtg gggctgtca gtcatcatct      780
ccagctcaac ttttgtcttc aaccaaaaat acaacaccca aggcagcgat gtctgtgaac     840
ccaagtacca gactgtctcg gagcccatca ggtggaagct gctgatgttg gggcttgagc     900
tactctttgg tttctttatc cctttgatgt tcatgatatt ttgttacacg ttcattgtca     960
aaaccttggt gcaagctcag aattctaaaa ggcacaaagc catccgtgta atcatagctg    1020
tggtgcttgt gtttctggct tgtcagattc ctcataacat ggtcctgctt gtgacggctg    1080
caaatttggg taaaatgaac cgatcctgcc agagcgaaaa gctaattggc tatacgaaaa    1140
ctgtcacaga agtcctggct ttcctgcact gctgcctgaa ccctgtgctc tacgctttta    1200
ttgggcagaa gttcagaaac tactttctga agatcttgaa ggacctgtgg tgtgtgagaa    1260
ggaagtacaa gtcctcaggc ttctcctgtg ccgggaggta ctcagaaaac atttctcggc    1320
```

agaccagtga gaccgcagat aacgacaatg cgtcgtcctt cactatgtga tagaaagctg 1380
agtctcccta aggcatgtgt gaaacatact catagatgtt atgcaaaaaa aagtctatgg 1440
ccaggtatgc atggaaaatg tgggaattaa gcaaaatcaa gcaagcctct ctcctgcggg 1500
acttaacgtg ctcatgggct gtgtgatctc ttcagggtgg ggtggtctct gataggtagc 1560
attttccagc actttgcaag gaatgttttg tagctctagg gtatatatcc gcctggcatt 1620
tcacaaaaca gcctttggga aatgctgaat taaagtgaat tgttgacaaa tgtaaacatt 1680
ttcagaaata ttcatgaagc ggtcacagat cacagtgtct tttggttaca gcacaaaatg 1740
atggcagtgg tttgaaaaac taaaacagaa aaaaaaatgg aagccaacac atcactcatt 1800
ttaggcaaat gtttaaacat ttttatctat cagaatgttt attgttgctg gttataagca 1860
gcaggattgg ccggctagtg tttcctctca tttccctttg atacagtcaa caagcctgac 1920
cctgtaaaat ggaggtggaa agacaagctc aagtgttcac aacctggaag tgcttcggga 1980
agaaggggac aatggcagaa caggtgttgg tgacaattgt caccaattgg ataaagcagc 2040
tcaggttgta gtgggccatt aggaaactgt cggtttgctt tgatttccct gggagctgtt 2100
ctctgtcgtg agtgtctctt gtctaaacgt ccattaagct gagagtgcta tgaagacagg 2160
atctagaata atcttgctca cagctgtgct ctgagtgcct agcggagttc cagcaaacaa 2220
aatggactca agagagattt gattaatgaa tcgtaatgaa gttggggttt attgtacagt 2280
ttaaaatgtt agatgttttt aattttttaa ataaatggaa tacttttttt ttttttttaa 2340
agaaagcaac tttactgaga caatgtagaa agaagttttg ttccgtttct ttaatgtggt 2400
tgaagagcaa tgtgtggctg aagacttttg ttatgaggag ctgcagatta gctaggggac 2460
agctggaatt atgctggctt ctgataatta ttttaaaggg gtctgaaatt tgtgatggaa 2520
tcagatttta acagctctct tcaatgacat agaaagttca tggaactcat gtttttaaag 2580
ggctatgtaa atatatgaac attagaaaaa tagcaacttg tgttacaaaa atacaaacac 2640
atgttaggaa ggtactgtca tgggctaggc atggtggctc acacctgtaa tcccagcatt 2700
ttgggaagct aagatgggtg gatcacttga ggtcaggagt ttgagaccag cctggccaac 2760
atggcgaaac ccctctctac taaaaataca aaaatttgcc aggcgtggtg gcgggtgcct 2820
gtaatcccag ctacttggga ggctgaggca agagaatcgc ttgaacccag gaggcagagg 2880
ttgcagtgag ccgagatcgt gccattgcac tccagcctgg gtgacaaagc gagactccat 2940
ctcaaaaaaa aaaaaaaaaa aaaaggaaag aactgtcatg taaacatacc aacatgttta 3000
aacctgacaa tggtgttatt tgaaacttta tattgttctt gtaagcttta actatatctc 3060
tctttaaaat gcaaaataat gtcttaagat tcaaagtctg tatttttaaa gcatggcttt 3120
ggctttgcaa aataaaaaat gtgttttgta catgaa 3156

<210> 2
<211> 374
<212> **PRT**
<213> Homo sapiens

<400> 2

```
Met Ser Gly Glu Ser Met Asn Phe Ser Asp Val Phe Asp Ser Ser Glu
1               5                   10                  15

Asp Tyr Phe Val Ser Val Asn Thr Ser Tyr Tyr Ser Val Asp Ser Glu
            20                  25                  30

Met Leu Leu Cys Ser Leu Gln Glu Val Arg Gln Phe Ser Arg Leu Phe
        35                  40                  45

Val Pro Ile Ala Tyr Ser Leu Ile Cys Val Phe Gly Leu Leu Gly Asn
    50                  55                  60

Ile Leu Val Val Ile Thr Phe Ala Phe Tyr Lys Lys Ala Arg Ser Met
65                  70                  75                  80

Thr Asp Val Tyr Leu Leu Asn Met Ala Ile Ala Asp Ile Leu Phe Val
                85                  90                  95

Leu Thr Leu Pro Phe Trp Ala Val Ser His Ala Thr Gly Ala Trp Val
            100                 105                 110

Phe Ser Asn Ala Thr Cys Lys Leu Leu Lys Gly Ile Tyr Ala Ile Asn
        115                 120                 125

Phe Asn Cys Gly Met Leu Leu Leu Thr Cys Ile Ser Met Asp Arg Tyr
    130                 135                 140

Ile Ala Ile Val Gln Ala Thr Lys Ser Phe Arg Leu Arg Ser Arg Thr
145                 150                 155                 160

Leu Pro Arg Ser Lys Ile Ile Cys Leu Val Val Trp Gly Leu Ser Val
                165                 170                 175

Ile Ile Ser Ser Ser Thr Phe Val Phe Asn Gln Lys Tyr Asn Thr Gln
            180                 185                 190

Gly Ser Asp Val Cys Glu Pro Lys Tyr Gln Thr Val Ser Glu Pro Ile
        195                 200                 205

Arg Trp Lys Leu Leu Met Leu Gly Leu Glu Leu Leu Phe Gly Phe Phe
    210                 215                 220
```

```
Ile Pro Leu Met Phe Met Ile Phe Cys Tyr Thr Phe Ile Val Lys Thr
225                 230                 235                 240

Leu Val Gln Ala Gln Asn Ser Lys Arg His Lys Ala Ile Arg Val Ile
                245                 250                 255

Ile Ala Val Val Leu Val Phe Leu Ala Cys Gln Ile Pro His Asn Met
            260                 265                 270

Val Leu Leu Val Thr Ala Ala Asn Leu Gly Lys Met Asn Arg Ser Cys
        275                 280                 285

Gln Ser Glu Lys Leu Ile Gly Tyr Thr Lys Thr Val Thr Glu Val Leu
    290                 295                 300

Ala Phe Leu His Cys Cys Leu Asn Pro Val Leu Tyr Ala Phe Ile Gly
305                 310                 315                 320

Gln Lys Phe Arg Asn Tyr Phe Leu Lys Ile Leu Lys Asp Leu Trp Cys
                325                 330                 335

Val Arg Arg Lys Tyr Lys Ser Ser Gly Phe Ser Cys Ala Gly Arg Tyr
            340                 345                 350

Ser Glu Asn Ile Ser Arg Gln Thr Ser Glu Thr Ala Asp Asn Asp Asn
        355                 360                 365

Ala Ser Ser Phe Thr Met
    370
```

<210> 3
<211> 3419
<212> **DNA**
<213> Homo sapiens

<400> 3

```
tgctcccagg agataaccag gtaaaggagt atgaaagttt gggtacaaac tcattgctgc      60
aaattgaaaa ccatgcaaag gctgtcttcc tctggggagt tcaatgcctc tctttttctt     120
atcactttac cattggttgg actttgattc cagggatcct acgattactc aataccctac     180
aggatataca tggttaacca tttgcatttg ggcaaatagg cgttactttt caataggaag     240
tggcaatcca gaacttgctt ttgggcaatt ctagtagctc accgcttttt tcttaatgac     300
tgctagaagc tgcatcttat tgacagatgg tcatcacatt ggtgagctgg agtcatcaga     360
ttgtggggcc cggagtgagg ctgaagggag tggatcagag cactgcctga gagtcacctc     420
tactttcctg ctaccgctgc ctgtgagctg aaggggctga accatacact cctttttcta     480
caaccagctt gcatttttc tgcccacaat gagcggggaa tcaatgaatt tcagcgatgt      540
```

tttcgactcc agtgaagatt attttgtgtc agtcaatact tcatattact cagttgattc 600
tgagatgtta ctgtgctcct tgcaggaggt caggcagttc tccaggctat ttgtaccgat 660
tgcctactcc ttgatctgtg tctttggcct cctggggaat attctggtgg tgatcacctt 720
tgctttttat aagaaggcca ggtctatgac agacgtctat ctcttgaaca tggccattgc 780
agacatcctc tttgttctta ctctcccatt ctgggcagtg agtcatgcca ccggtgcgtg 840
ggttttcagc aatgccacgt gcaagttgct aaaaggcatc tatgccatca actttaactg 900
cgggatgctg ctcctgactt gcattagcat ggaccggtac atcgccattg tacaggcgac 960
taagtcattc cggctccgat ccagaacact accgcgcagc aaaatcatct gccttgttgt 1020
gtgggggctg tcagtcatca tctccagctc aacttttgtc ttcaaccaaa aatacaacac 1080
ccaaggcagc gatgtctgtg aacccaagta ccagactgtc tcggagccca tcaggtggaa 1140
gctgctgatg ttggggcttg agctactctt tggtttcttt atccctttga tgttcatgat 1200
attttgttac acgttcattg tcaaaacctt ggtgcaagct cagaattcta aaaggcacaa 1260
agccatccgt gtaatcatag ctgtggtgct tgtgtttctg gcttgtcaga ttcctcataa 1320
catggtcctg cttgtgacgg ctgcaaattt gggtaaaatg aaccgatcct gccagagcga 1380
aaagctaatt ggctatacga aaactgtcac agaagtcctg gctttcctgc actgctgcct 1440
gaaccctgtg ctctacgctt ttattgggca gaagttcaga aactactttc tgaagatctt 1500
gaaggacctg tggtgtgtga gaaggaagta caagtcctca ggcttctcct gtgccgggag 1560
gtactcagaa aacatttctc ggcagaccag tgagaccgca gataacgaca atgcgtcgtc 1620
cttcactatg tgatagaaag ctgagtctcc ctaaggcatg tgtgaaacat actcatagat 1680
gttatgcaaa aaaaagtcta tggccaggta tgcatggaaa atgtgggaat taagcaaaat 1740
caagcaagcc tctctcctgc gggacttaac gtgctcatgg gctgtgtgat ctcttcaggg 1800
tggggtggtc tctgataggt agcattttcc agcactttgc aaggaatgtt ttgtagctct 1860
agggtatata tccgcctggc atttcacaaa acagcctttg ggaaatgctg aattaaagtg 1920
aattgttgac aaatgtaaac attttcagaa atattcatga agcggtcaca gatcacagtg 1980
tcttttggtt acagcacaaa atgatggcag tggtttgaaa aactaaaaca gaaaaaaaaa 2040
tggaagccaa cacatcactc attttaggca aatgtttaaa catttttatc tatcagaatg 2100
tttattgttg ctggttataa gcagcaggat tggccggcta gtgtttcctc tcatttccct 2160
ttgatacagt caacaagcct gaccctgtaa aatggaggtg gaaagacaag ctcaagtgtt 2220
cacaacctgg aagtgcttcg ggaagaaggg gacaatggca gaacaggtgt tggtgacaat 2280
tgtcaccaat tggataaagc agctcaggtt gtagtgggcc attaggaaac tgtcggtttg 2340
ctttgatttc cctgggagct gttctctgtc gtgagtgtct cttgtctaaa cgtccattaa 2400

```
gctgagagtg ctatgaagac aggatctaga ataatcttgc tcacagctgt gctctgagtg     2460
cctagcggag ttccagcaaa caaaatggac tcaagagaga tttgattaat gaatcgtaat     2520
gaagttgggg tttattgtac agtttaaaat gttagatgtt tttaattttt taaataaatg     2580
gaatactttt tttttttttt taaagaaagc aactttactg agacaatgta gaaagaagtt     2640
ttgttccgtt tctttaatgt ggttgaagag caatgtgtgg ctgaagactt ttgttatgag     2700
gagctgcaga ttagctaggg gacagctgga attatgctgg cttctgataa ttattttaaa     2760
ggggtctgaa atttgtgatg gaatcagatt ttaacagctc tcttcaatga catagaaagt     2820
tcatggaact catgttttta aagggctatg taaatatatg aacattagaa aaatagcaac     2880
ttgtgttaca aaaatacaaa cacatgttag gaaggtactg tcatgggcta ggcatggtgg     2940
ctcacacctg taatcccagc attttgggaa gctaagatgg gtggatcact tgaggtcagg     3000
agtttgagac cagcctggcc aacatggcga aacccctctc tactaaaaat acaaaaattt     3060
gccaggcgtg gtggcgggtg cctgtaatcc cagctacttg ggaggctgag gcaagagaat     3120
cgcttgaacc caggaggcag aggttgcagt gagccgagat cgtgccattg cactccagcc     3180
tgggtgacaa agcgagactc catctcaaaa aaaaaaaaaa aaaaaaagga aagaactgtc     3240
atgtaaacat accaacatgt ttaaacctga caatggtgtt atttgaaact ttatattgtt     3300
cttgtaagct ttaactatat ctctctttaa aatgcaaaat aatgtcttaa gattcaaagt     3360
ctgtattttt aaagcatggc tttggctttg caaaataaaa aatgtgtttt gtacatgaa      3419
```

<210> 4
<211> 374
<212> **PRT**
<213> Homo sapiens

<400> 4

```
Met Ser Gly Glu Ser Met Asn Phe Ser Asp Val Phe Asp Ser Ser Glu
1               5                   10                  15

Asp Tyr Phe Val Ser Val Asn Thr Ser Tyr Tyr Ser Val Asp Ser Glu
            20                  25                  30

Met Leu Leu Cys Ser Leu Gln Glu Val Arg Gln Phe Ser Arg Leu Phe
        35                  40                  45

Val Pro Ile Ala Tyr Ser Leu Ile Cys Val Phe Gly Leu Leu Gly Asn
    50                  55                  60

Ile Leu Val Val Ile Thr Phe Ala Phe Tyr Lys Lys Ala Arg Ser Met
65                  70                  75                  80

Thr Asp Val Tyr Leu Leu Asn Met Ala Ile Ala Asp Ile Leu Phe Val
```

85 90 95

Leu Thr Leu Pro Phe Trp Ala Val Ser His Ala Thr Gly Ala Trp Val
100 105 110

Phe Ser Asn Ala Thr Cys Lys Leu Leu Lys Gly Ile Tyr Ala Ile Asn
115 120 125

Phe Asn Cys Gly Met Leu Leu Leu Thr Cys Ile Ser Met Asp Arg Tyr
130 135 140

Ile Ala Ile Val Gln Ala Thr Lys Ser Phe Arg Leu Arg Ser Arg Thr
145 150 155 160

Leu Pro Arg Ser Lys Ile Ile Cys Leu Val Val Trp Gly Leu Ser Val
165 170 175

Ile Ile Ser Ser Ser Thr Phe Val Phe Asn Gln Lys Tyr Asn Thr Gln
180 185 190

Gly Ser Asp Val Cys Glu Pro Lys Tyr Gln Thr Val Ser Glu Pro Ile
195 200 205

Arg Trp Lys Leu Leu Met Leu Gly Leu Glu Leu Leu Phe Gly Phe Phe
210 215 220

Ile Pro Leu Met Phe Met Ile Phe Cys Tyr Thr Phe Ile Val Lys Thr
225 230 235 240

Leu Val Gln Ala Gln Asn Ser Lys Arg His Lys Ala Ile Arg Val Ile
245 250 255

Ile Ala Val Val Leu Val Phe Leu Ala Cys Gln Ile Pro His Asn Met
260 265 270

Val Leu Leu Val Thr Ala Ala Asn Leu Gly Lys Met Asn Arg Ser Cys
275 280 285

Gln Ser Glu Lys Leu Ile Gly Tyr Thr Lys Thr Val Thr Glu Val Leu
290 295 300

Ala Phe Leu His Cys Cys Leu Asn Pro Val Leu Tyr Ala Phe Ile Gly
305 310 315 320

Gln Lys Phe Arg Asn Tyr Phe Leu Lys Ile Leu Lys Asp Leu Trp Cys
325 330 335

Val Arg Arg Lys Tyr Lys Ser Ser Gly Phe Ser Cys Ala Gly Arg Tyr
340 345 350

Ser Glu Asn Ile Ser Arg Gln Thr Ser Glu Thr Ala Asp Asn Asp Asn
355 360 365

Ala Ser Ser Phe Thr Met
370

<210> 5
<211> 851
<212> DNA
<213> Homo sapiens

<400> 5

agaatataac agcactccca aagaactggg tactcaacac tgagcagatc tgttctttga 60

gctaaaaacc atgtgctgta ccaagagttt gctcctggct gctttgatgt cagtgctgct 120

actccacctc tgcggcgaat cagaagcagc aagcaacttt gactgctgtc ttggatacac 180

agaccgtatt cttcatccta aatttattgt gggcttcaca cggcagctgg ccaatgaagg 240

ctgtgacatc aatgctatca tctttcacac aaagaaaaag ttgtctgtgt gcgcaaatcc 300

aaaacagact tgggtgaaat atattgtgcg tctcctcagt aaaaaagtca agaacatgta 360

aaaactgtgg cttttctgga atggaattgg acatagccca agaacagaaa gaaccttgct 420

ggggttggag gtttcacttg cacatcatgg agggtttagt gcttatctaa tttgtgcctc 480

actggacttg tccaattaat gaagttgatt catattgcat catagtttgc tttgtttaag 540

catcacatta aagttaaact gtattttatg ttatttatag ctgtaggttt tctgtgttta 600

gctatttaat actaattttc cataagctat tttggtttag tgcaaagtat aaaattatat 660

ttggggggga ataagattat atggactttc ttgcaagcaa caagctattt tttaaaaaaa 720

actatttaac attcttttgt ttatattgtt ttgtctccta aattgttgta attgcattat 780

aaaataagaa aaatattaat aagacaaata ttgaaaataa agaaacaaaa agttcttctg 840

ttaaaaaaaa a 851

<210> 6
<211> 96
<212> PRT
<213> Homo sapiens

<400> 6

```
Met Cys Cys Thr Lys Ser Leu Leu Leu Ala Ala Leu Met Ser Val Leu
1               5                   10                  15

Leu Leu His Leu Cys Gly Glu Ser Glu Ala Ala Ser Asn Phe Asp Cys
            20                  25                  30

Cys Leu Gly Tyr Thr Asp Arg Ile Leu His Pro Lys Phe Ile Val Gly
        35                  40                  45

Phe Thr Arg Gln Leu Ala Asn Glu Gly Cys Asp Ile Asn Ala Ile Ile
    50                  55                  60

Phe His Thr Lys Lys Lys Leu Ser Val Cys Ala Asn Pro Lys Gln Thr
65                  70                  75                  80

Trp Val Lys Tyr Ile Val Arg Leu Leu Ser Lys Lys Val Lys Asn Met
                85                  90                  95
```

<210> 7
<211> 848
<212> DNA
<213> Homo sapiens

<400> 7

```
agaatataac agcactccca aagaactggg tactcaacac tgagcagatc tgttctttga      60
gctaaaaacc atgtgctgta ccaagagttt gctcctggct gctttgatgt cagtgctgct     120
actccacctc tgcggcgaat cagaagcaag caactttgac tgctgtcttg gatacacaga     180
ccgtattctt catcctaaat ttattgtggg cttcacacgg cagctggcca atgaaggctg     240
tgacatcaat gctatcatct ttcacacaaa gaaaaagttg tctgtgtgcg caaatccaaa     300
acagacttgg gtgaaatata ttgtgcgtct cctcagtaaa aaagtcaaga acatgtaaaa     360
actgtggctt ttctggaatg gaattggaca tagcccaaga acagaaagaa ccttgctggg     420
gttggaggtt tcacttgcac atcatggagg gtttagtgct tatctaattt gtgcctcact     480
ggacttgtcc aattaatgaa gttgattcat attgcatcat agtttgcttt gtttaagcat     540
cacattaaag ttaaactgta ttttatgtta tttatagctg taggttttct gtgtttagct     600
atttaatact aattttccat aagctatttt ggtttagtgc aaagtataaa attatatttg     660
ggggggaata agattatatg gactttcttg caagcaacaa gctatttttt aaaaaaaact     720
atttaacatt cttttgttta tattgttttg tctcctaaat tgttgtaatt gcattataaa     780
ataagaaaaa tattaataag acaaatattg aaaataaaga aacaaaaagt tcttctgtta     840
aaaaaaaa                                                              848
```

<210> 8
<211> 95
<212> PRT
<213> Homo sapiens

<400> 8

```
Met Cys Cys Thr Lys Ser Leu Leu Leu Ala Ala Leu Met Ser Val Leu
1               5                   10                  15

Leu Leu His Leu Cys Gly Glu Ser Glu Ala Ser Asn Phe Asp Cys Cys
            20                  25                  30

Leu Gly Tyr Thr Asp Arg Ile Leu His Pro Lys Phe Ile Val Gly Phe
        35                  40                  45

Thr Arg Gln Leu Ala Asn Glu Gly Cys Asp Ile Asn Ala Ile Ile Phe
    50                  55                  60

His Thr Lys Lys Lys Leu Ser Val Cys Ala Asn Pro Lys Gln Thr Trp
65                  70                  75                  80

Val Lys Tyr Ile Val Arg Leu Leu Ser Lys Lys Val Lys Asn Met
                85                  90                  95
```